# EUROPÄISCHE PATENTSCHRIFT

(11) **EP 0 736 014 B1**
(45) Veröffentlichungstag und Bekanntmachung des Hinweises auf die Patenterteilung: **04.04.2001**
(21) Anmeldenummer: 95902132.0
(22) Anmeldetag: 07.12.1994
(51) Int. Cl.: C07D 239/56, A01N 43/54

(54) **N-CYANOARYL-STICKSTOFFHETEROCYCLEN MIT SCHWEFELHALTIGEN GRUPPIERUNGEN**
N-CYANOARYL NITROGEN HETEROCYCLES WITH SULPHUR-CONTAINING GROUPINGS
HETEROCYCLES AZOTES DU N-CYANOARYLE AVEC DES GROUPEMENTS SOUFRES

(30) Priorität: 20.12.1993 DE 4343451; 11.07.1994 DE 4424401
(43) Veröffentlichungstag der Anmeldung: 09.10.1996
(73) Patentinhaber: BAYER AG, 51368 Leverkusen (DE)
(72) Erfinder: ANDREE, Roland, D-40764 Langenfeld (DE); DREWES, Mark, Wilhelm, D-40764 Langenfeld (DE); SANTEL, Hans-Joachim, D-51371 Leverkusen (DE); DOLLINGER, Markus, D-51381 Leverkusen (DE); ERDELEN, Christoph, D-42799 Leichlingen (DE)
(86) Internationale Anmeldenummer: EP9404070
(87) Internationale Veröffentlichungsnummer: WO9517391

(56) Entgegenhaltungen:
- EP-A- 0 408 382
- EP-A- 0 438 209
- EP-A- 0 473 551
- EP-A- 0 563 384
- WO-A-91/00278
- WO-A-92/11244
- DE-A- 4 131 038
- DE-A- 4 237 920
- US-A- 5 084 084
- CHEMICAL ABSTRACTS, vol. 119, no. 11, 1993, Columbus, Ohio, US; abstract no. 117269m, Seite 936 ; & JP,A,0 525 149 (NISSAN CHEMICAL) 2. Februar 1993

## Beschreibung

Die Erfindung betrifft neue N-Cyanoaryl-Stickstoffheterocyclen mit schwefelhaltigen Gruppierungen, mehrere Verfahren zu ihrer Herstellung sowie ihre Verwendung als Herbizide und Insektizide.

Es ist bereits bekannt, daß bestimmte N-Cyanoaryl-Stickstoffheterocyclen herbizide Eigenschaften aufweisen (vgl. WO-A 9100278, WO-A 9211244, EP-A 408382, EP-A 438209, EP-A 473551, DE-A 4237920, US-A 5 084 084, EP-A 0 563 384, DE-A 41 31 038).

Die herbizide Wirkung bzw. die Verträglichkeit der vorbekannten N-Cyanoaryl-Stickstoffheterocyclen gegenüber Kulturpflanzen sind jedoch nicht ganz zufriedenstellend.

Es wurden nun die neuen N-Cyanoaryl-Stickstoffheterocyclen mit schwefelhaltigen Gruppierungen der allgemeinen Formel (I), 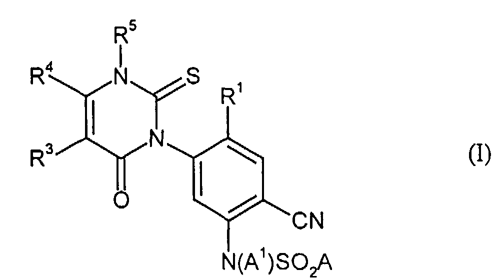 in welcher
- R¹: für Wasserstoff, Fluor, Chlor oder Brom steht,
A für einen jeweils gegebenenfalls durch Fluor, Chlor, Brom, Cyano oder C₁-C₄-Amkoxy substituierten Rest der Reihe Alkyl, Alkenyl;Alkinyl order Dialkylamino mit jeweils bis zu 10 kohlenstoffatomen steht,
A weiterhin für jeweils gegebenenfalls durch Fluor, Chlor, Brom, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl oder Phenyloxy (welche jeweils gegebenenfalls durch Fluor, Chlor, Brom, Cyano, Methyl, Methoxy, Trifluormethyl und/ oder Trifluormethoxy substituiert sind) substituiertes Aryl oder Arylalkyl mit 6 oder 10 Kohlenstoffatomen im Arylteil und gegebenenfalls 1 bis 4 Kohlenstoffatomen im Alkylteil steht, und
A¹ für Wasserstoff oder Formyl, oder für gegebenenfalls durch Fluor und/oder Chlor, Cyano oder C₁-C₄-Alkoxy-carbonyl substituiertes C₁-C₄-Alkyl, oder für jeweils gegebenenfalls durch Fluor, Chlor oder C₁-C₄-Alkoxy substituiertes C₁-C₄-Alkyl-carbonyl oder C₁-C₄-Alkoxy-carbonyl oder für gegebenenfalls durch Fluor und/oder Chlor substituiertes C₁-C₄-Alkylsulfonyl steht, oder
zusammen mit A für Alkandiyl mit 2 bis 8 Kohlenstoffatomen steht,
- R³: für Wasserstoff, Fluor, Chlor, Brom, Cyano oder für gegebenenfalls durch Fluor und/oder Chlor substituiertes Alkyl mit 1 bis 4 Kohlenstoffatomen steht,
- R⁴: für gegebenenfalls durch Fluor, Chlor oder Brom substituiertes Alkyl mit 1 bis 6 Kohlenstoffatomen steht, und
- R⁵: für Wasserstoff oder für jeweils gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl, Alkenyl, Alkinyl oder Alkylcarbonyl mit jeweils bis zu 6 Kohlenstoffatomen steht,
sowie Salze von Verbindungen der Formel (I) gefunden.

Man erhält die neuen N-Cyanoaryl-Stickstoffheterocyclen mit schwefelhaltigen Gruppierungen der allgemeinen Formel (I), wenn man
(a) Aminoalkensäure(thio)ester der allgemeinen Formel (II) 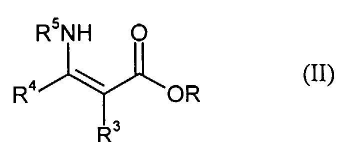 in welcher
   - R³, R⁴ und R⁵: die oben angegebenen Bedeutungen haben und
   - R: für Alkyl steht,
   mit Cyanoarylisothiocyanaten der allgemeinen Formel (III) 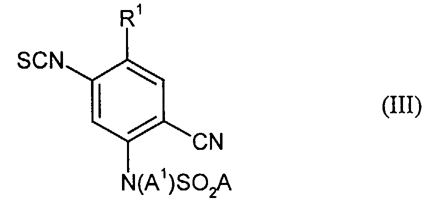 in welcher
   - R¹, A¹ and A: die oben angegebene Bedeutung hat,
   gegebenenfalls in Gegenwart eines Reaktionshilfsmittels und gegebenenfalls in Gegenwart eines Verdünnungsmittels umsetzt,
   oder wenn man
(b) zur Herstellung von Verbindungen der Formel (I), in welcher R⁵ für jeweils gegebenenfalls substituiertes Alkyl, Alkenyl oder Alkinyl mit jeweils bis zu 6 Kohlenstoffatomen steht sowie R¹, R³ und R⁴ die oben angegebenen Bedeutungen haben,
   N-Cyanoaryl-Stickstoffheterocyclen der allgemeinen Formel (I)
   in welchen R⁵ für Wasserstoff steht sowie R¹, R³ und R⁴ die oben angegebenen Bedeutungen haben,
   mit Alkylierungsmitteln der allgemeinen Formeln (IV) oder (V)

   X-R⁵ (IV)

   R⁵-O-SO₂-O-R⁵ (V)

   in welchen R⁵ für jeweils gegebenenfalls substituiertes Alkyl, Alkenyl oder Alkinyl steht,
   gegebenenfalls in Gegenwart eines Säureakzeptors und gegebenenfalls in Gegenwart eines Verdünnungsmittels umsetzt,
   oder wenn man
(c) zur Herstellung von Verbindungen der Formel (I), in welcher A, A¹, R¹, R³ und R⁴ die oben angegebenen Bedeutungen haben,
   N-Cyanoaryl-Stickstoffheterocyclen der allgemeinen Formel (IA), 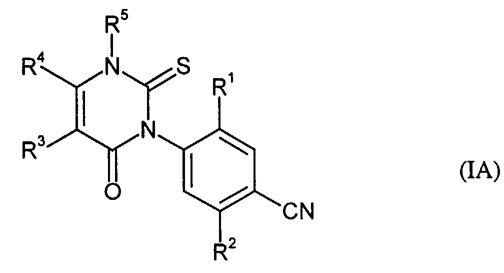 in welcher
   - R²: für Halogen steht sowie R¹, R³, R⁴ und R⁵ die oben angegebenen Bedeutungen haben,
   mit Amiden der allgemeinen Formel (VI)

   HN(A¹)SO₂A (VI)

   in welcher
   - A und A¹: die oben angegebenen Bedeutungen haben,
   gegebenenfalls in Gegenwart eines Säureakzeptors und gegebenenfalls in Gegenwart eines Verdünnungsmittels umsetzt.

Die Verbindungen der Formel (I) können analog Verfahren (a) auch durch Umsetzung von Aminoalkensäure(thio)estem der Formel (II) - oben - mit Cyanoarylthiourethanen der Formel (IIIa) 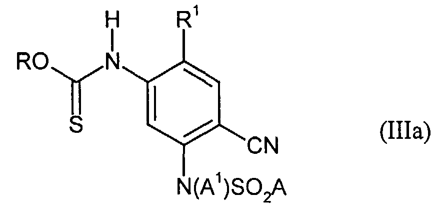 in welcher
- R¹, A¹ and A: die oben angegebene Bedeutung hat und
- R: für Methyl oder Phenyl steht,
erhalten werden.

Die neuen N-Cyanoaryl-Stickstoffheterocyclen mit schwefelhaltigen Gruppierungen der allgemeinen Formel (I) zeichnen sich durch starke herbizide Wirksamkeit aus.

In den Definitionen sind die gesättigten oder ungesättigten Kohlenwasserstoffketten, wie Alkyl, Alkandiyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I), in welcher
- R¹: für Wasserstoff, Fluor oder Chlor steht,
A für einen jeweils gegebenenfalls durch Fluor oder Chlor substituierten Rest der Reihe Methyl, Ethyl, n- oder i-Propyl, n-, i-, s- oder t-Butyl, Ethenyl, Propenyl, Butenyl, oder für Dimethylamino steht,
A weiterhin für jeweils gegebenenfalls durch Fluor, Chlor, Brom, Methyl und/oder Ethyl substituiertes Cyclopropyl, Cyclopentyl, Cyclohexyl, Cyclopropylmethyl, Cyclopentylmethyl oder Cyclohexylmethyl steht,
A weiterhin für jeweils gegebenenfalls durch Fluor, Chlor, Brom, Cyano, Nitro, Carboxy, Methyl, Trifluormethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl, Dimethylaminosulfonyl, Methoxycarbonyl, Ethoxycarbonyl, Propoxycarbonyl oder Phenyl substituiertes Phenyl, Naphthyl, Phenylmethyl oder Phenylethyl steht, und
A¹ für Wasserstoff, Formyl, Methyl, Ethyl, Acetyl, Propionyl, Methoxycarbonyl, Ethoxycarbonyl, Methylsulfonyl oder Ethylsulfonyl steht, oder
zusammen mit A für Trimethylen oder Tetramethylen steht,
- R³: für Wasserstoff, Fluor, Chlor, Brom, Methyl, Trifluormethyl, Ethyl, n- oder i-Propyl steht,
- R⁴: für jeweils gegebenenfalls durch Fluor oder Chlor substituiertes Methyl, Ethyl, n- oder i-Propyl steht,
oder zusammen mit R³ für Trimethylen oder Tetramethylen steht, und
- R⁵: für Wasserstoff, Methyl, Difluormethyl, Cyanomethyl, Ethyl, Fluorethyl, Chlorethyl, Difluorethyl, Dichlorethyl, Trifluorethyl, Chlordifluorethyl, Cyanoethyl, n- oder i-Propyl, Fluorpropyl, Chlorpropyl, Cyanopropyl, Allyl, Chlorallyl, Propargyl, Acetyl, Propionyl, Fluoracetyl, Chloracetyl, Difluoracetyl, Dichloracetyl, Trifluoracetyl oder Trichloracetyl steht,
Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Beispiele für die erfindungsgemäßen Verbindungen der Formel (II) sind in der nachstehenden Tabelle 1 aufgeführt.

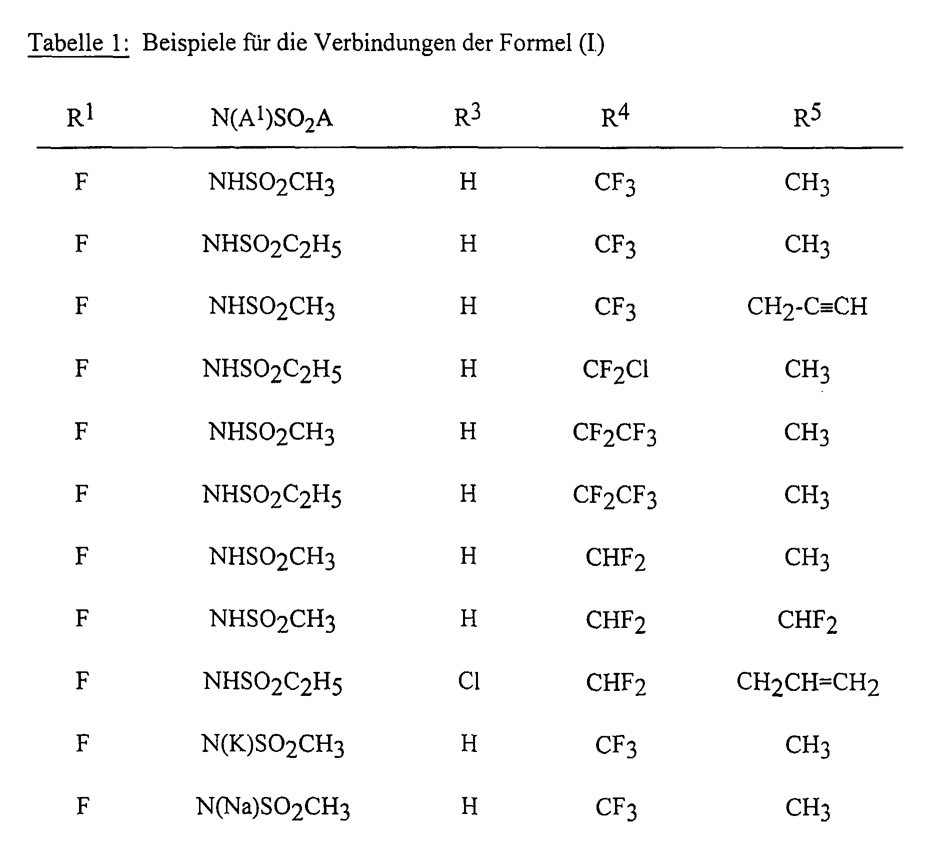

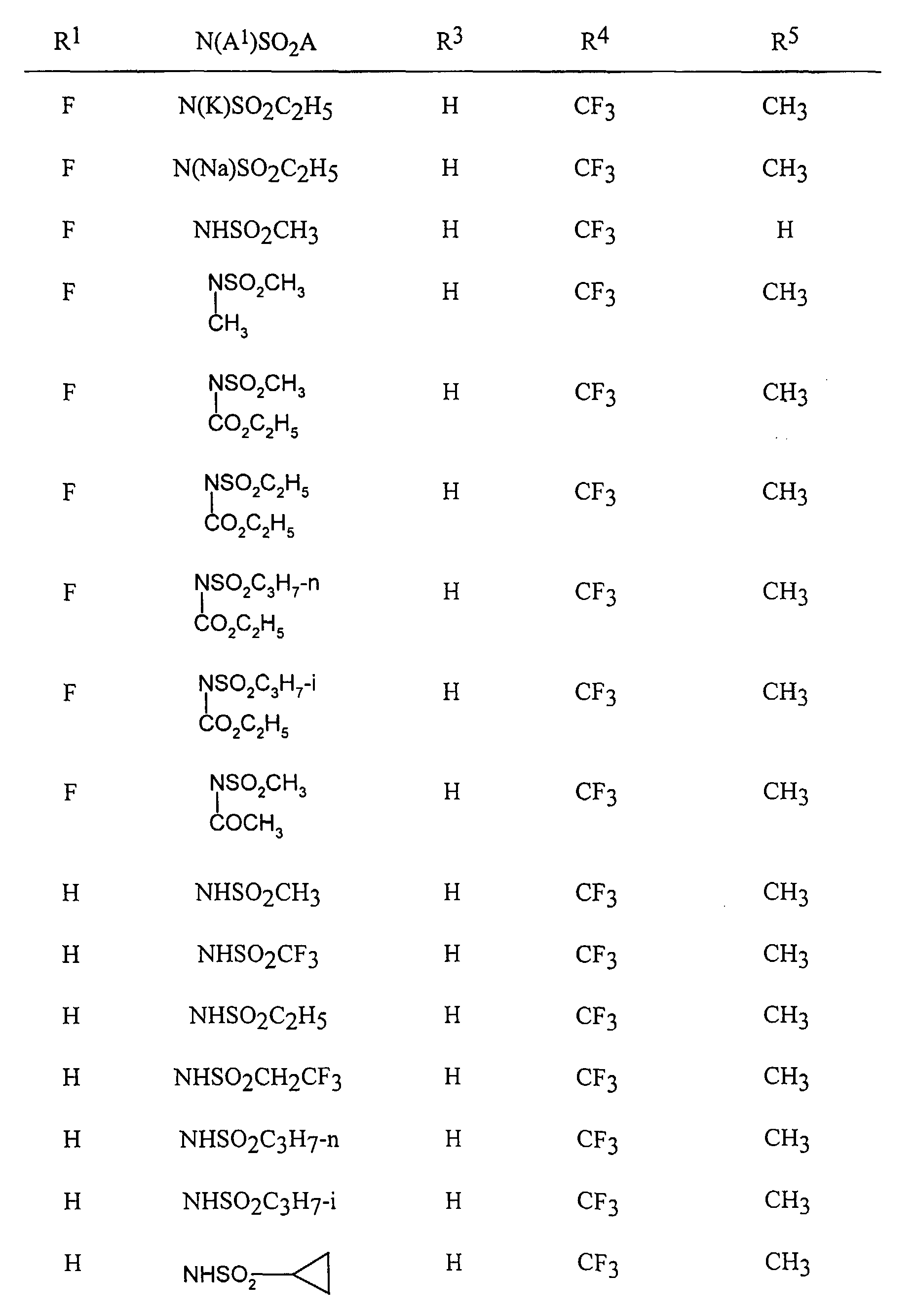

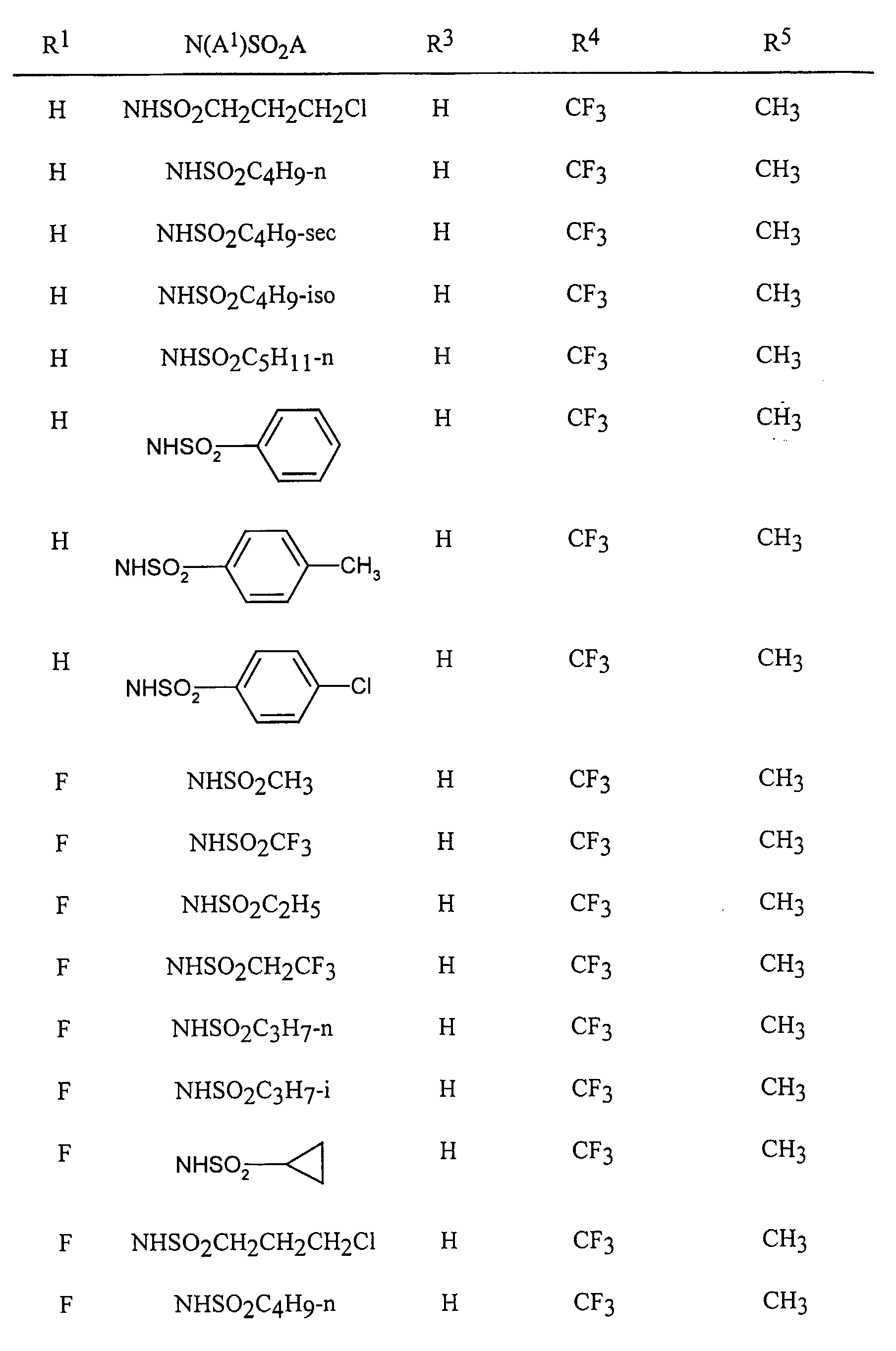

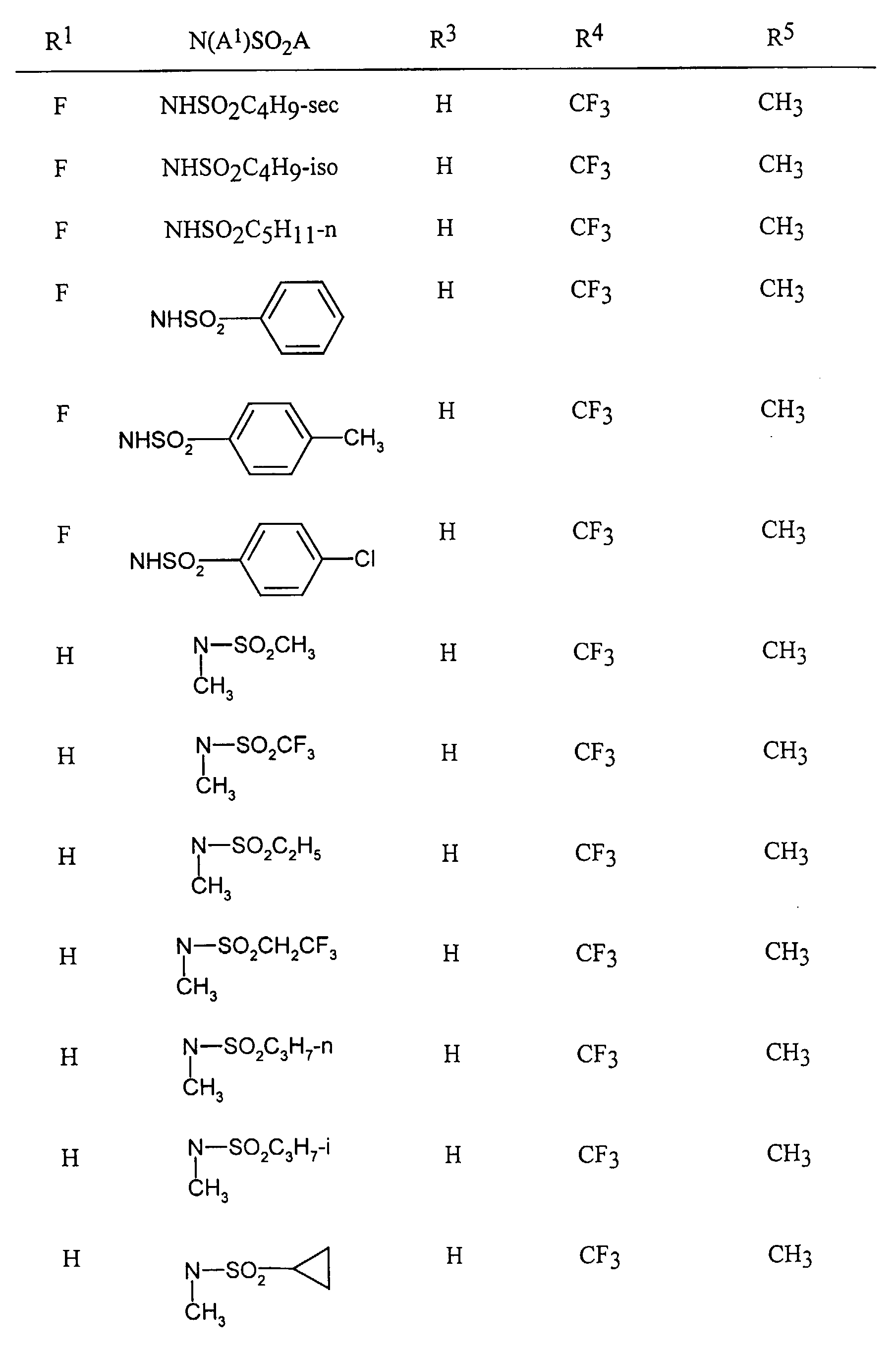

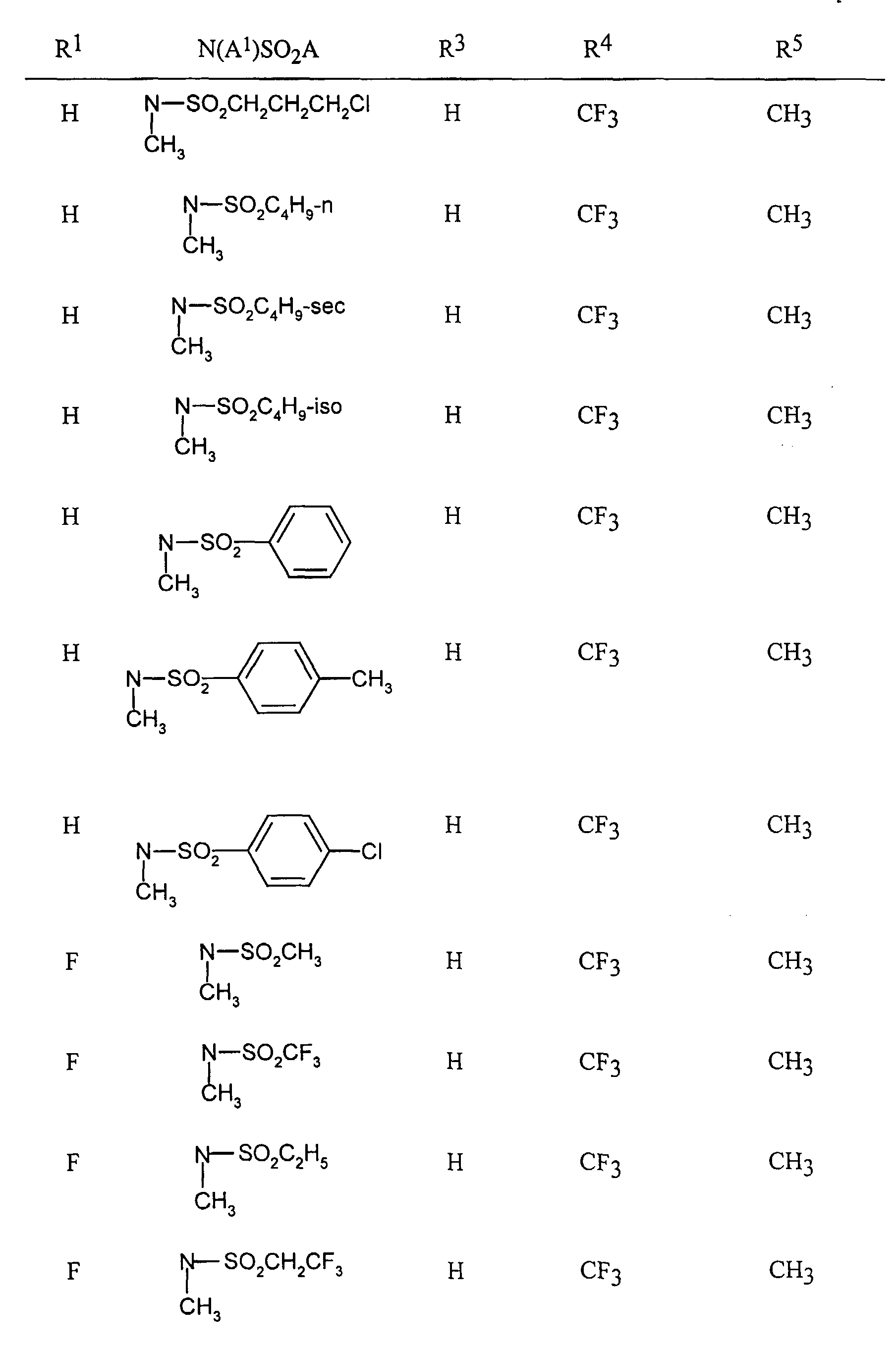

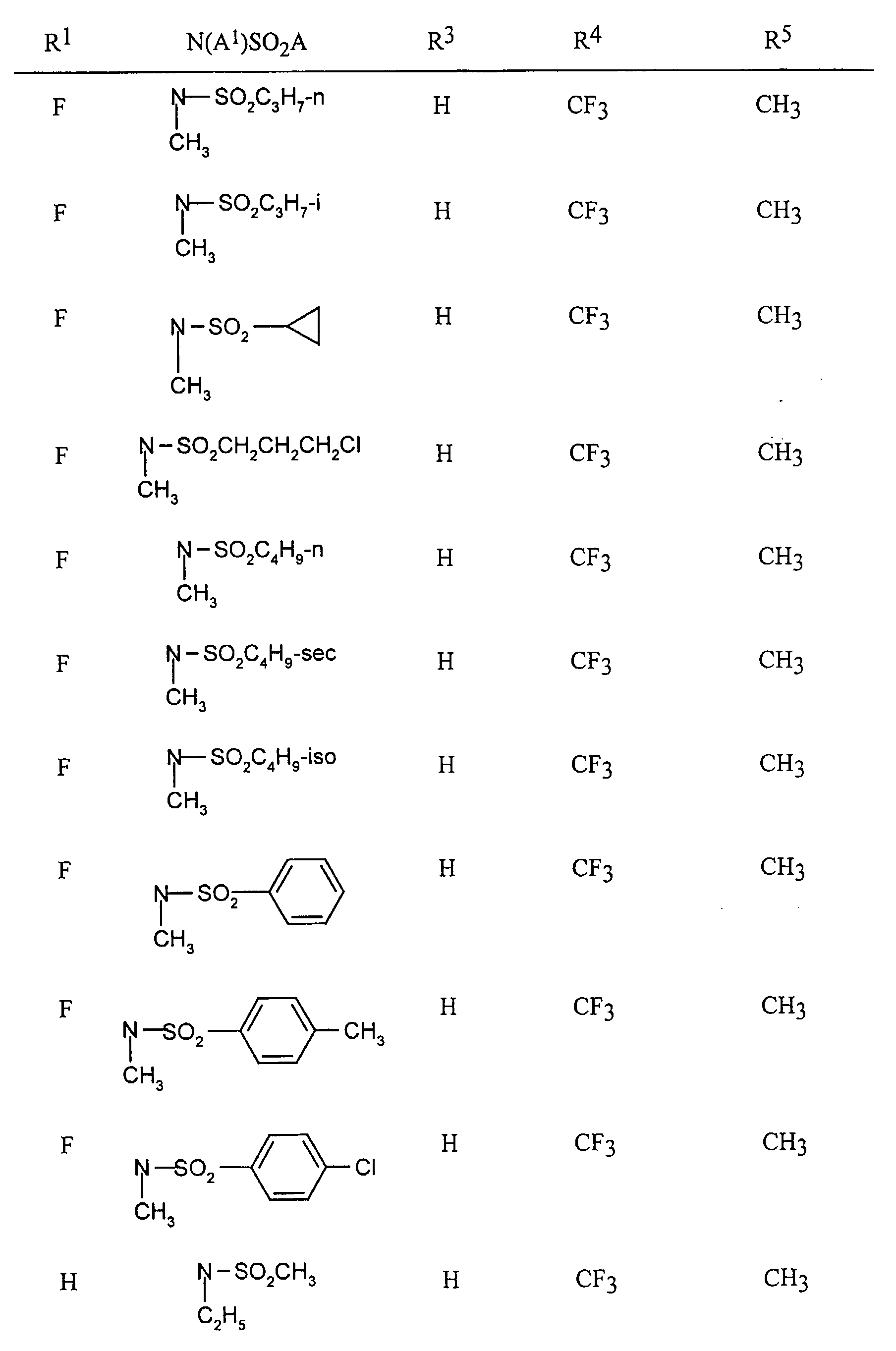

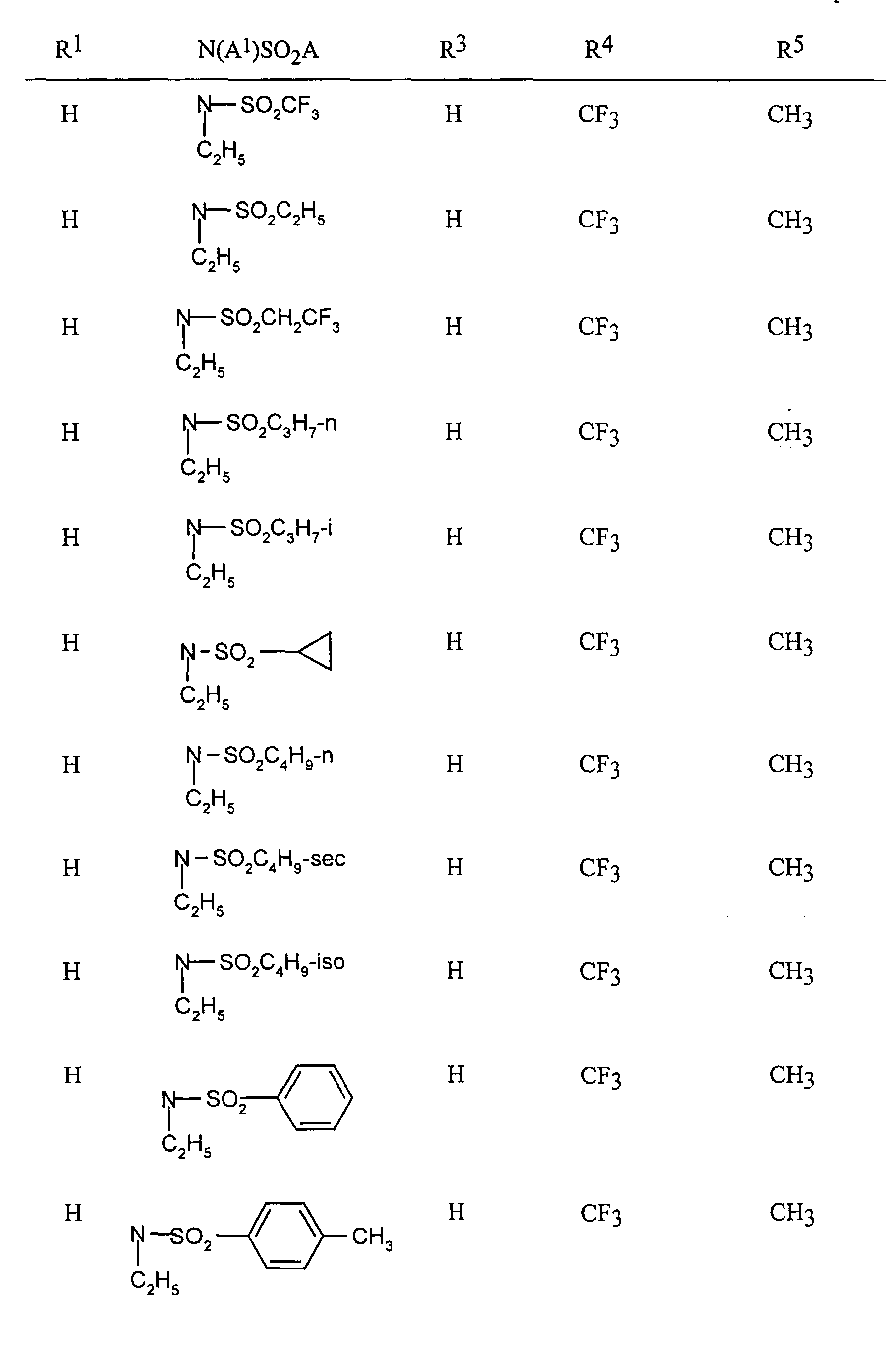

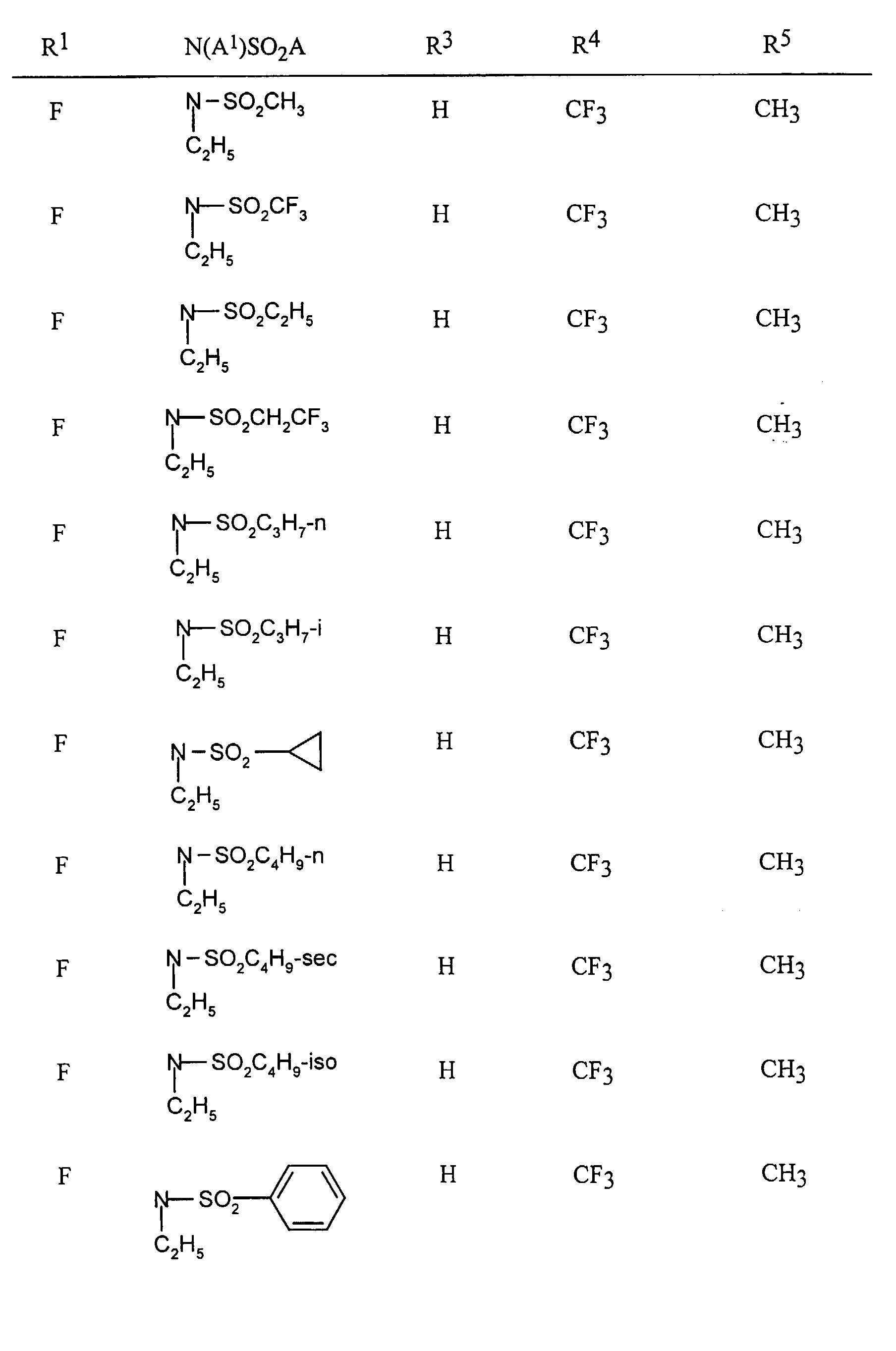

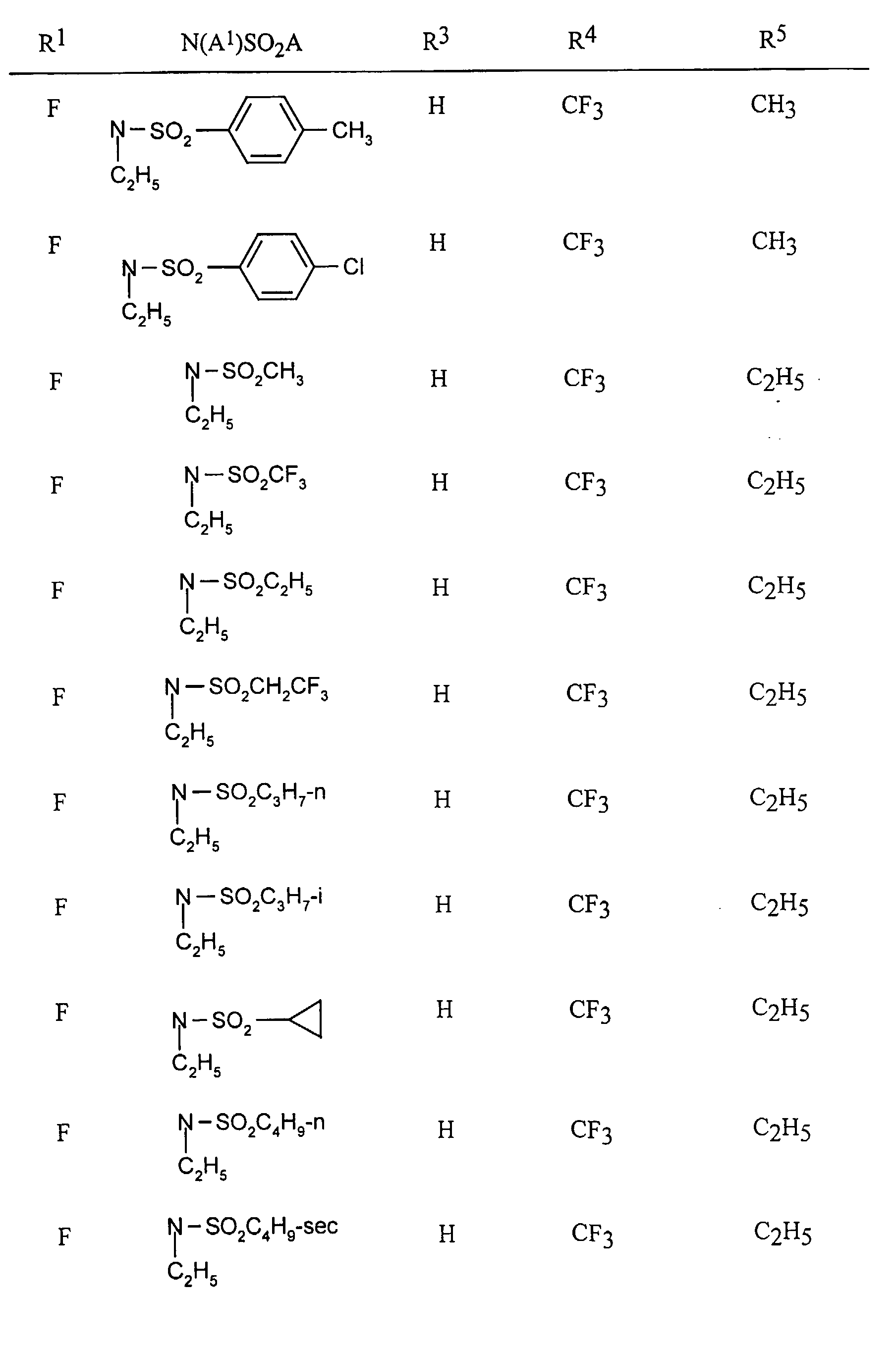

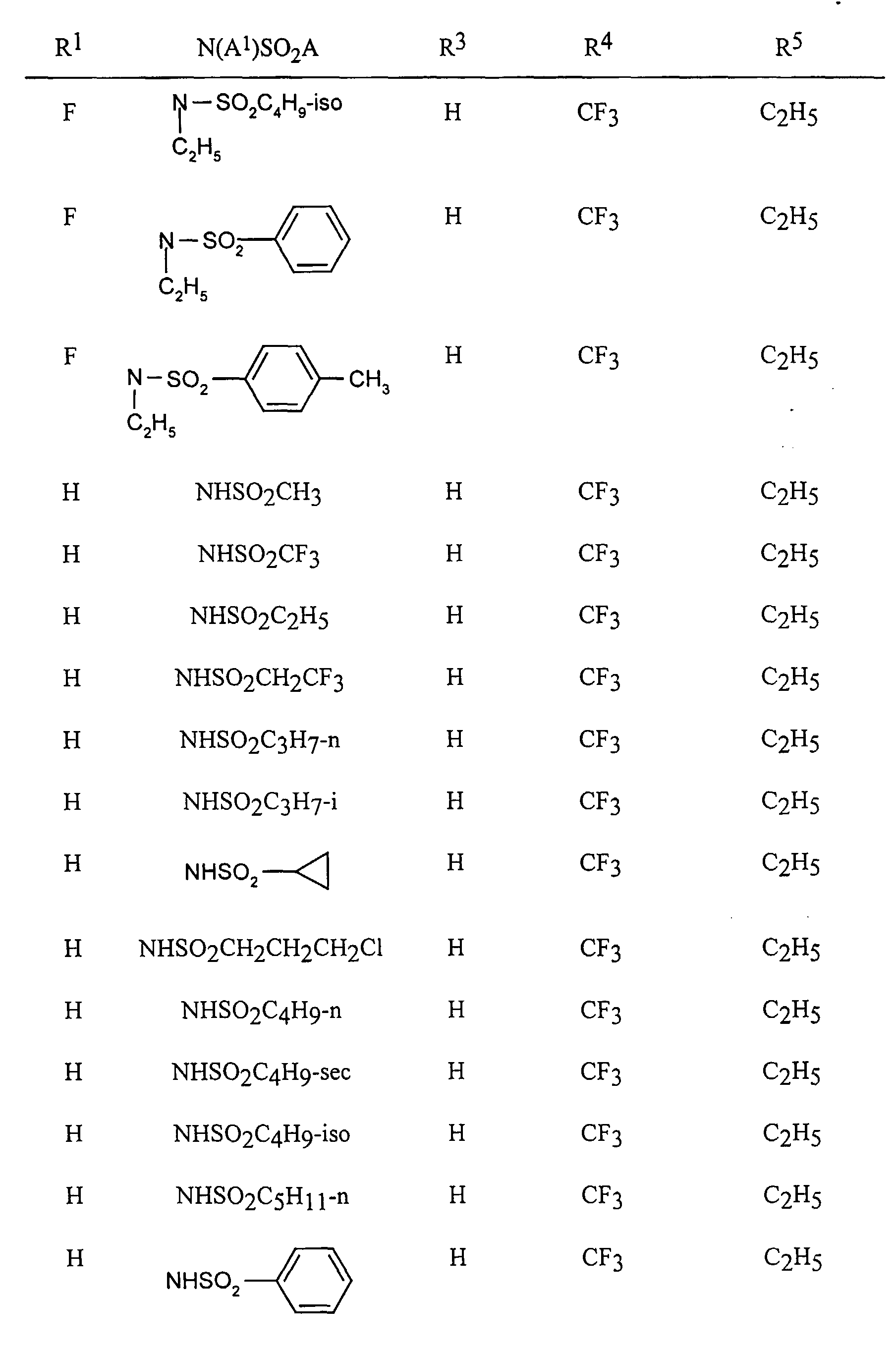

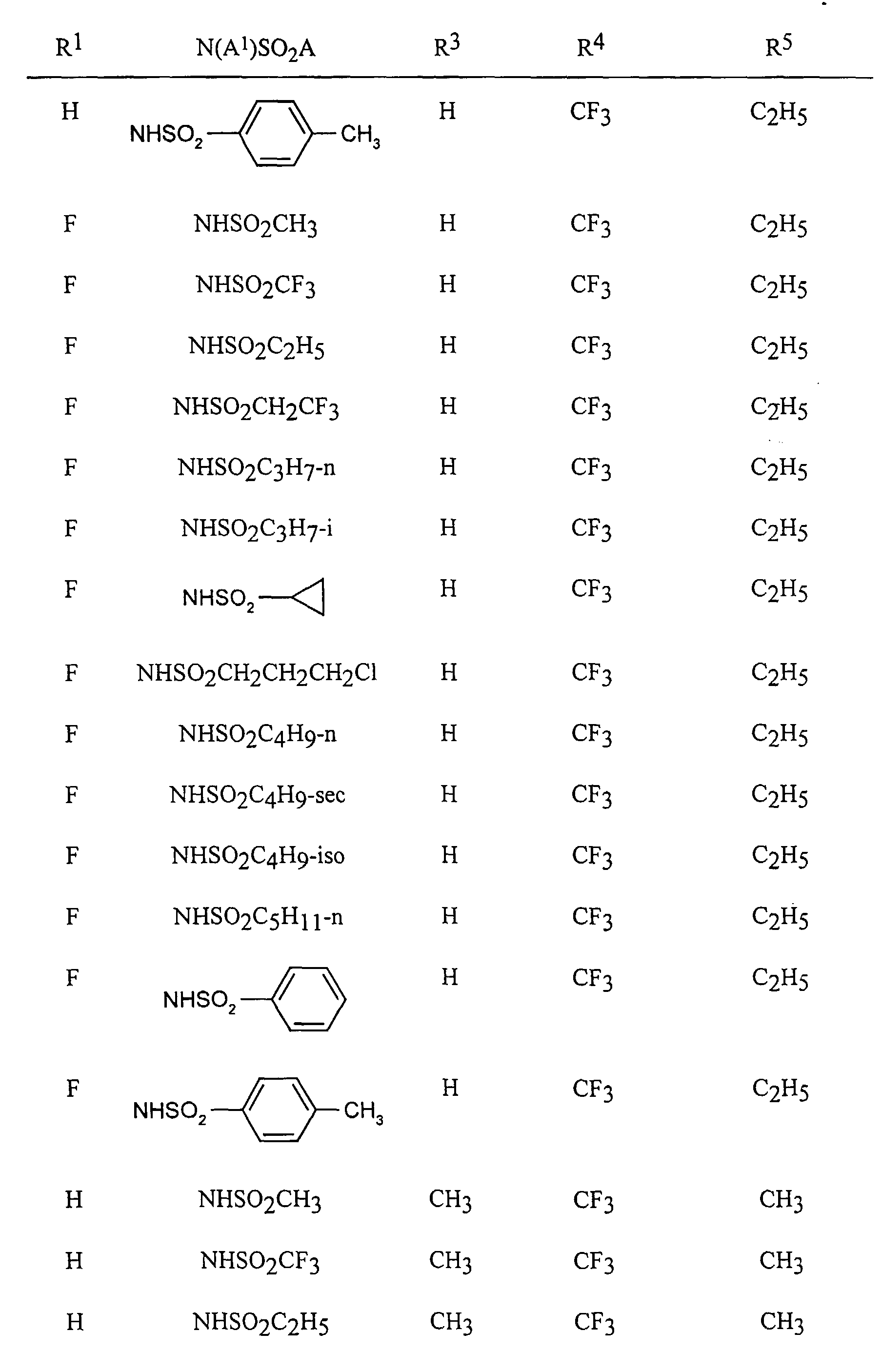

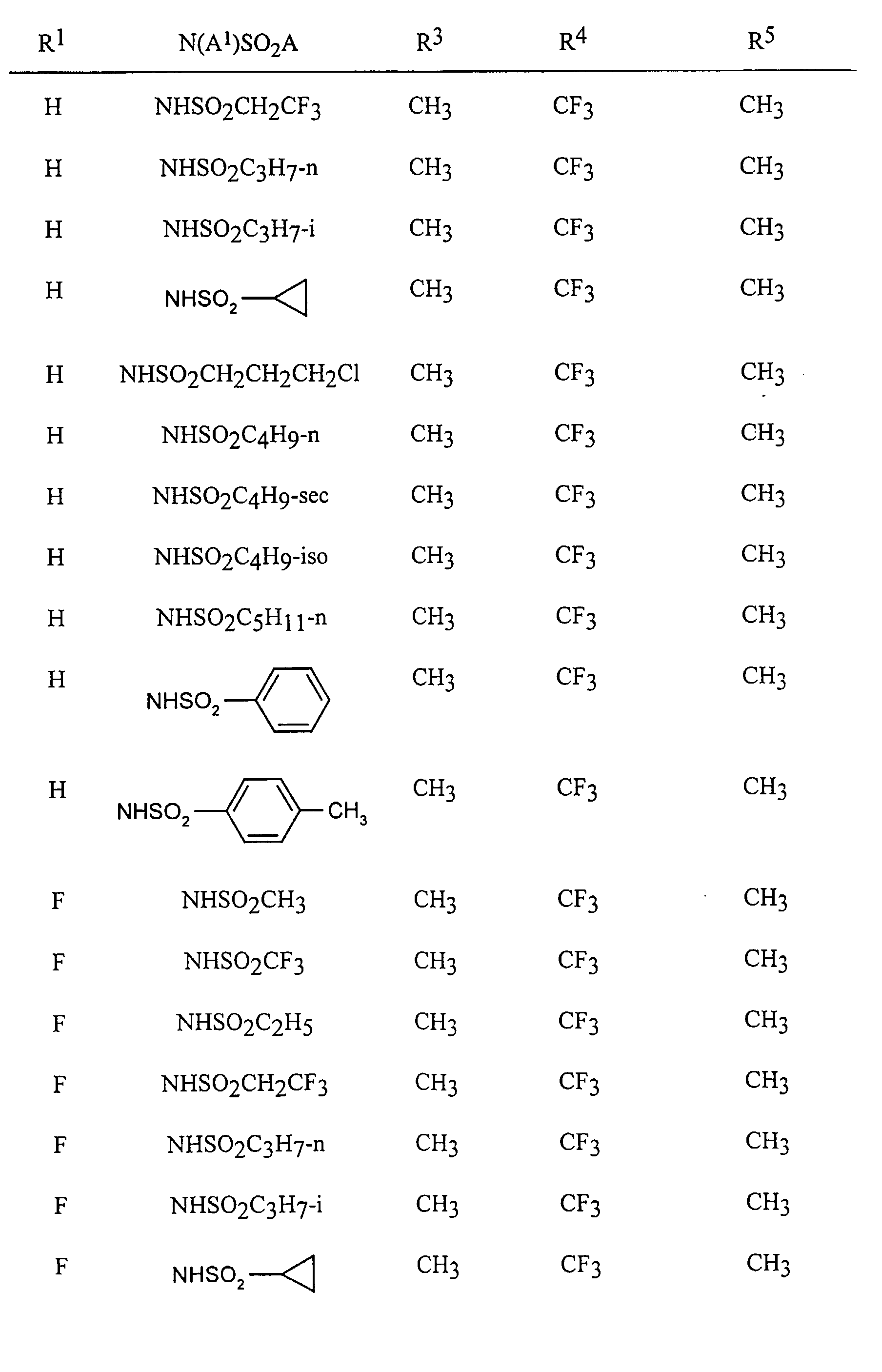

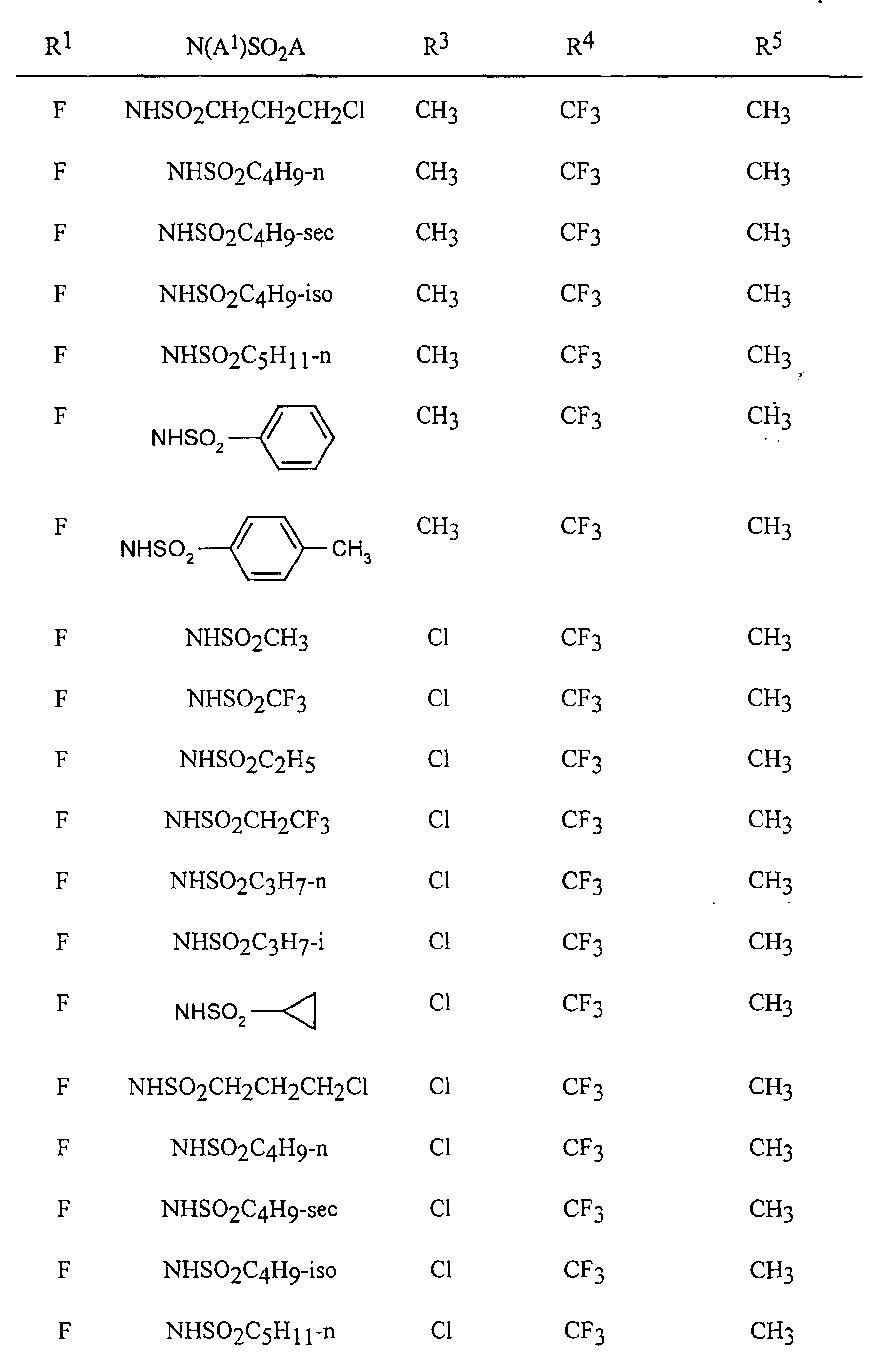

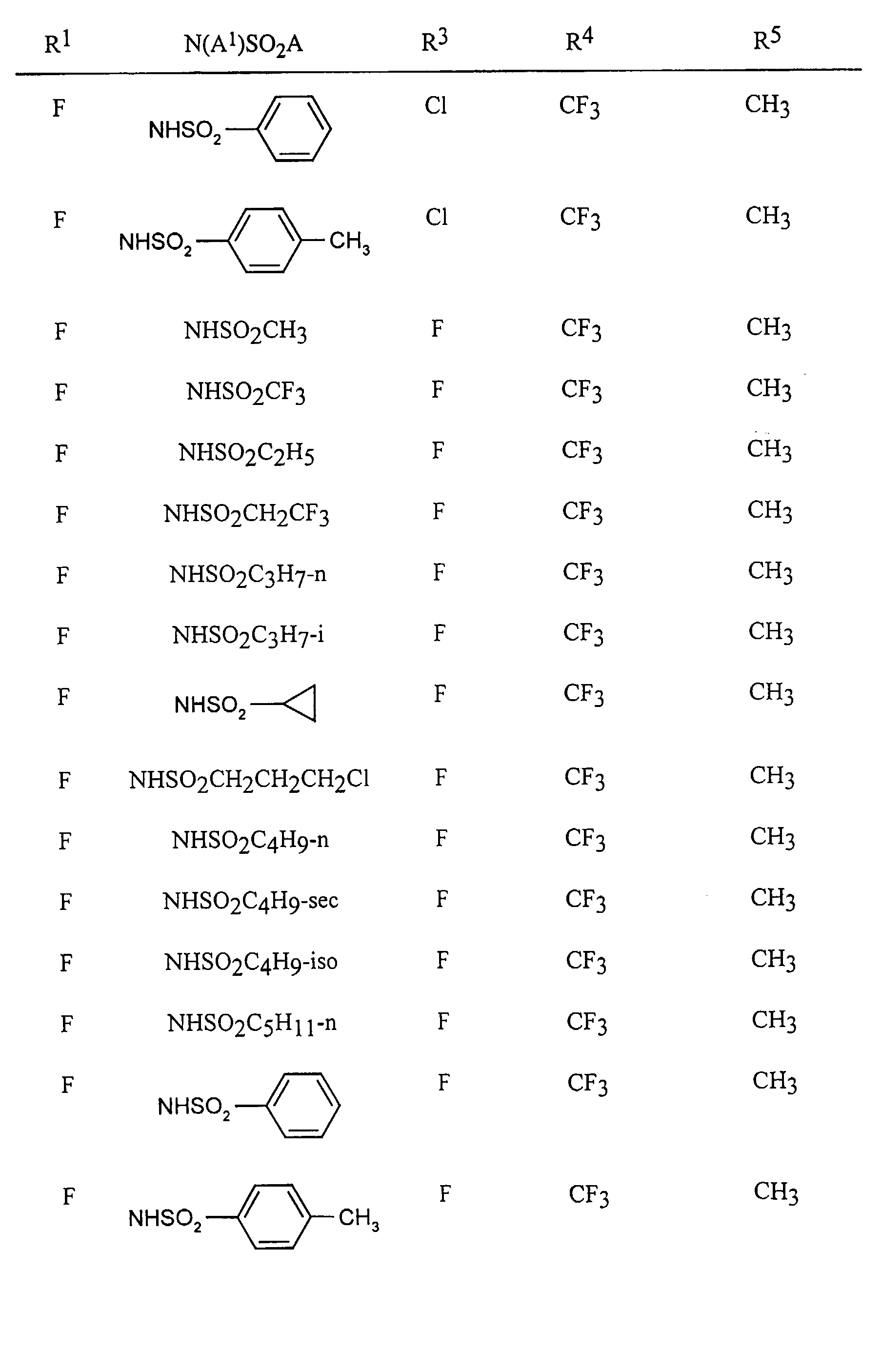

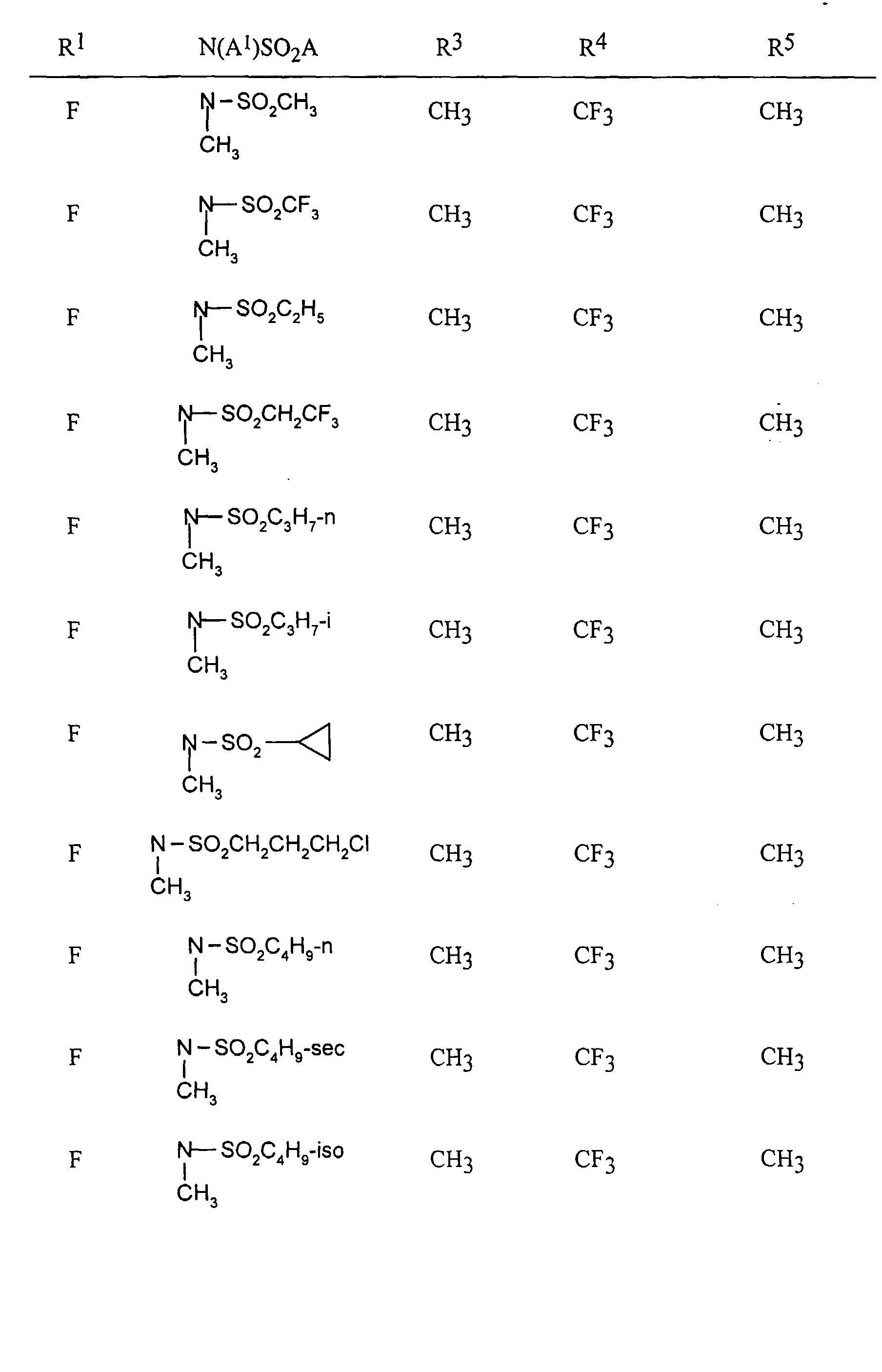

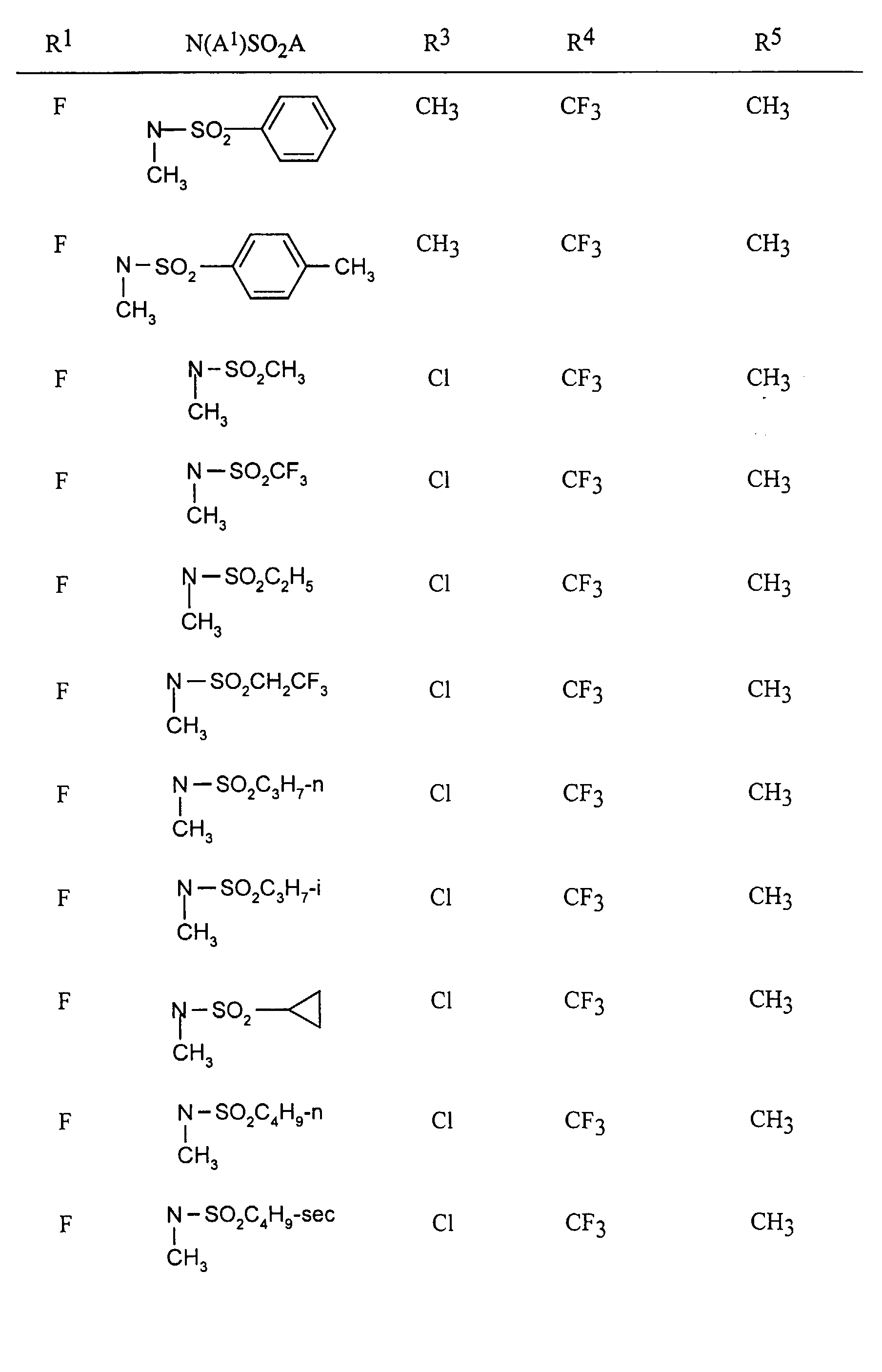

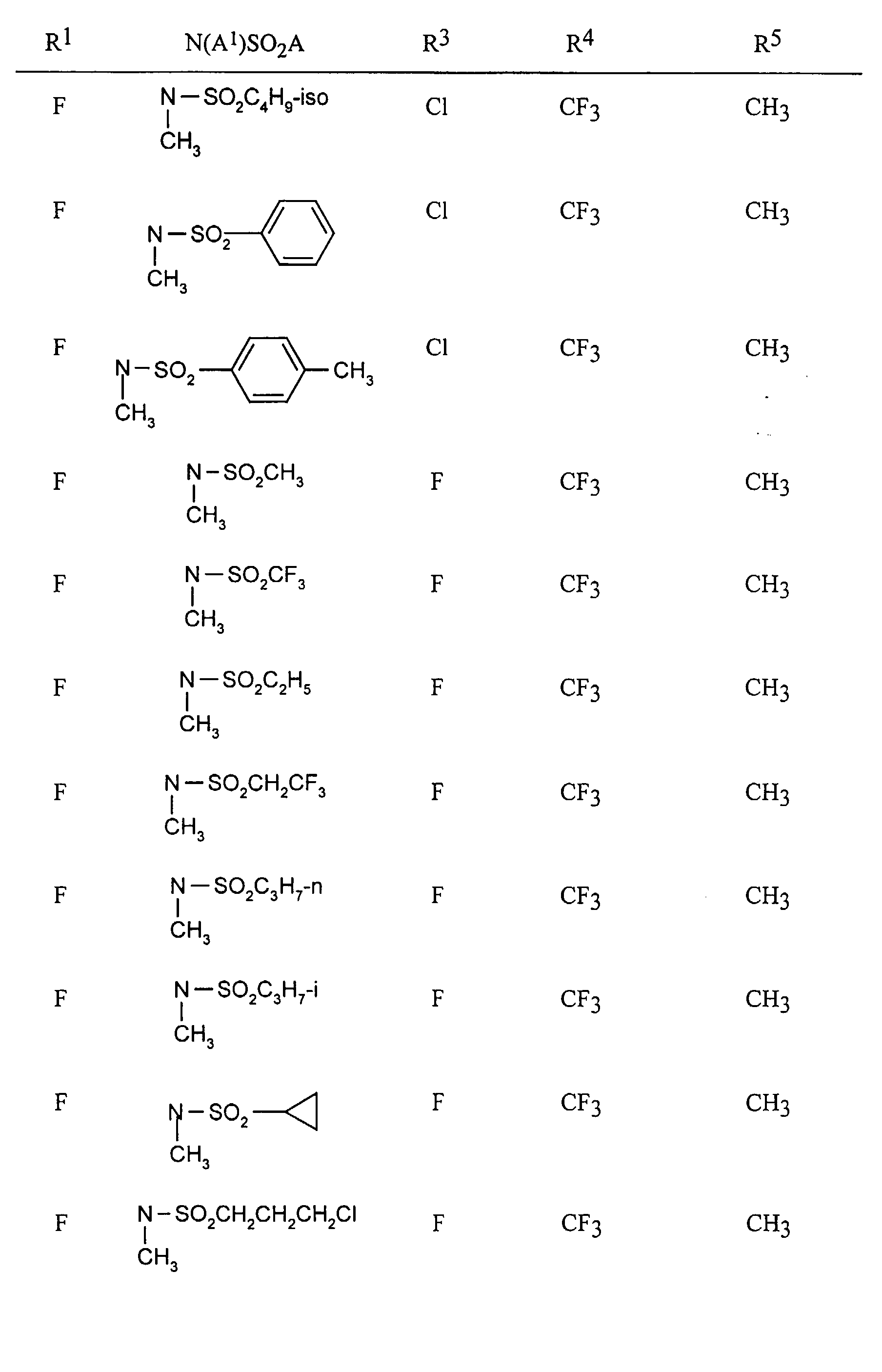

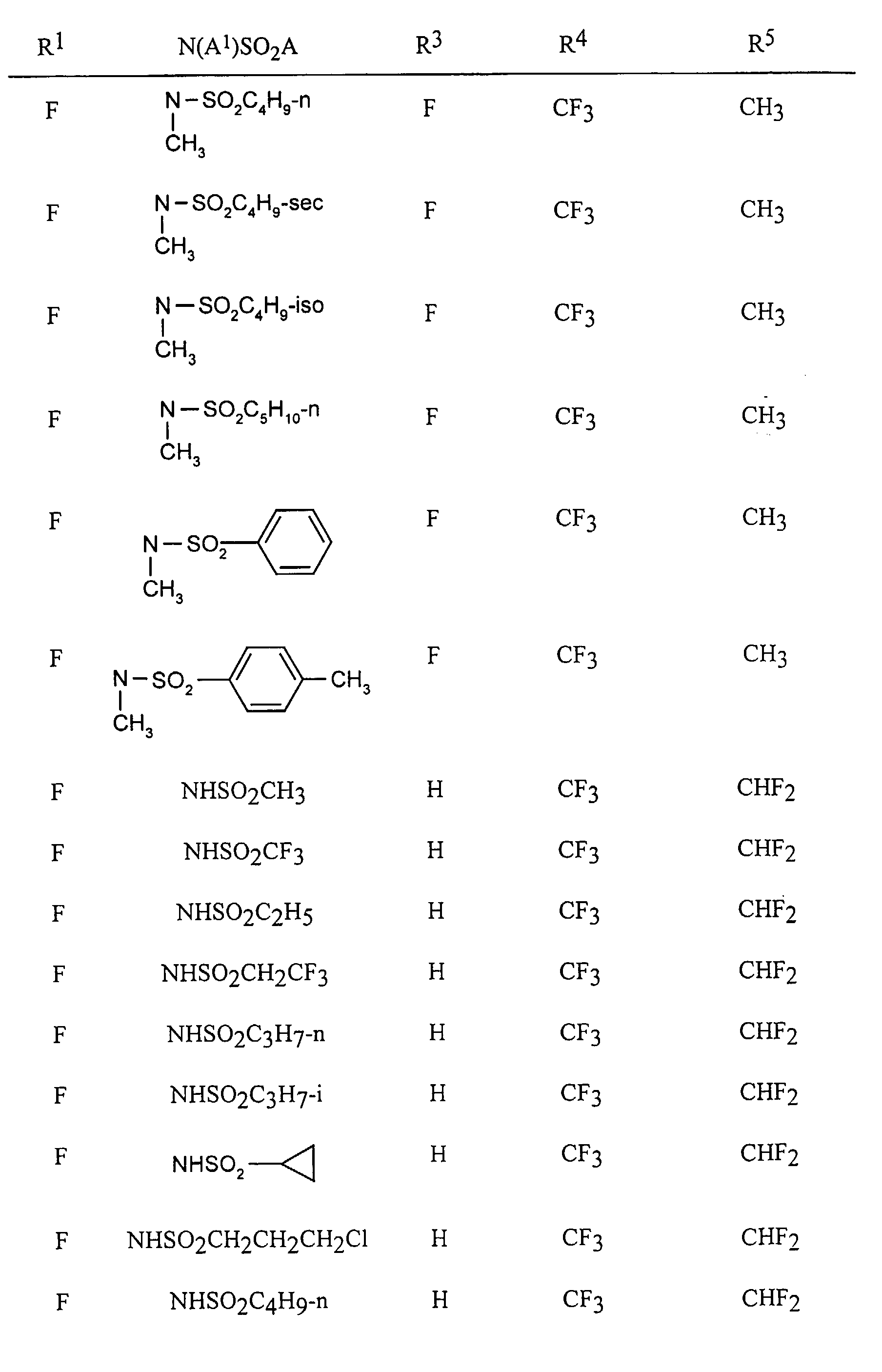

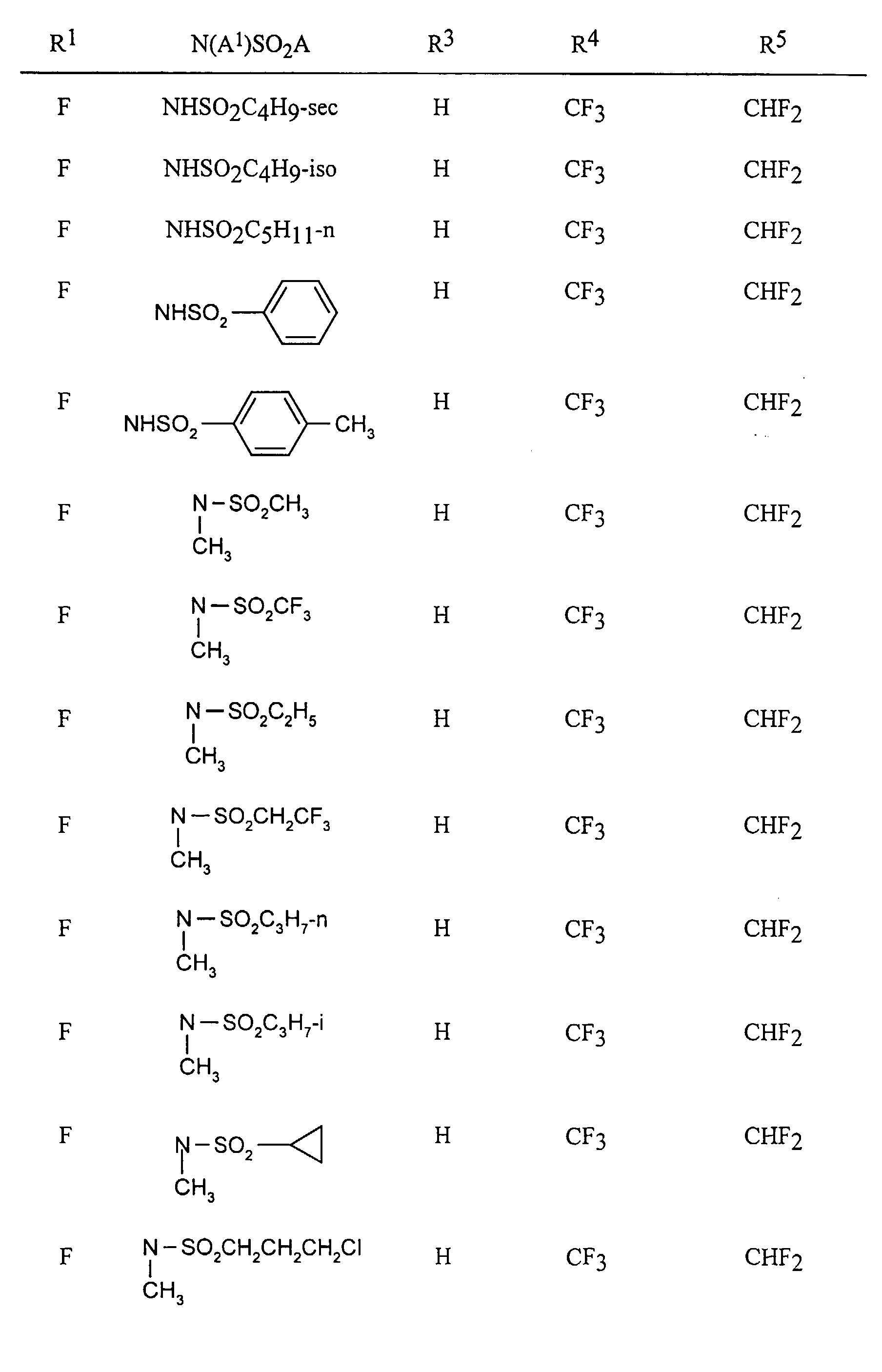

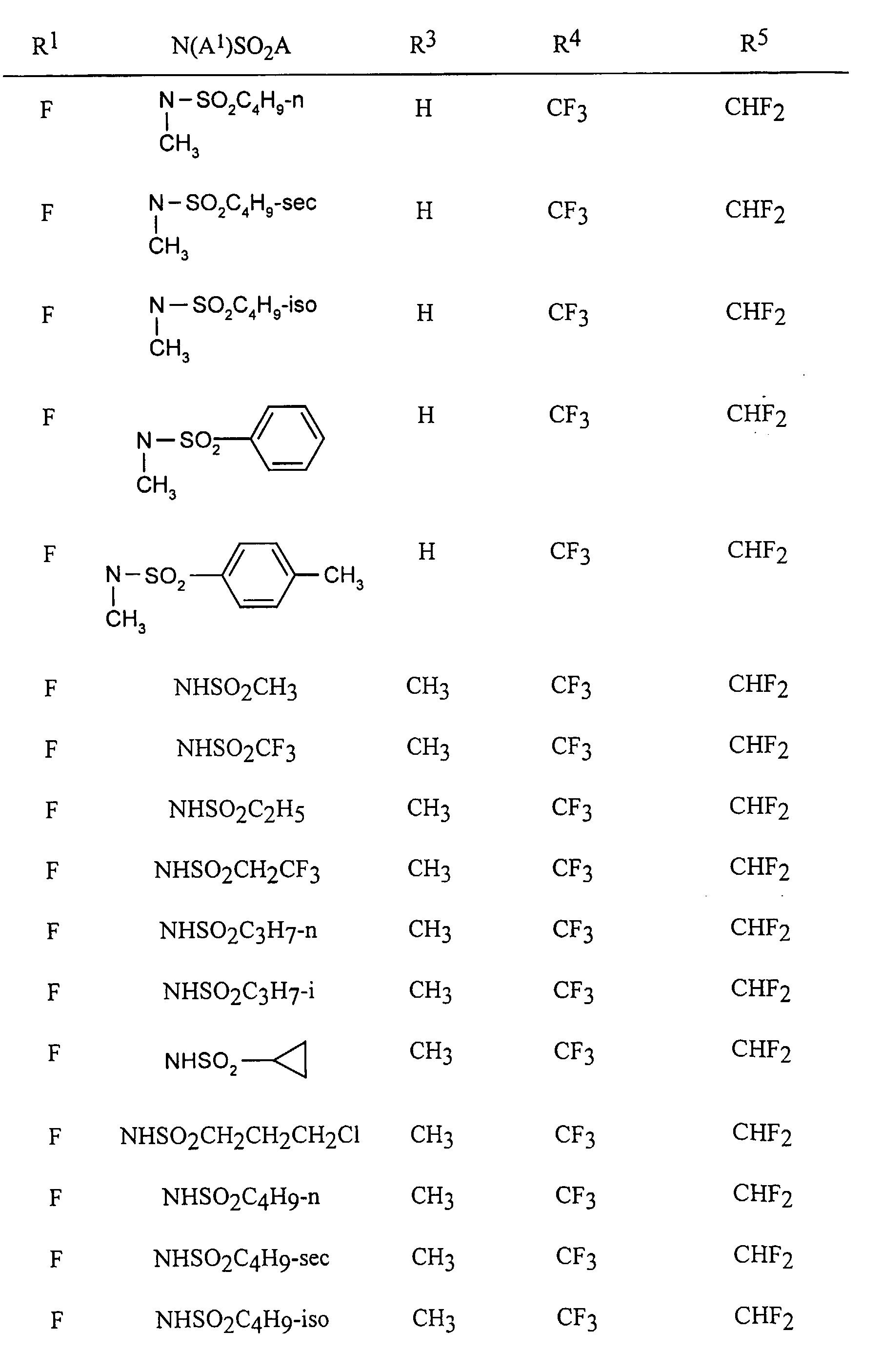

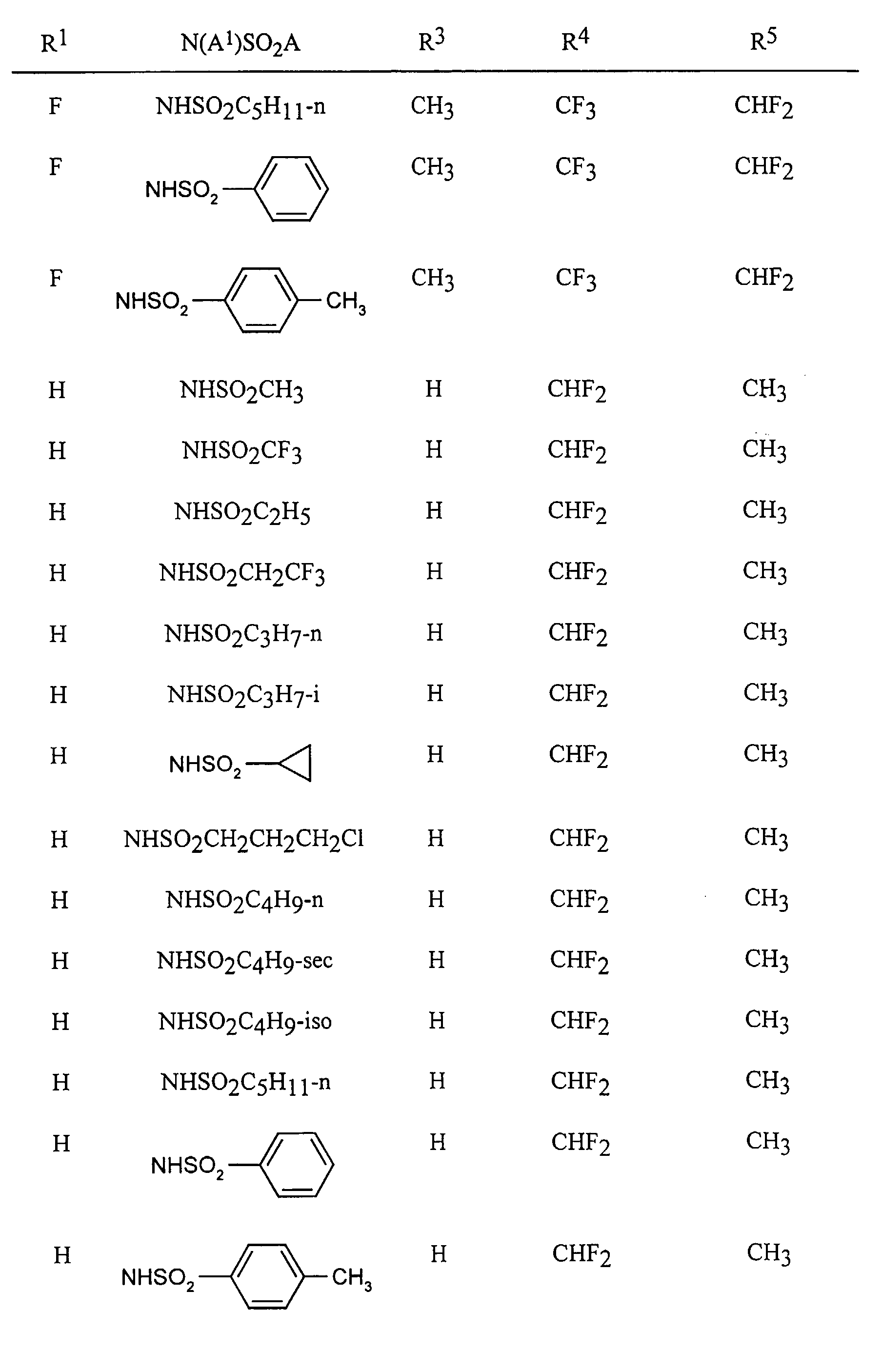

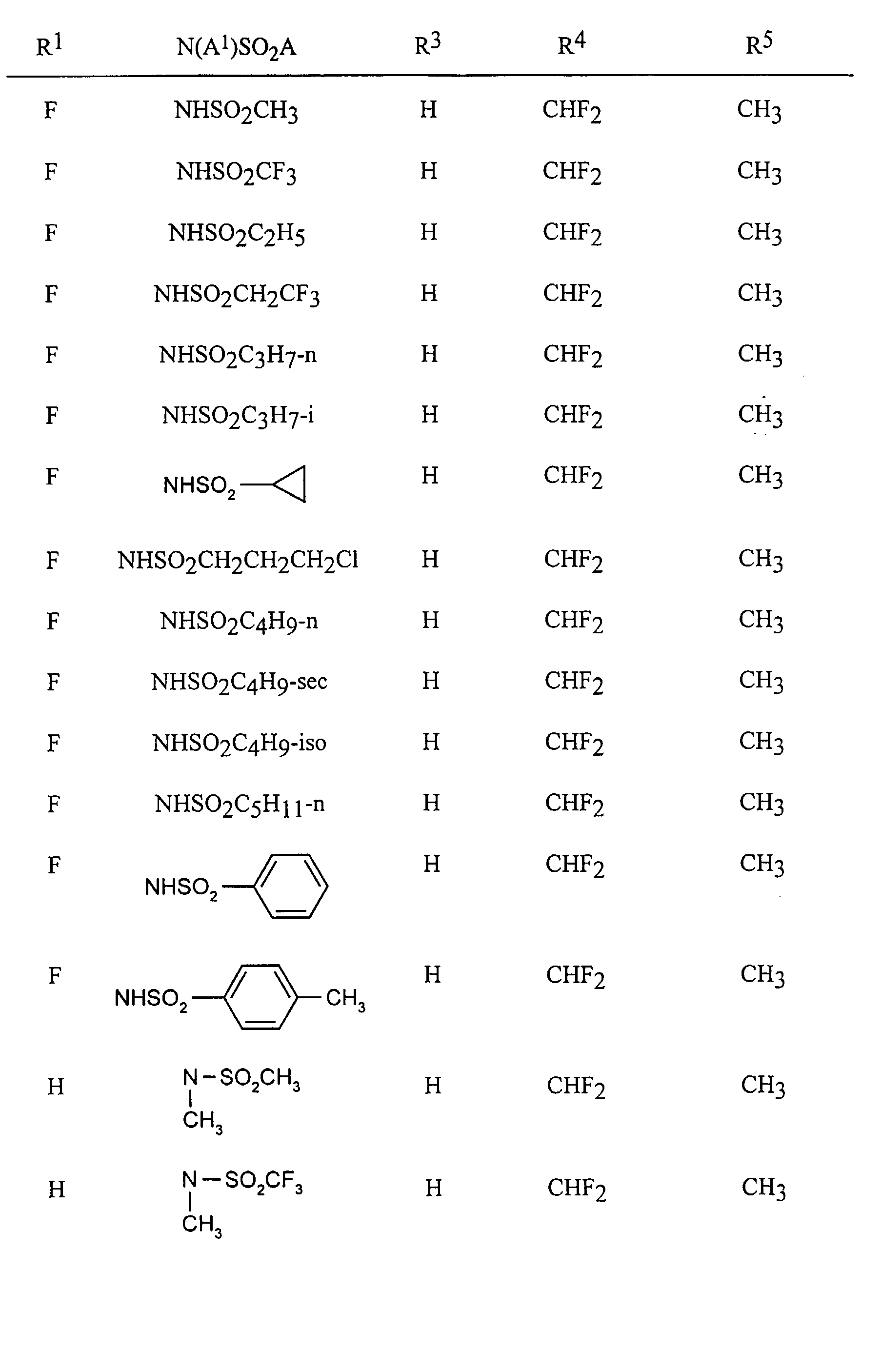

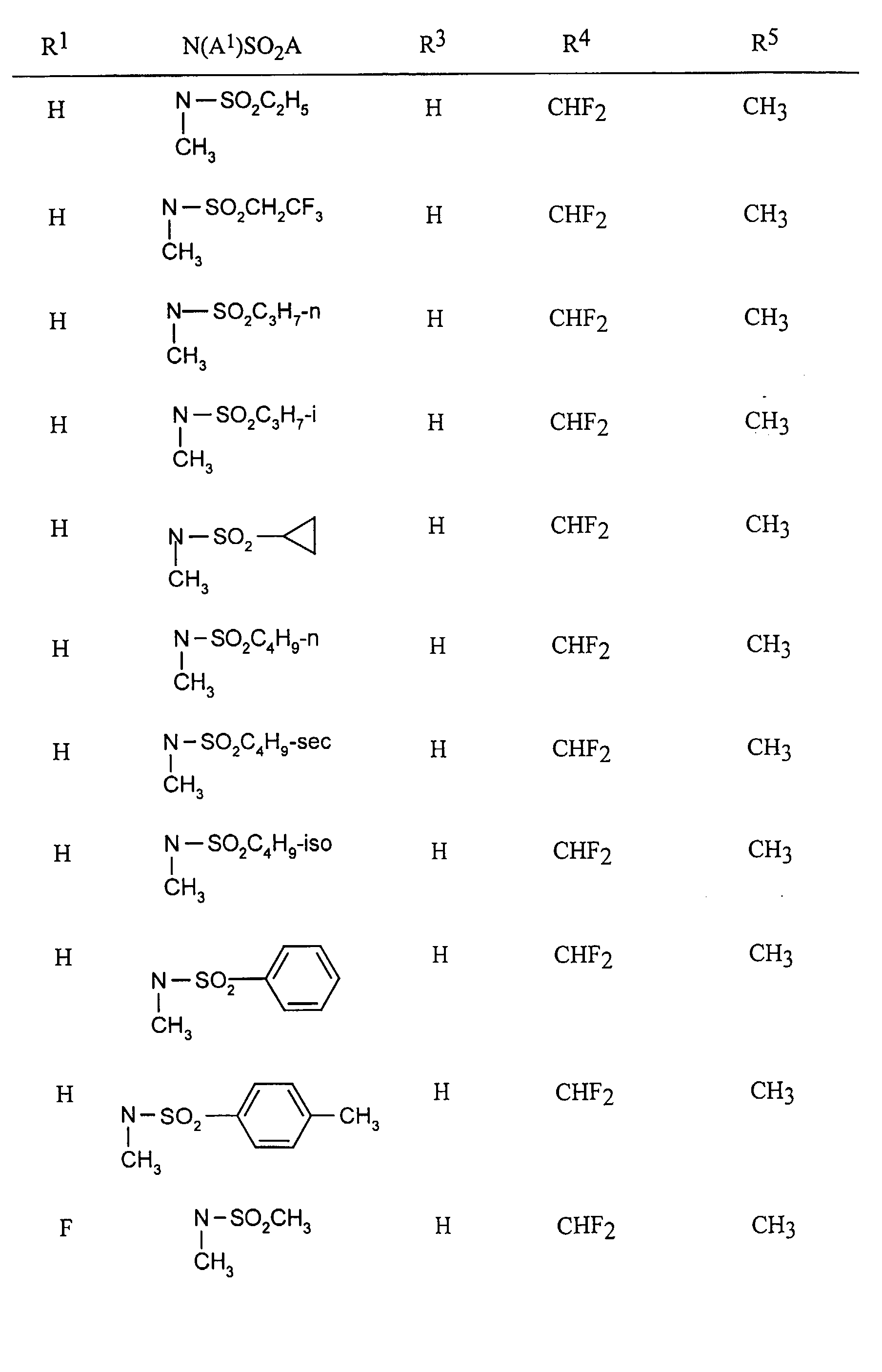

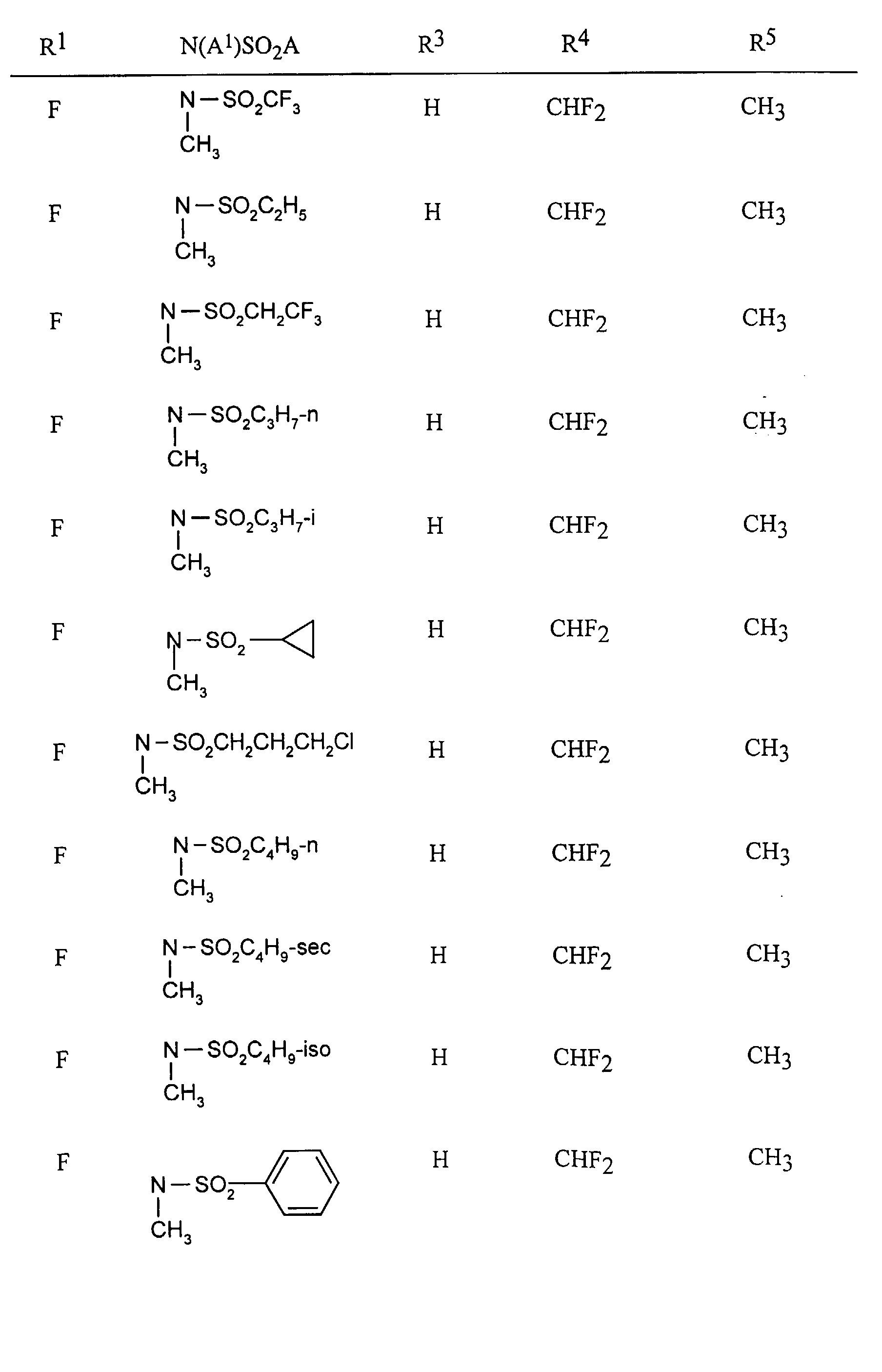

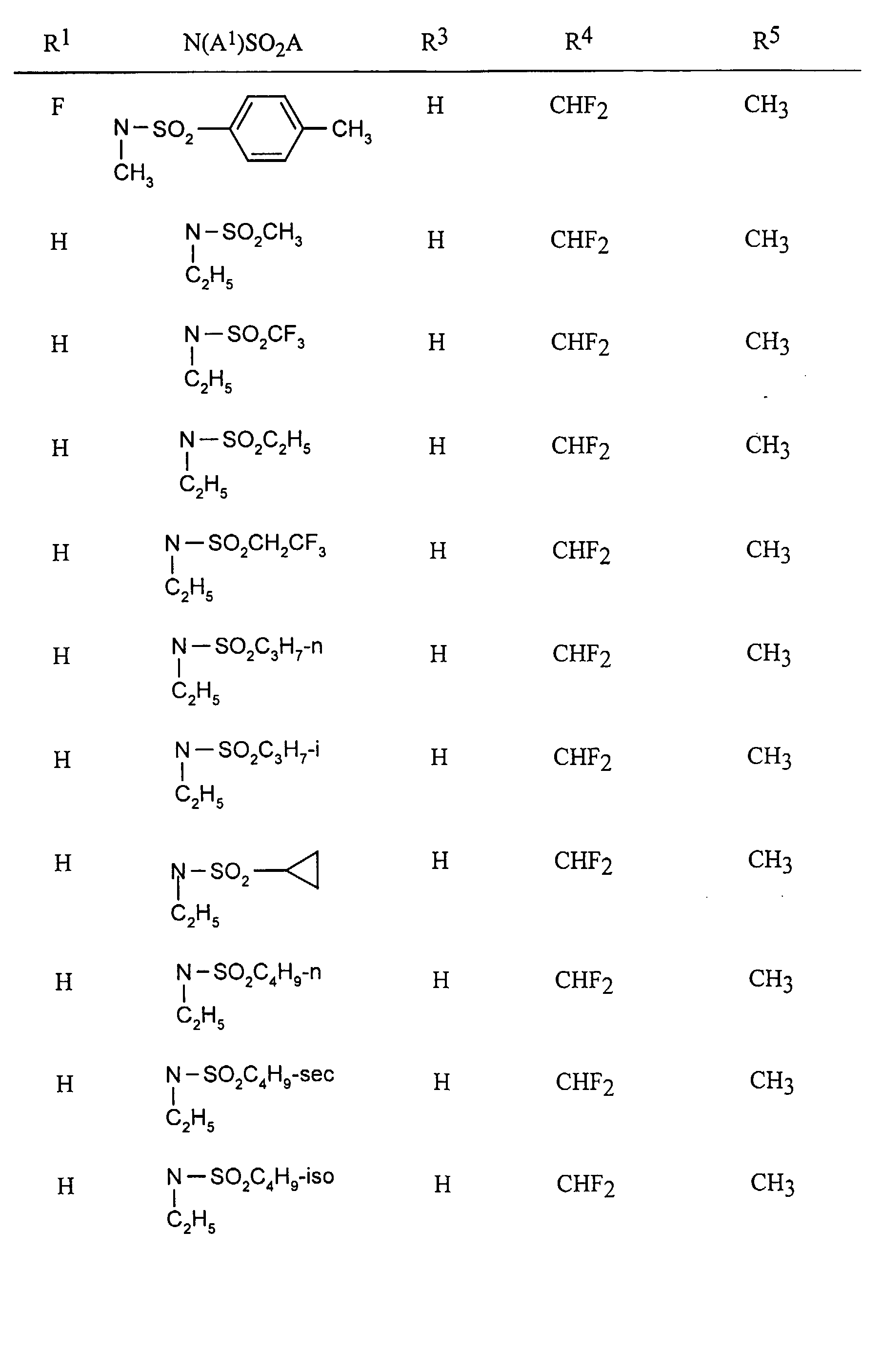

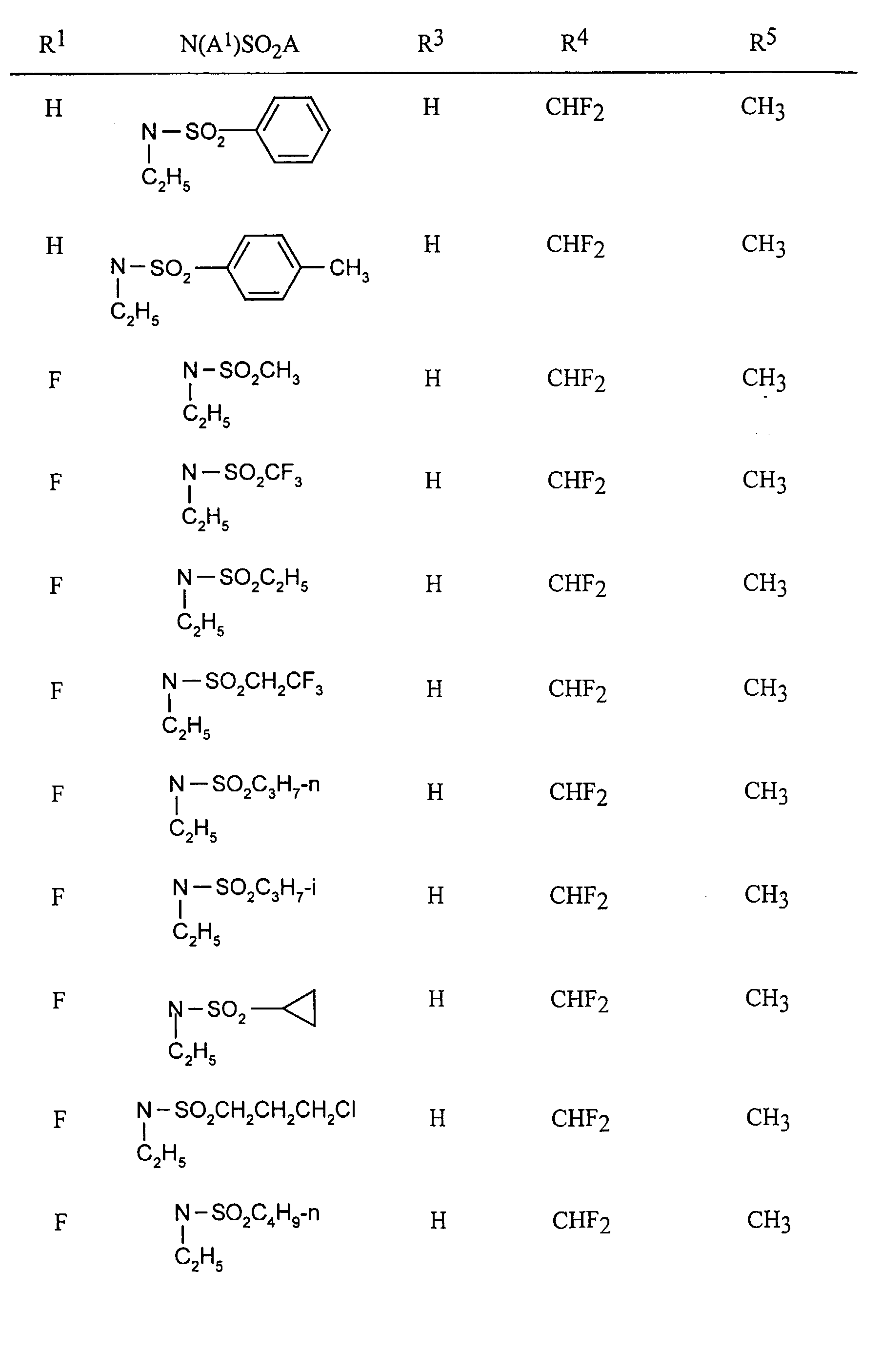

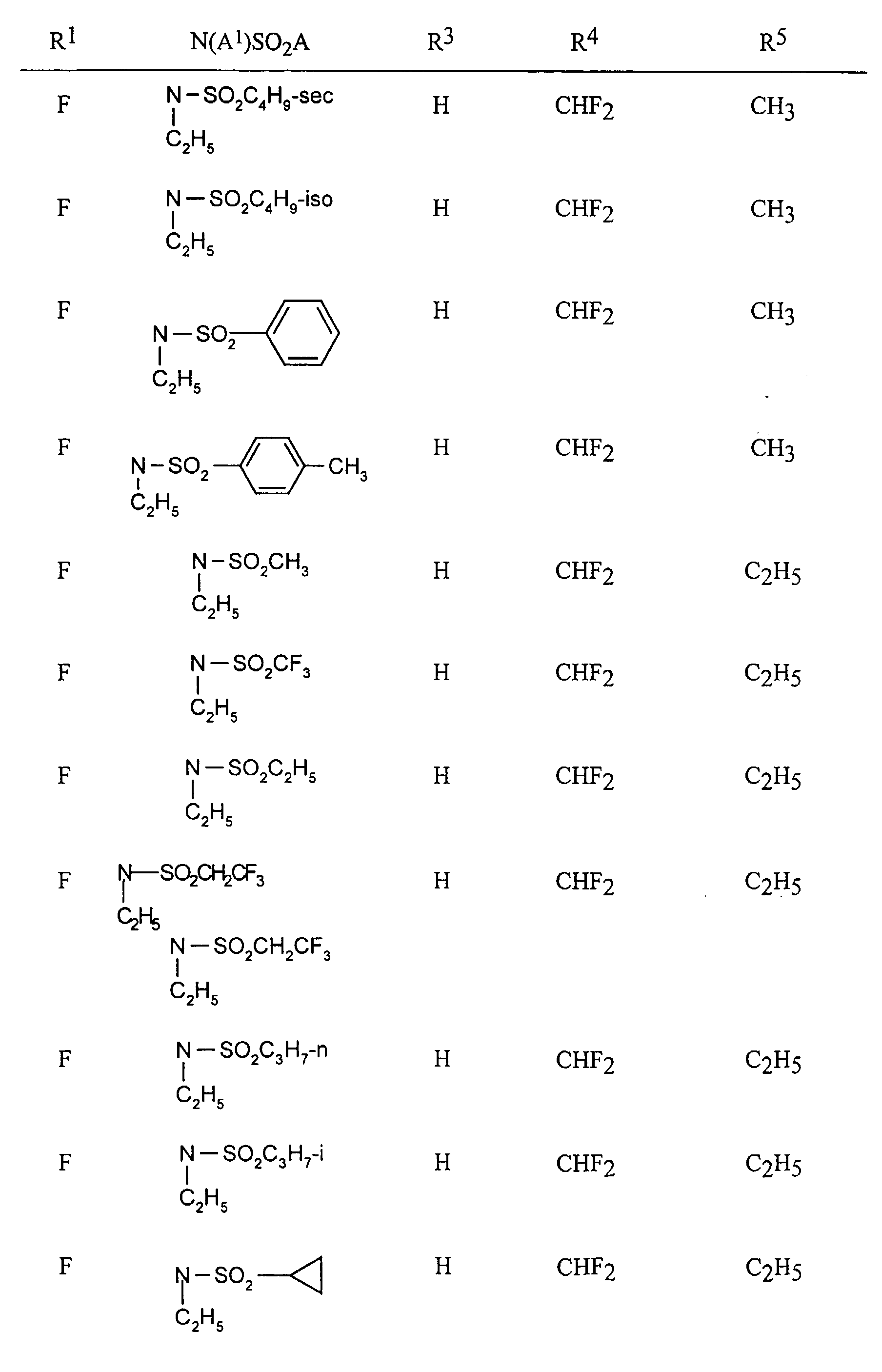

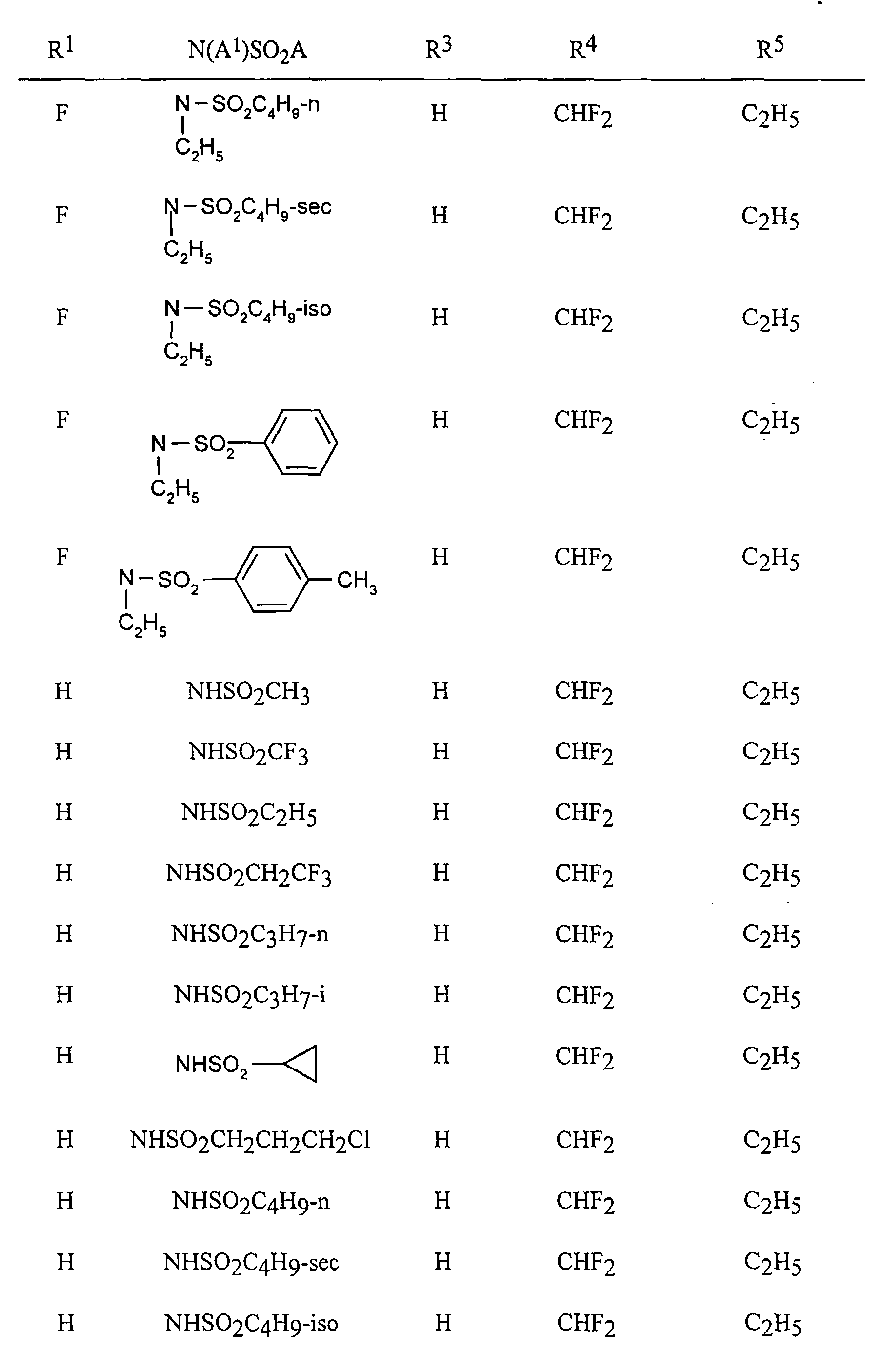

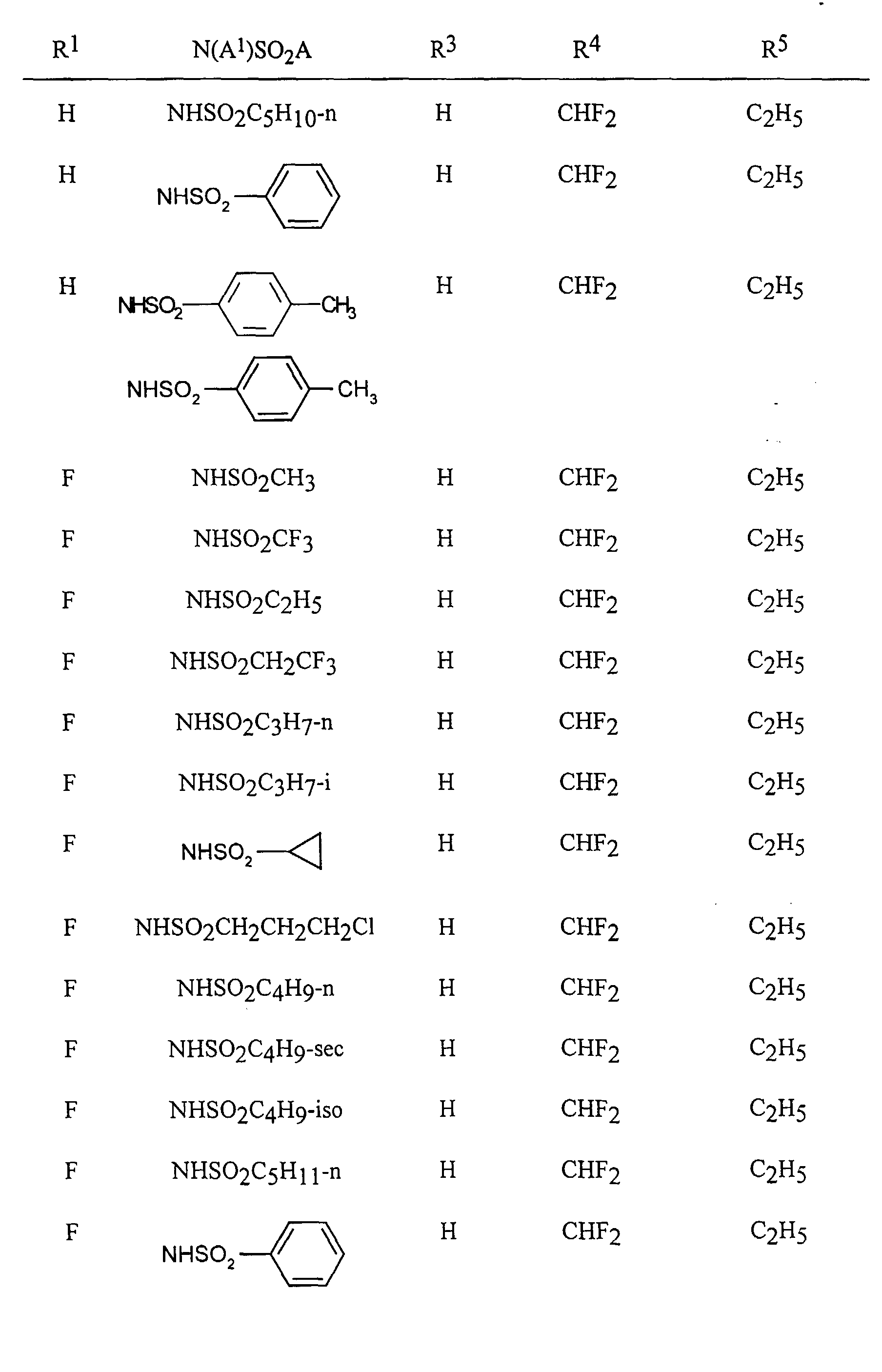

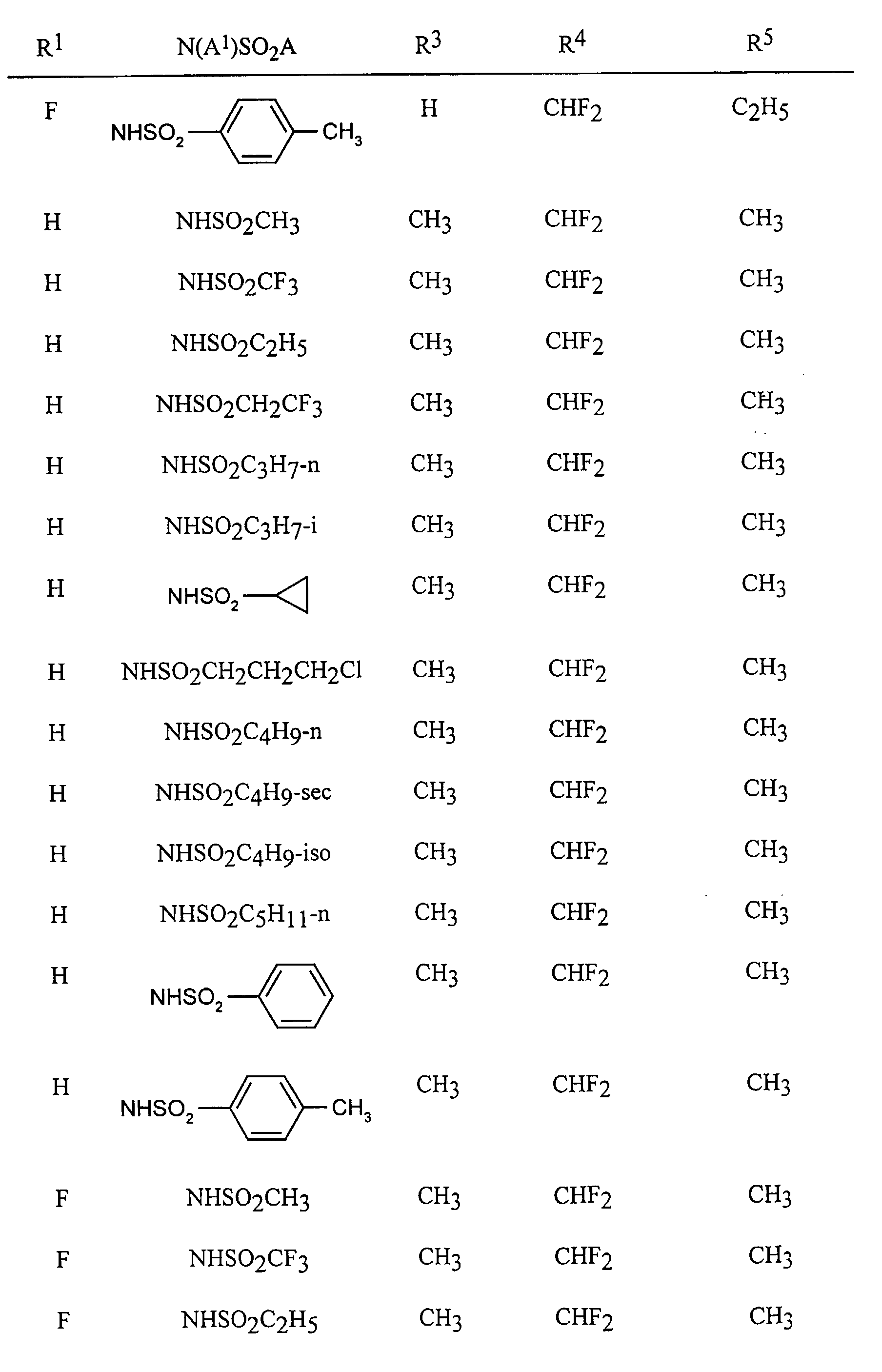

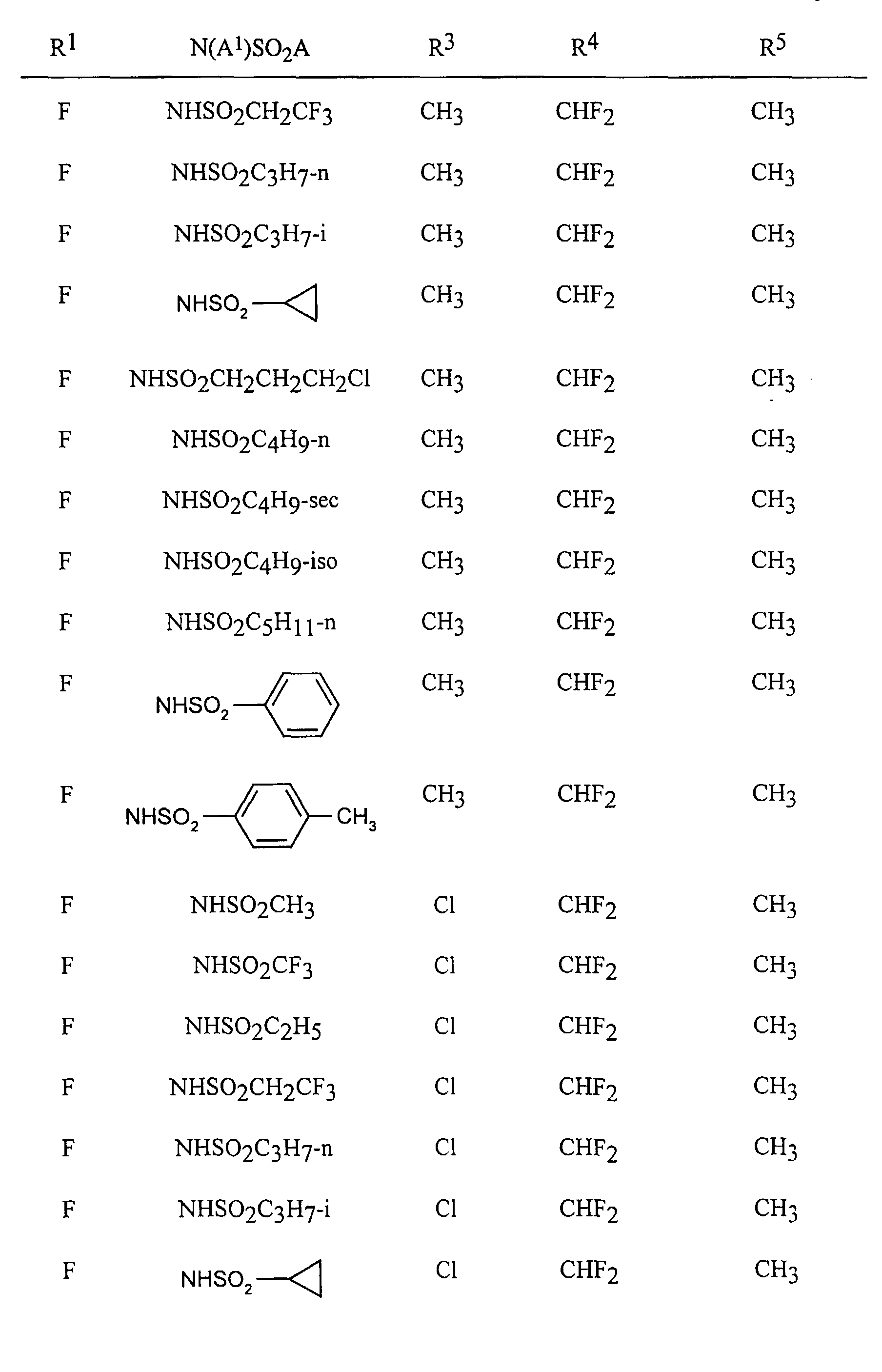

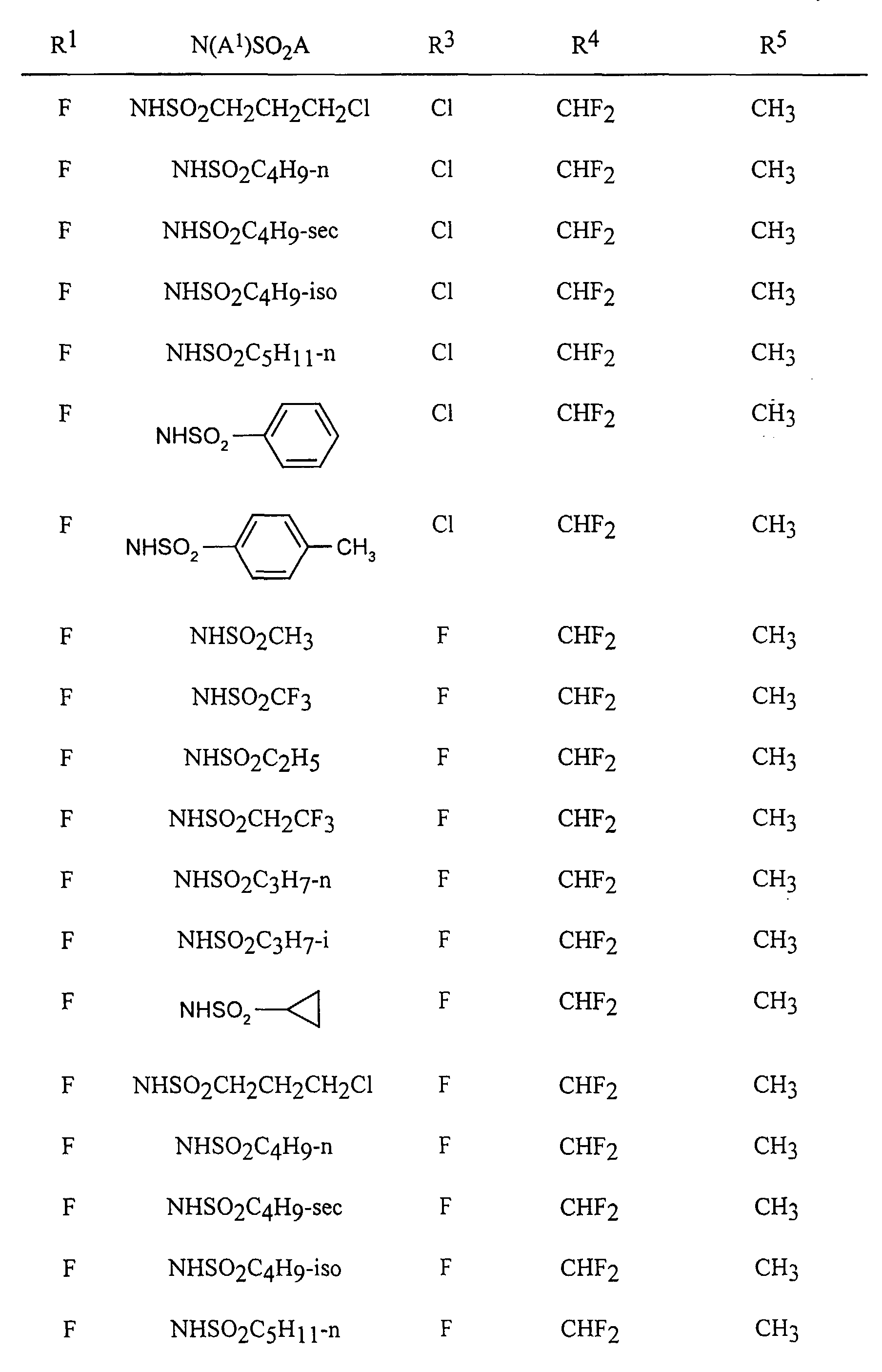

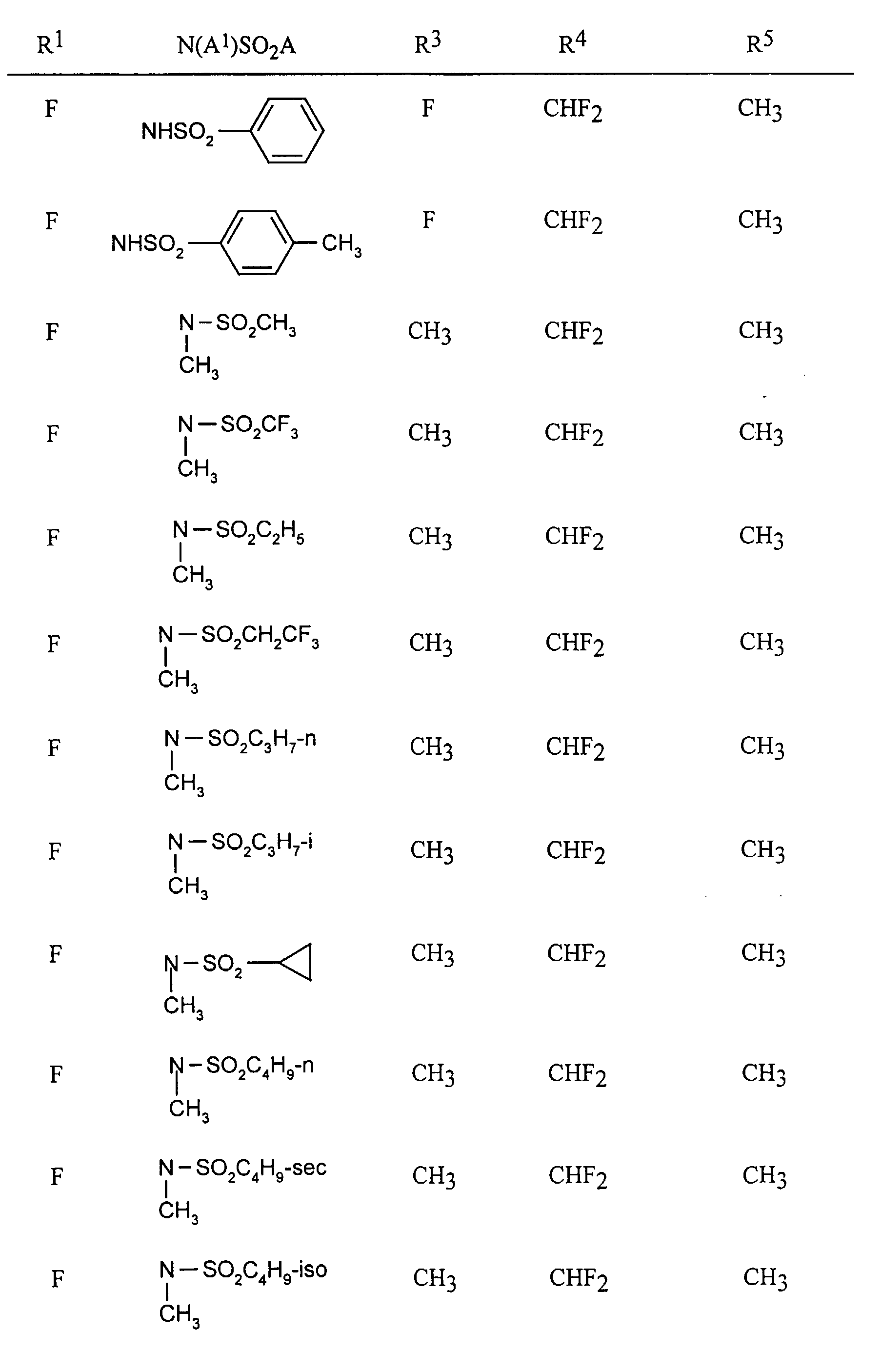

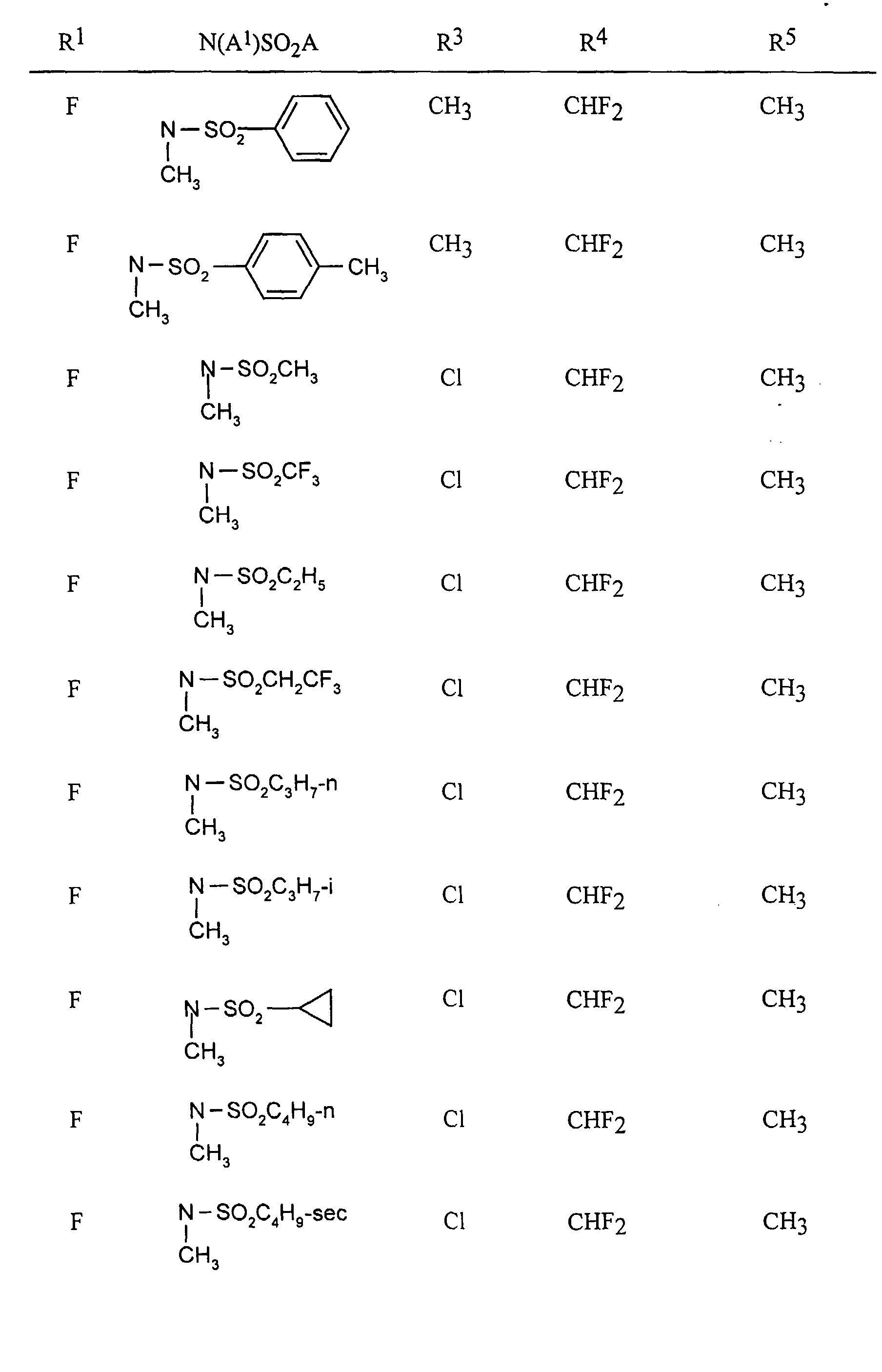

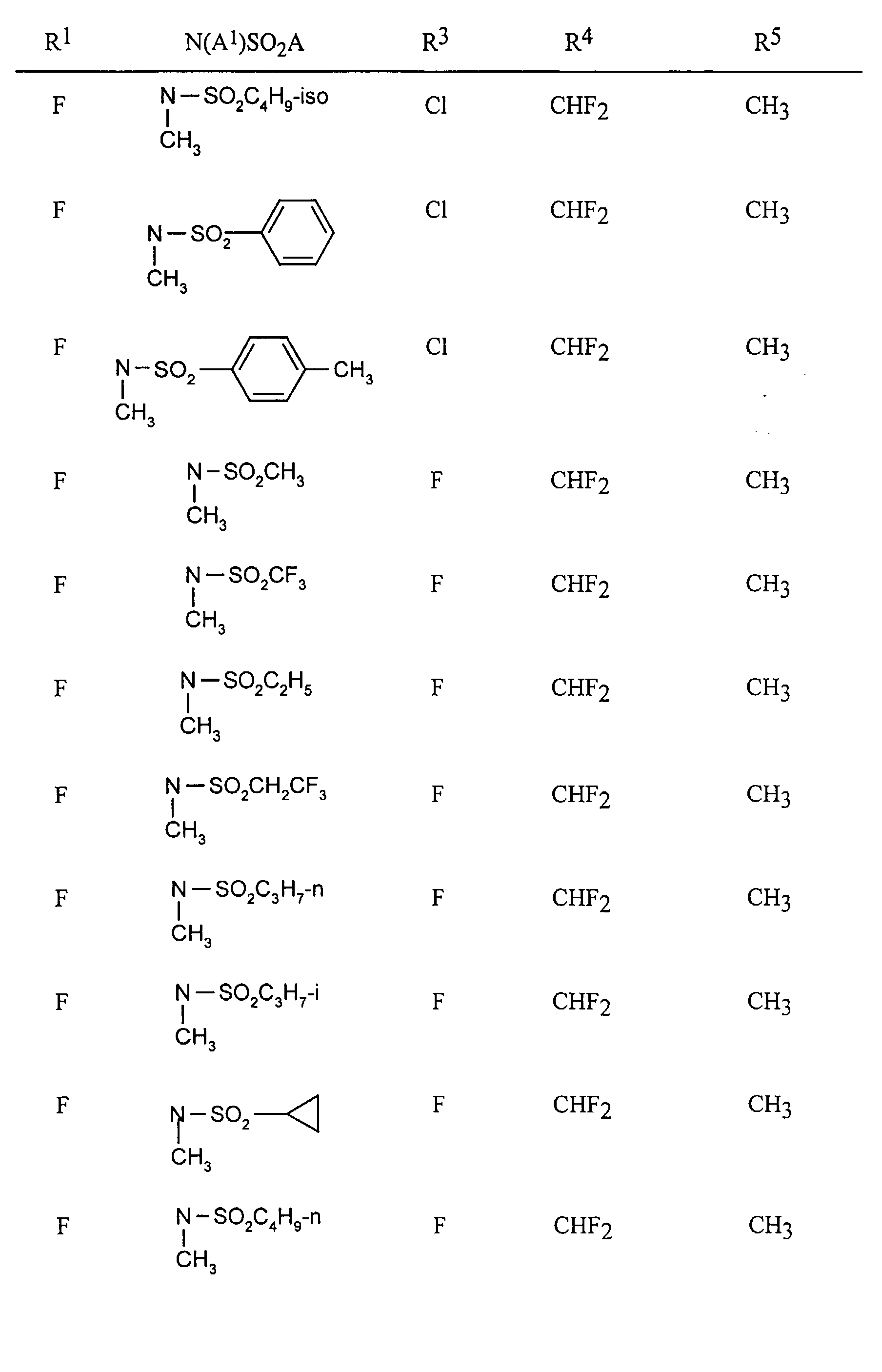

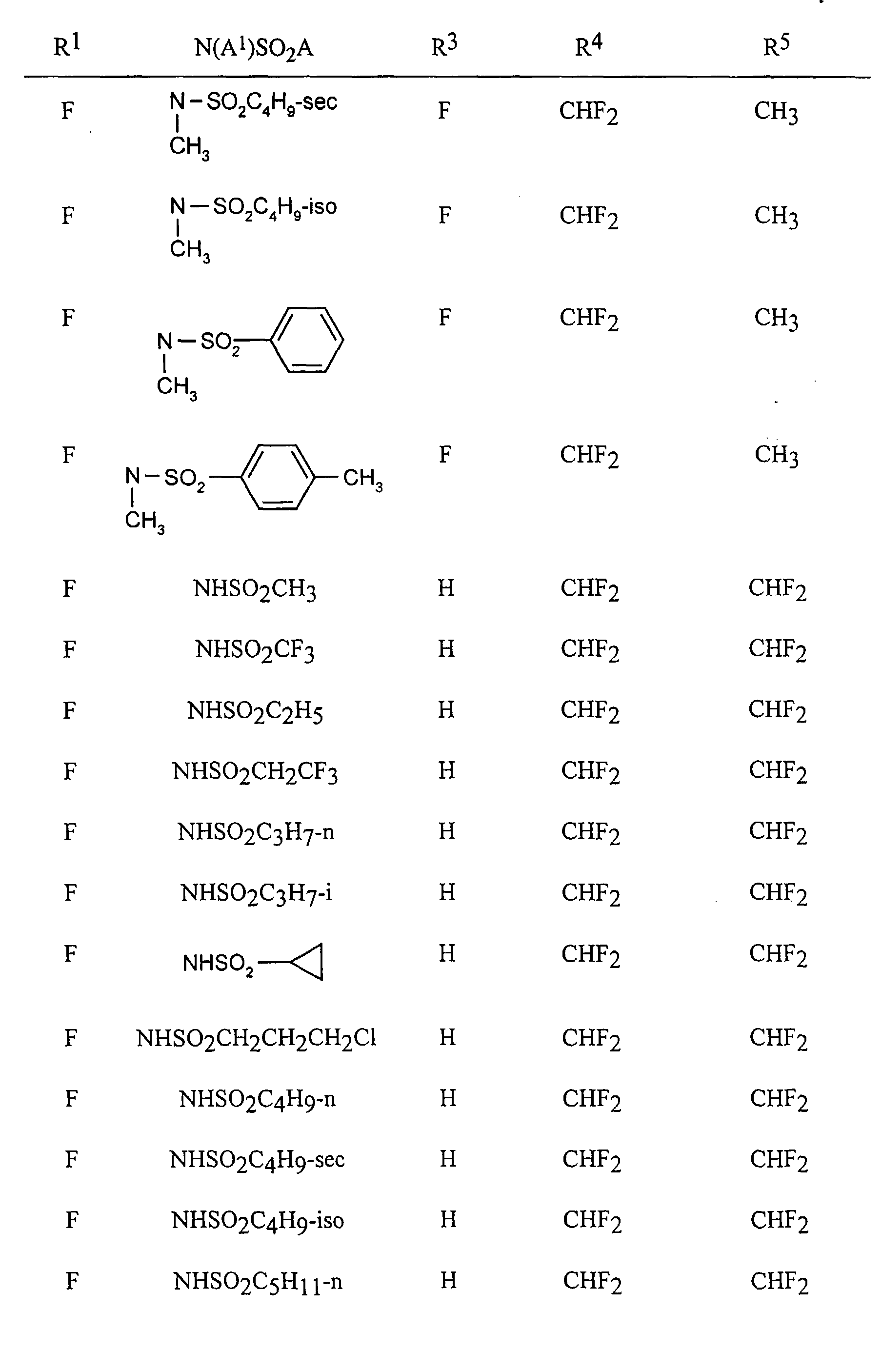

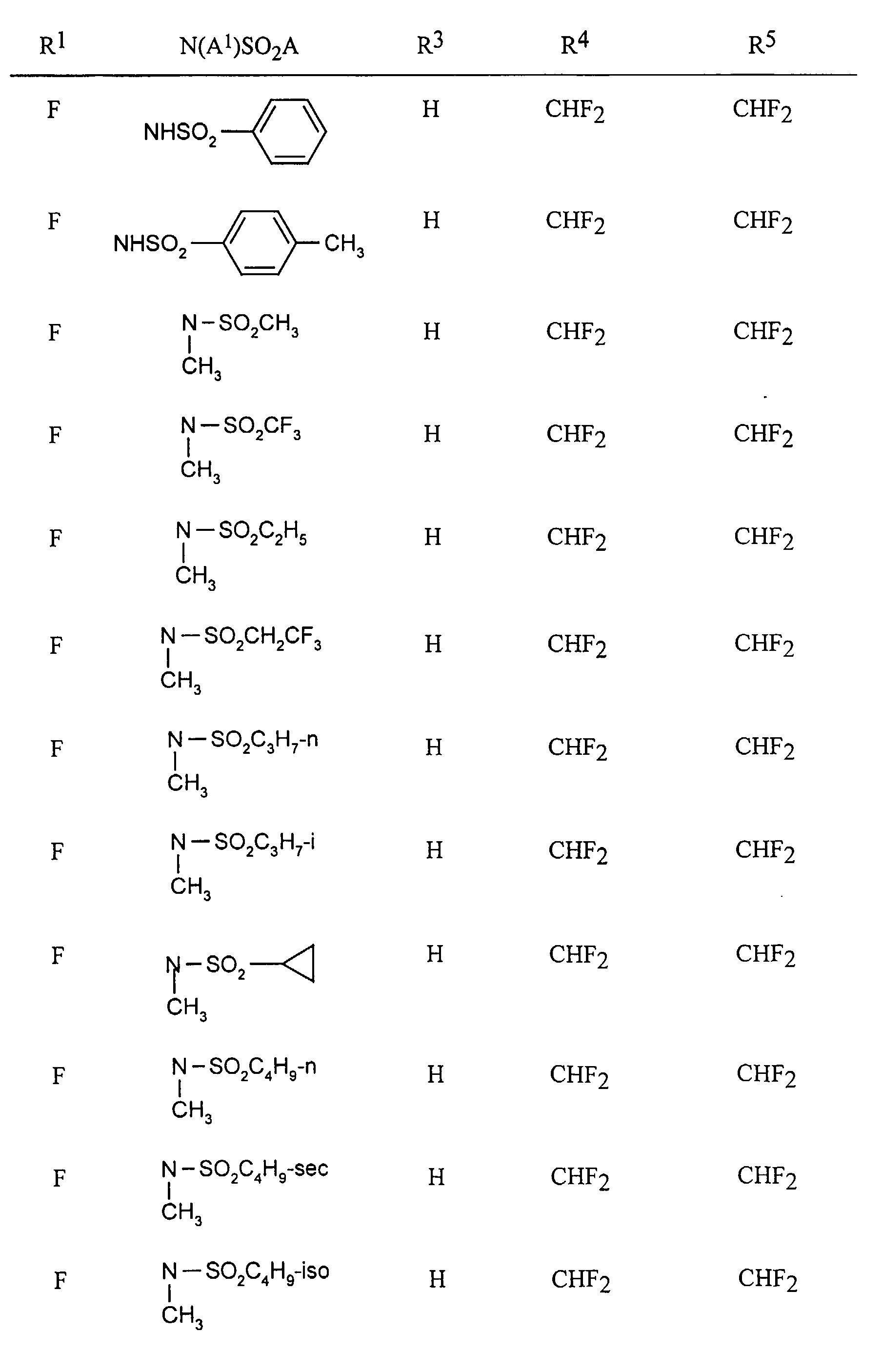

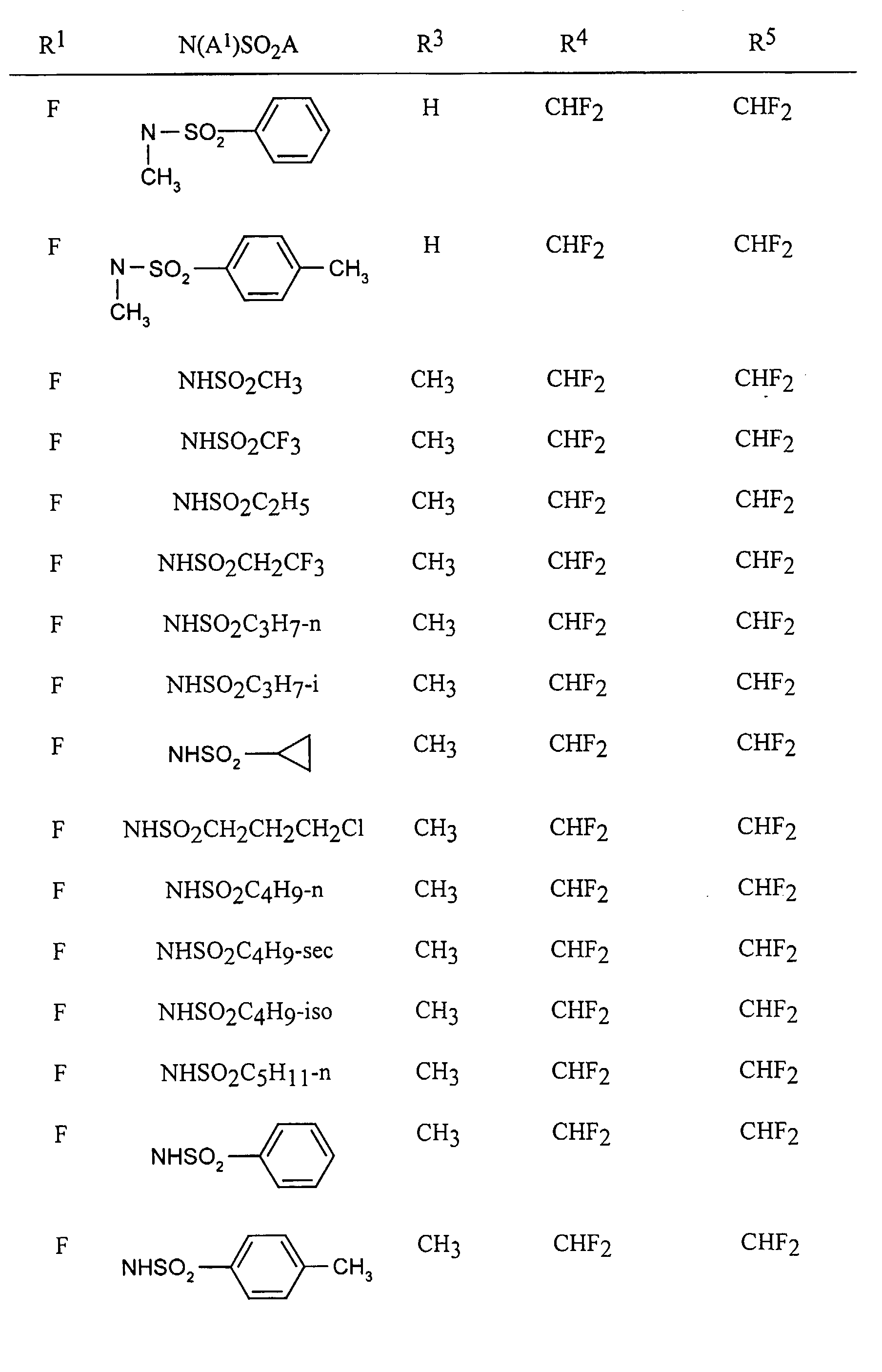

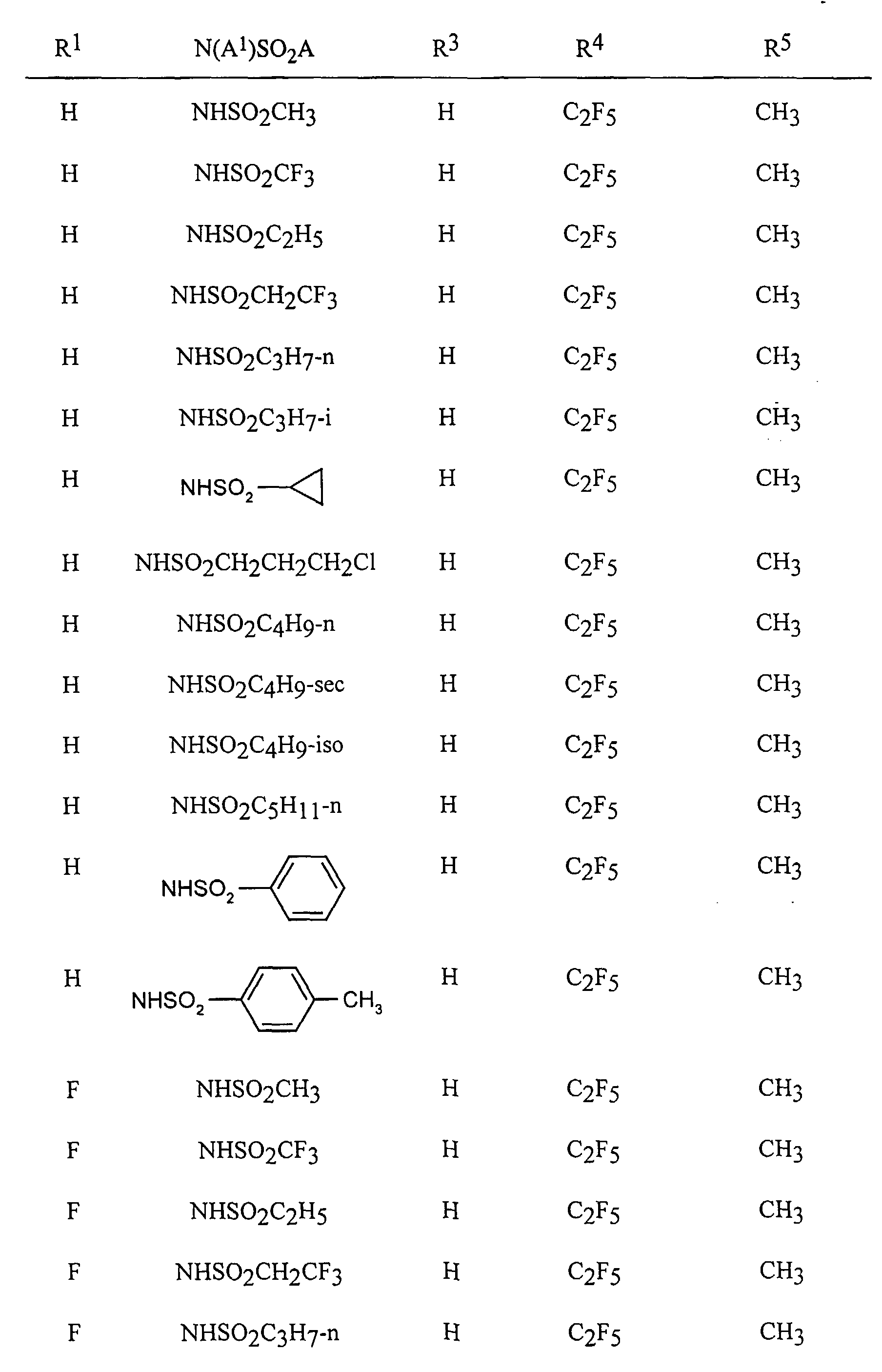

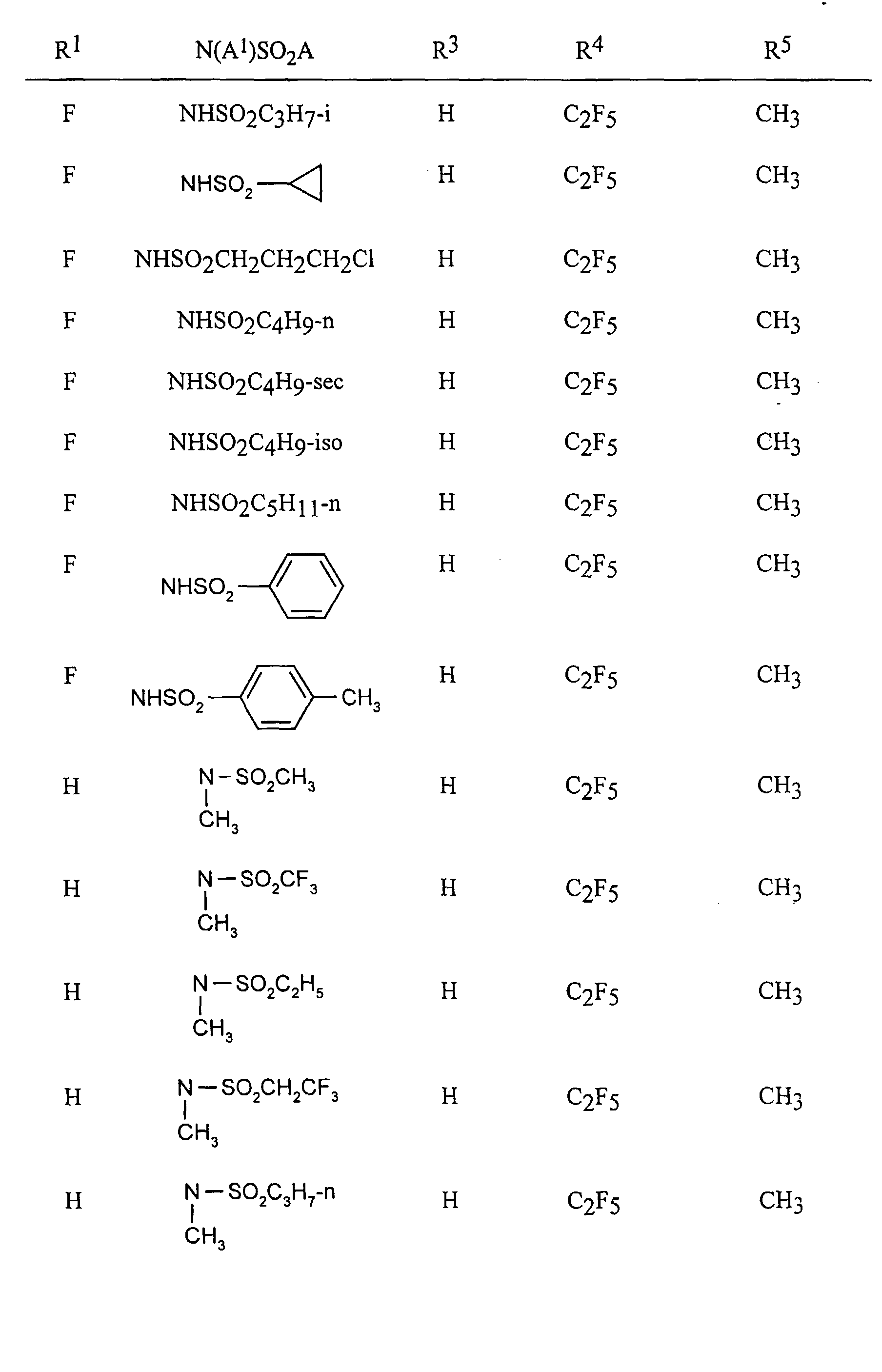

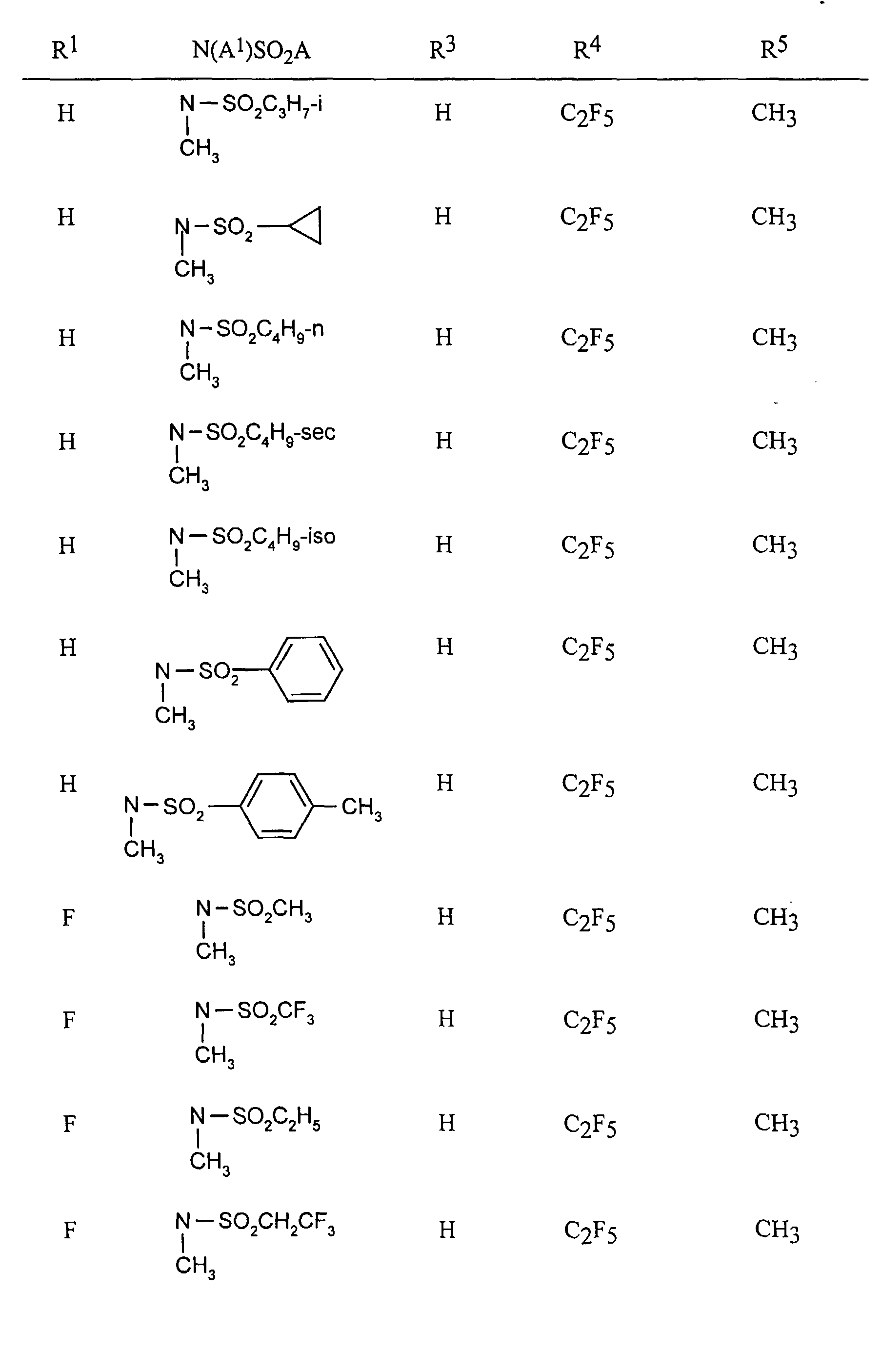

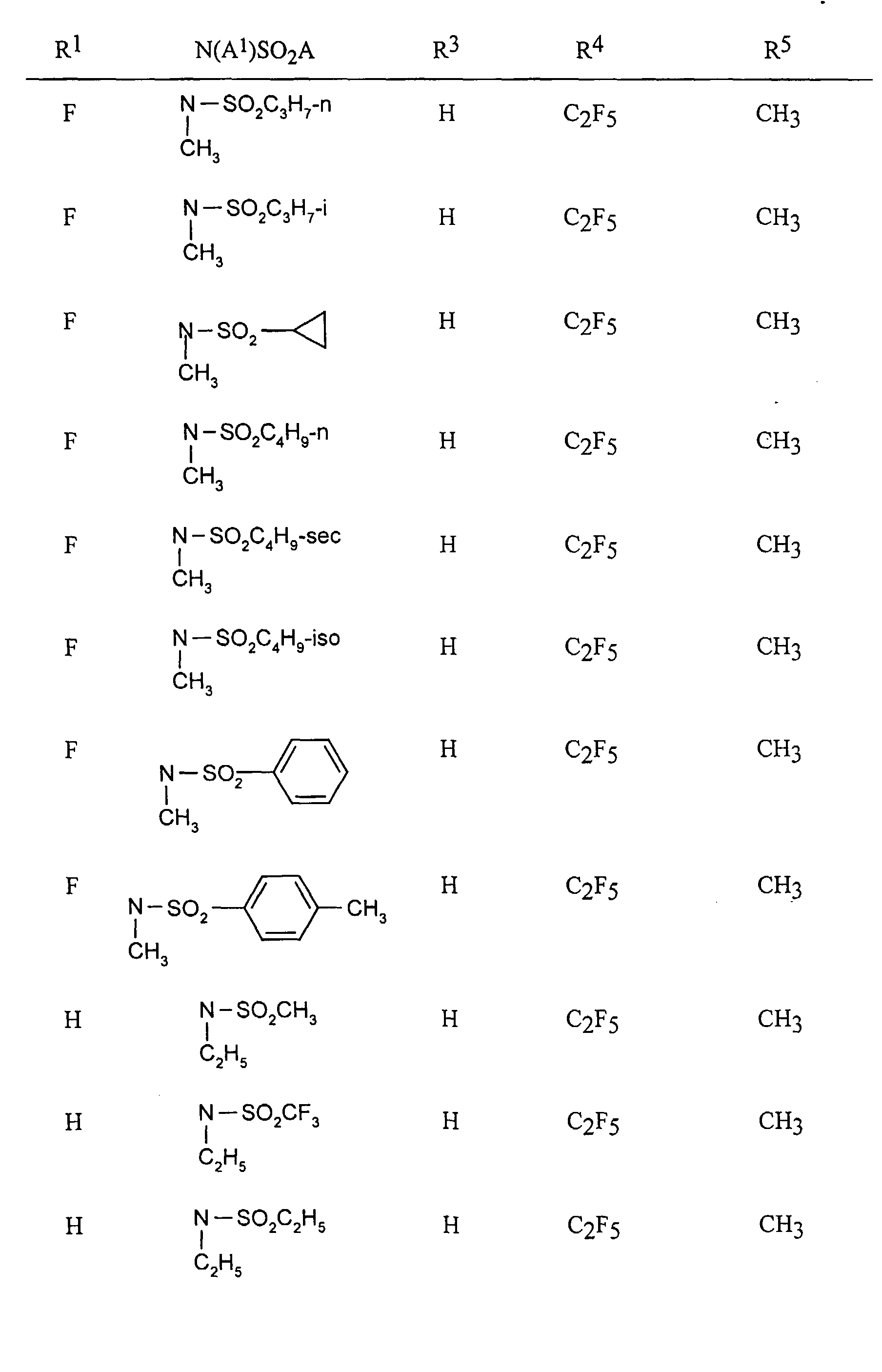

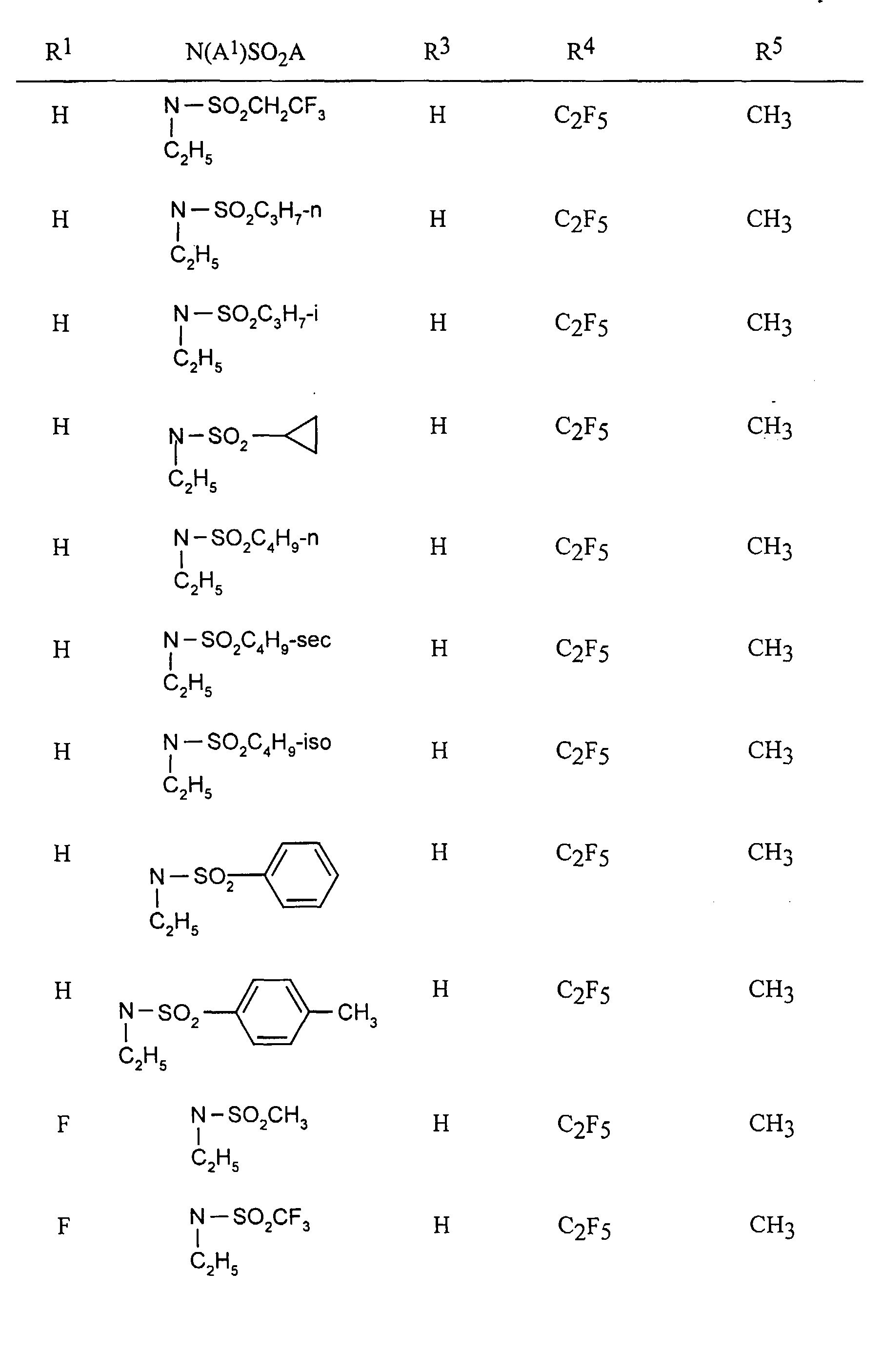

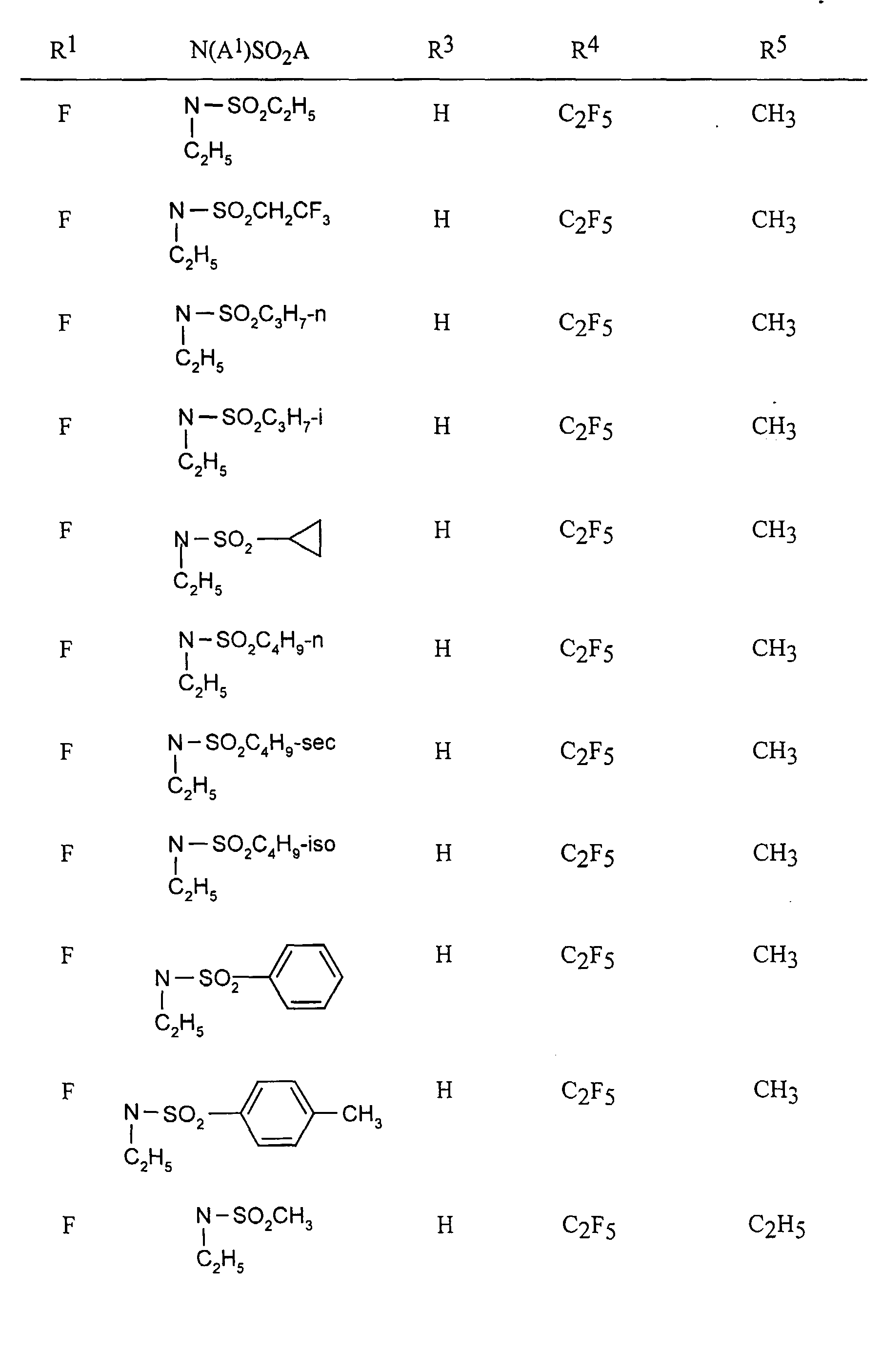

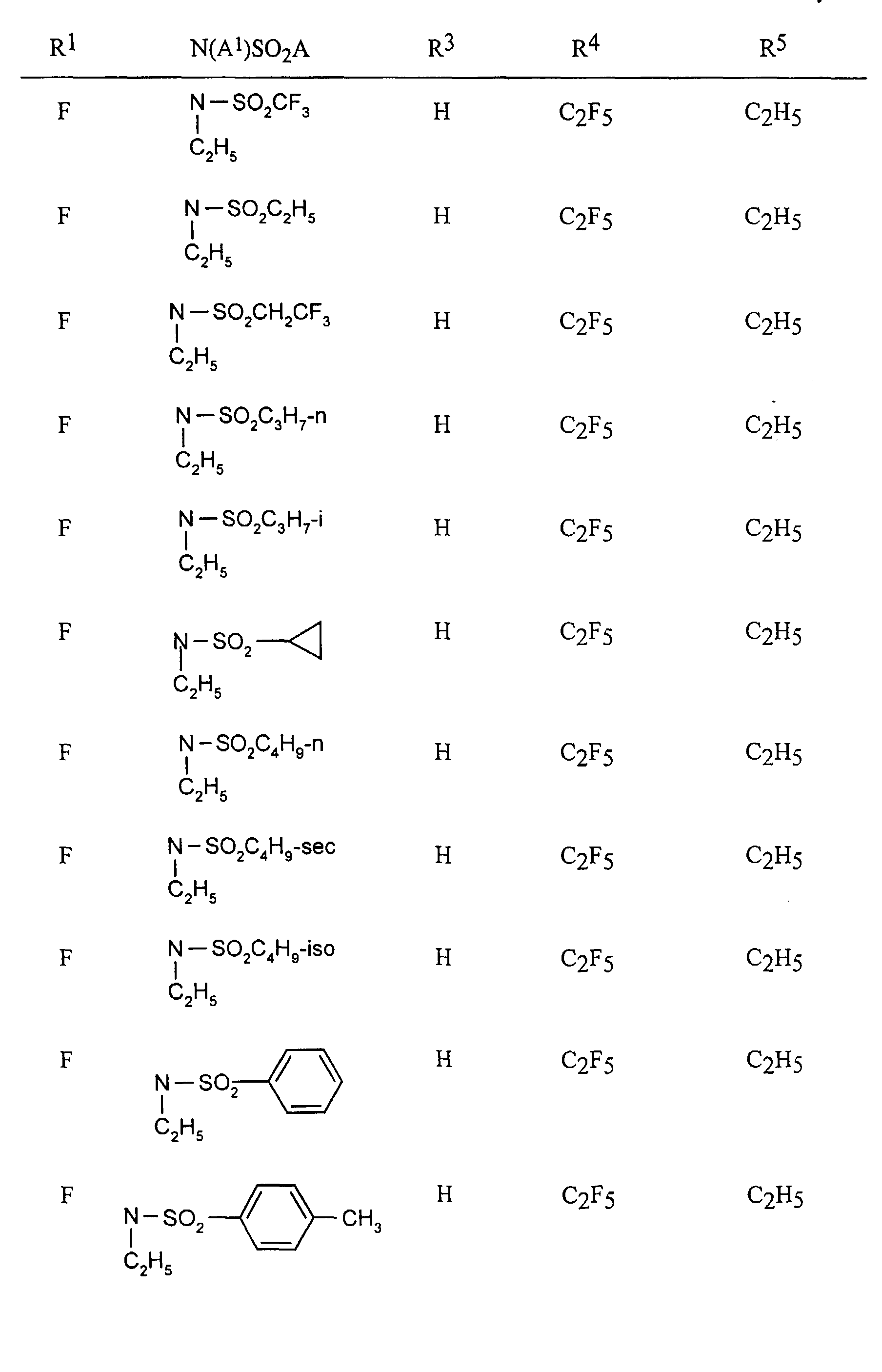

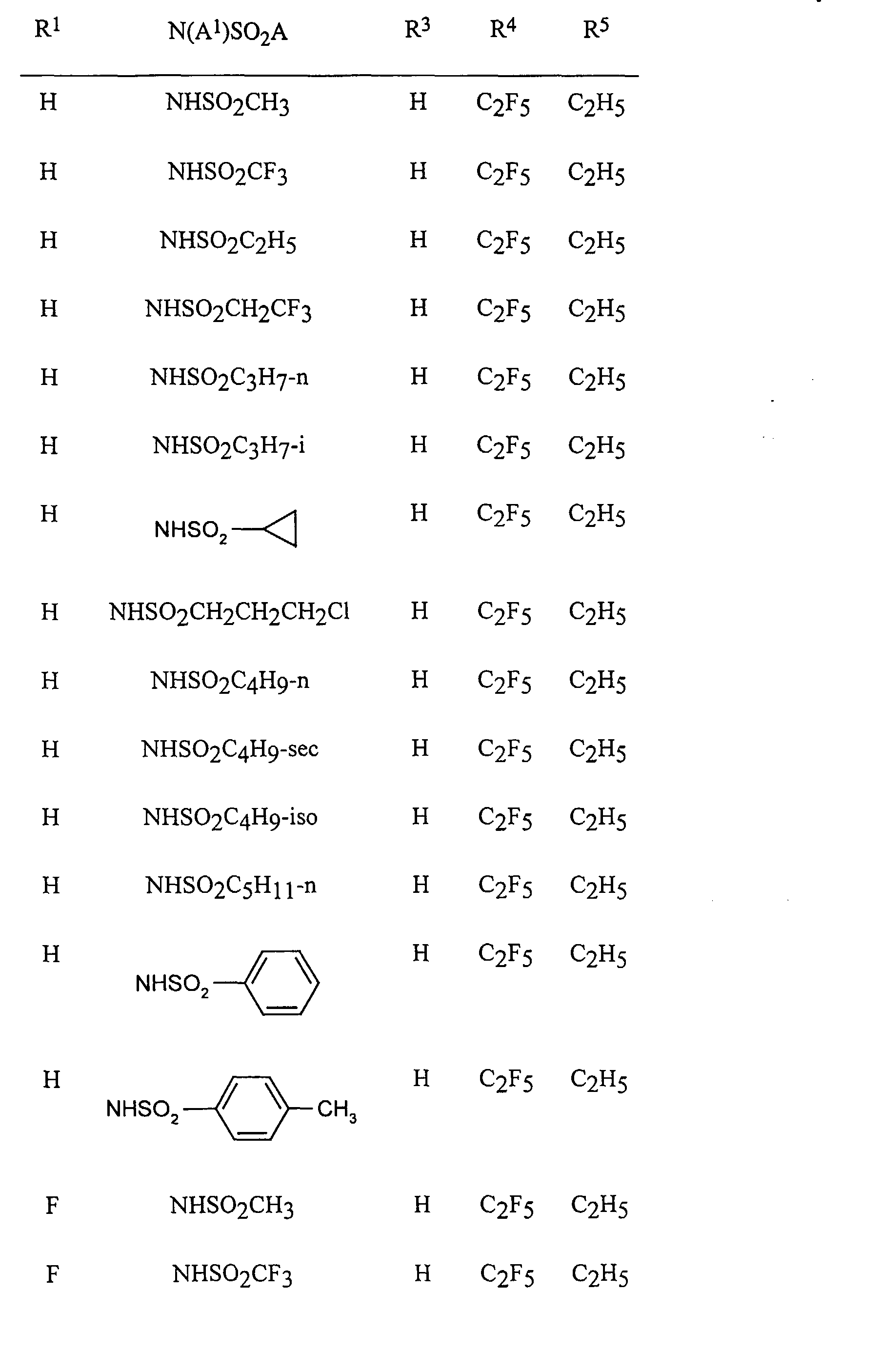

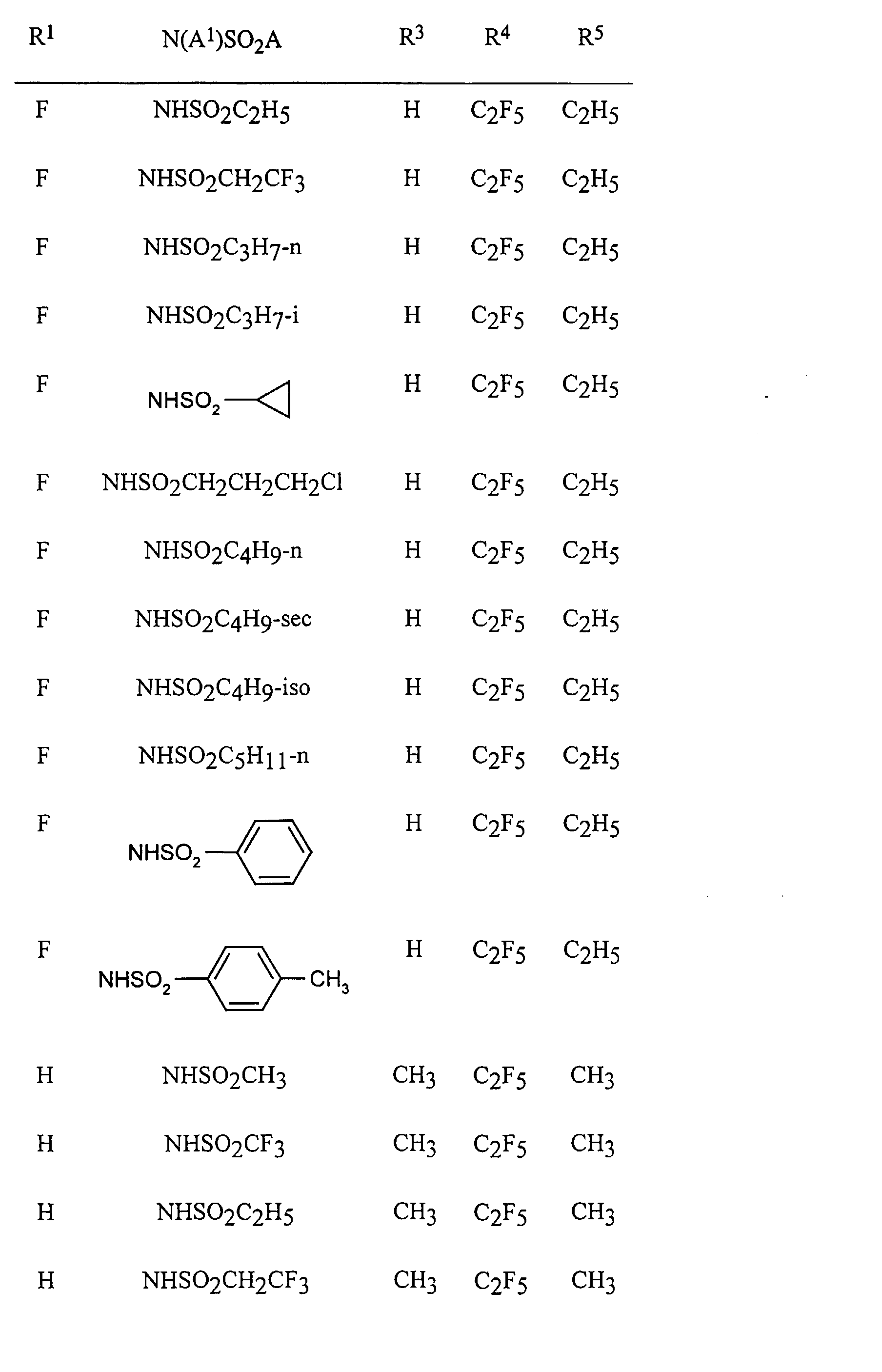

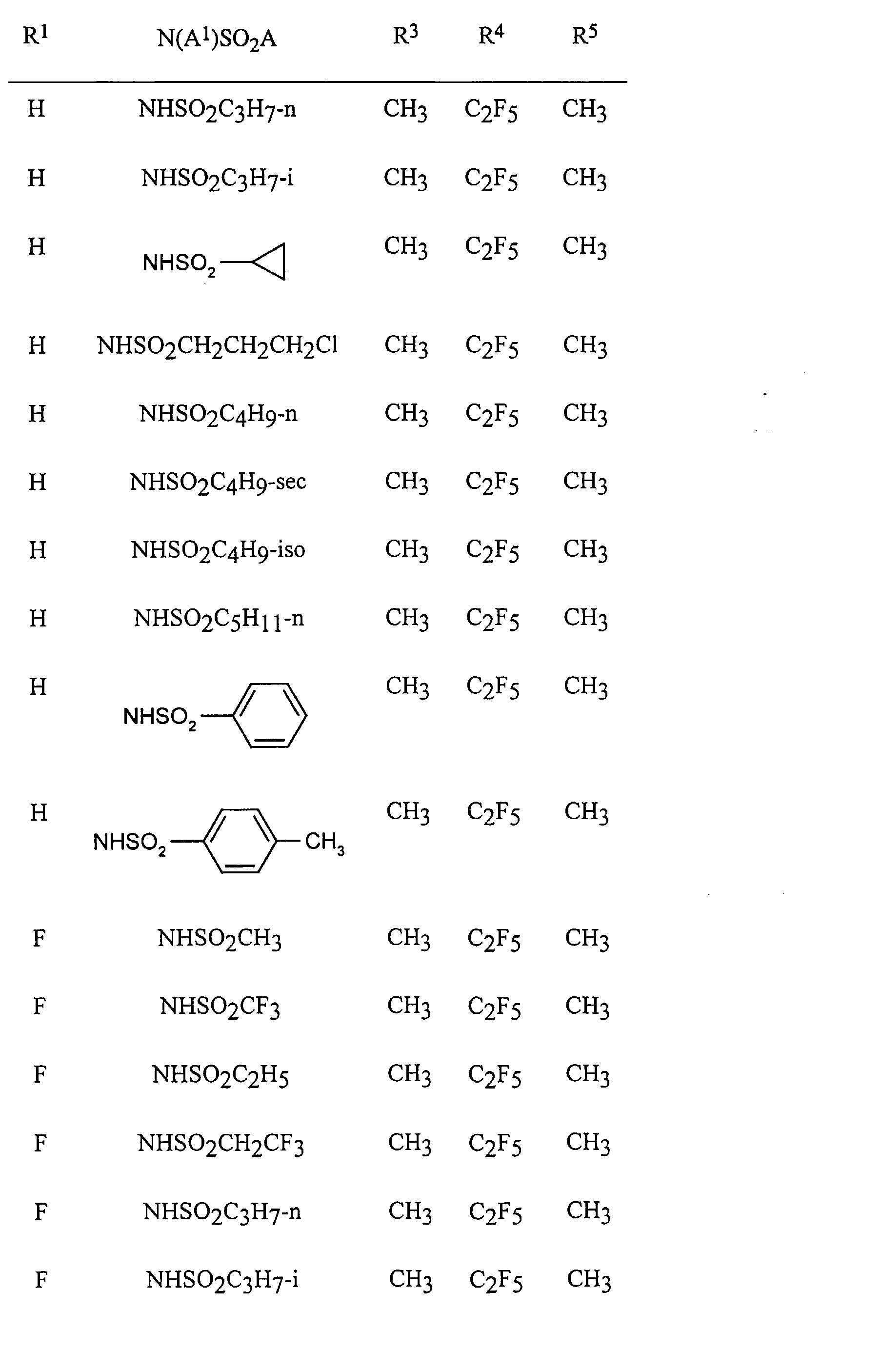

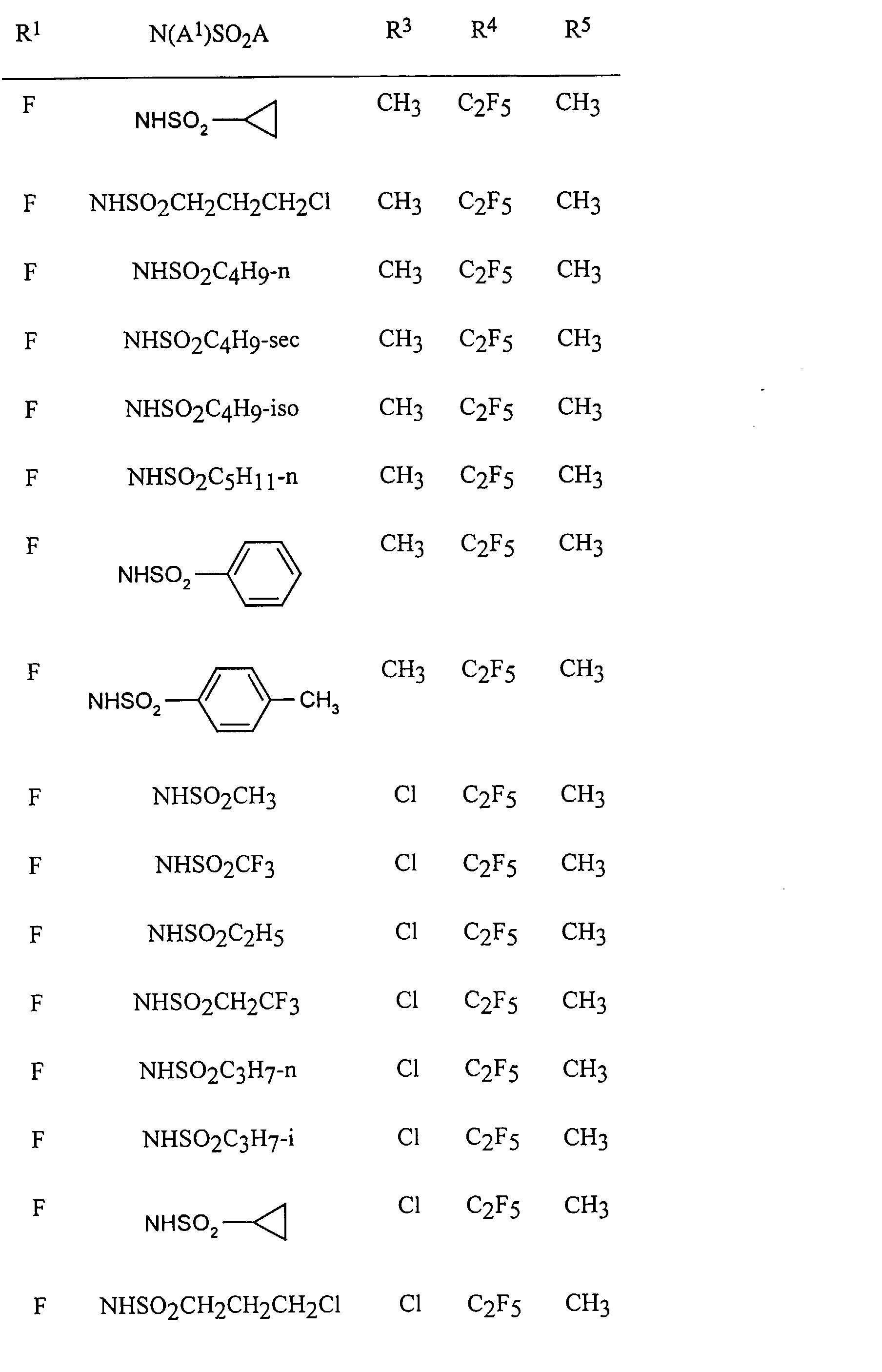

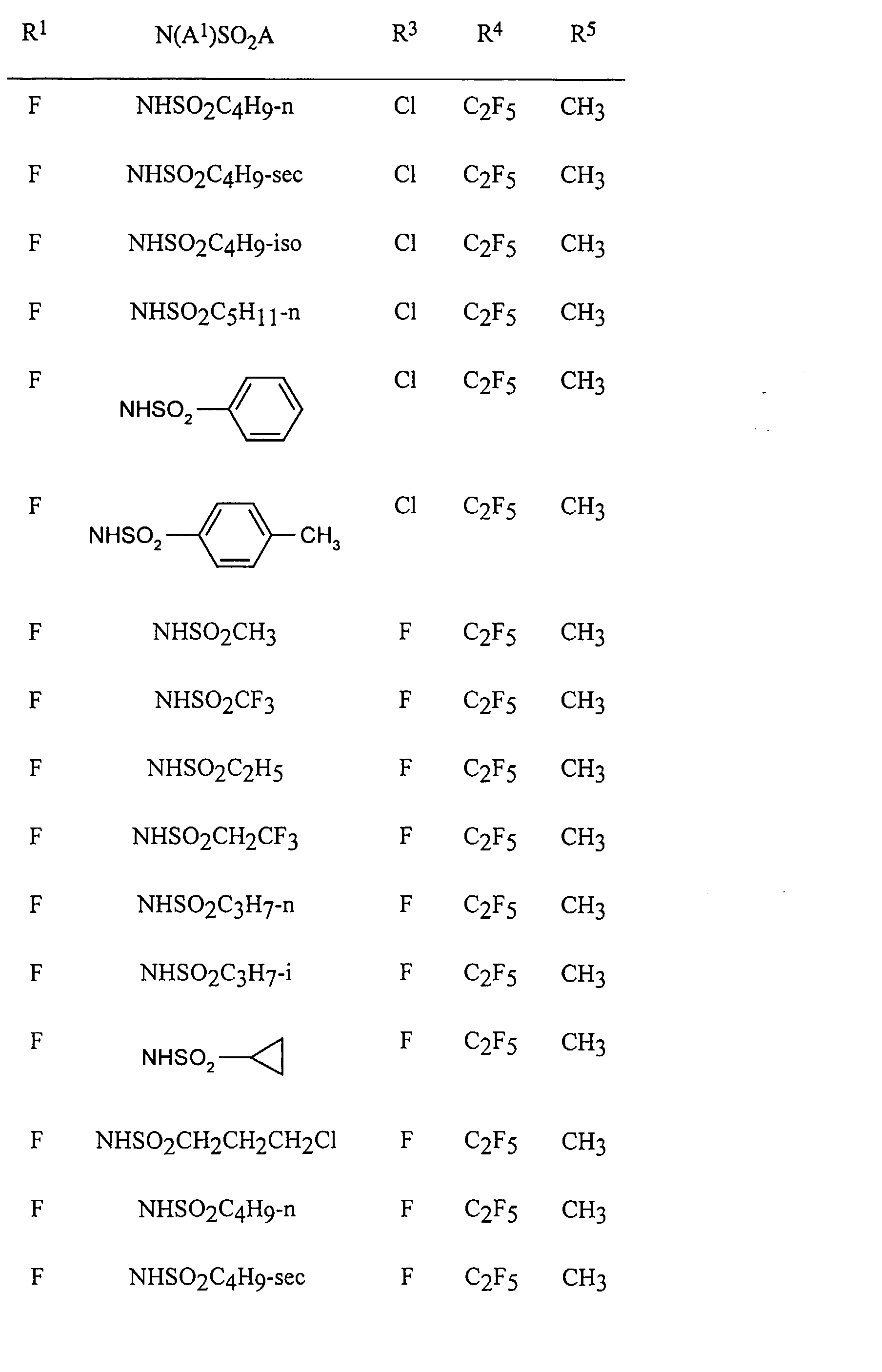

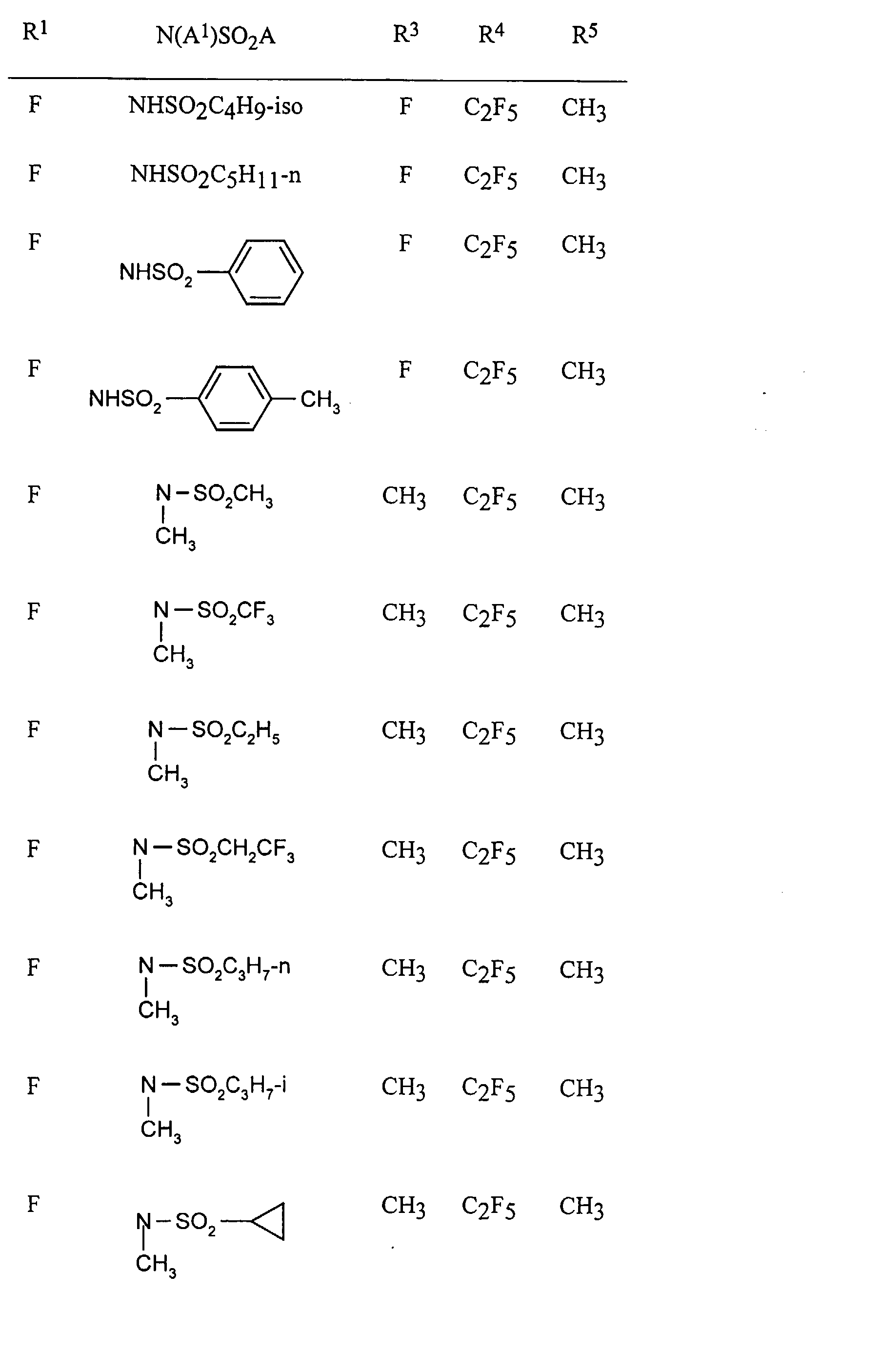

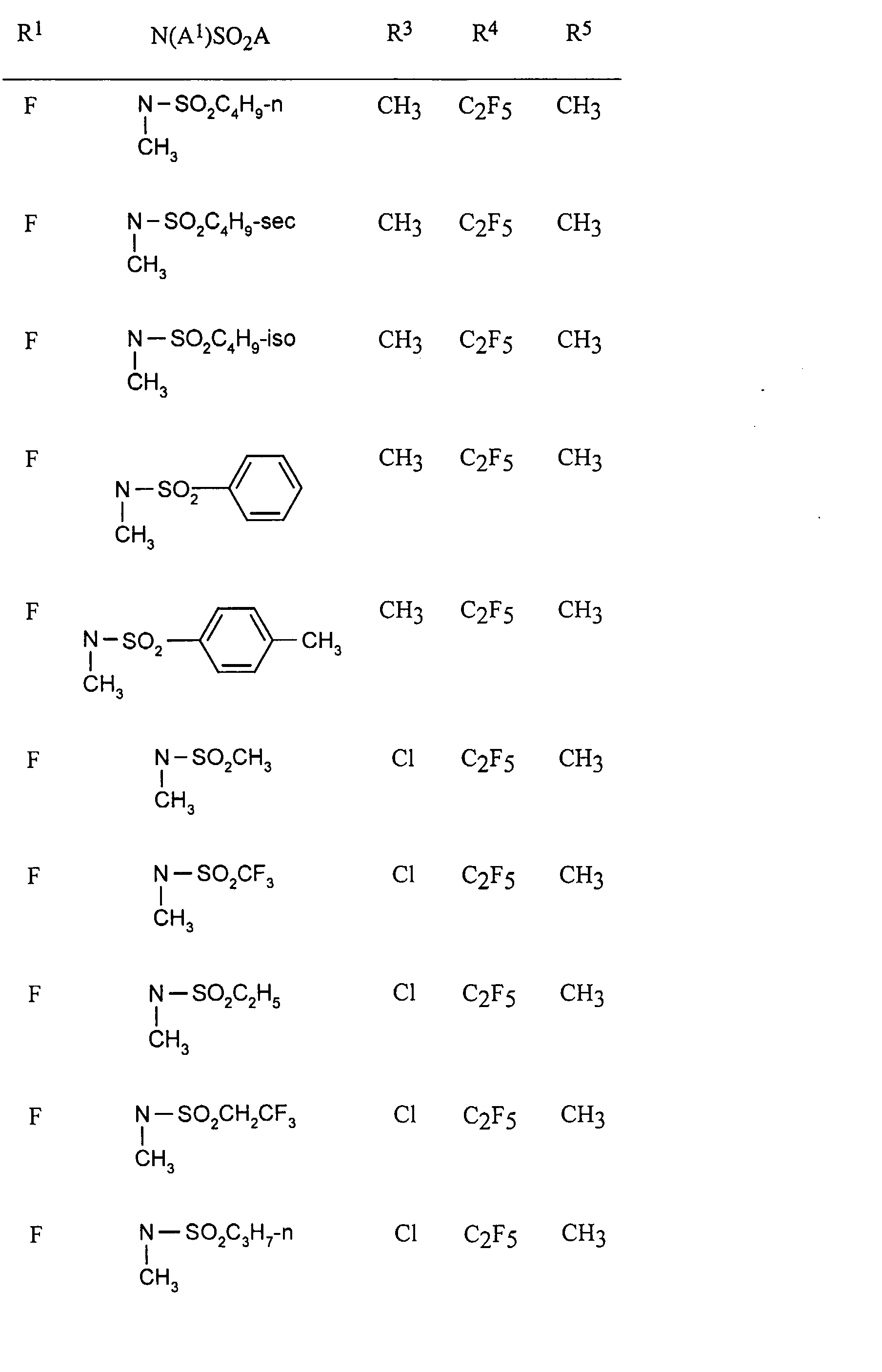

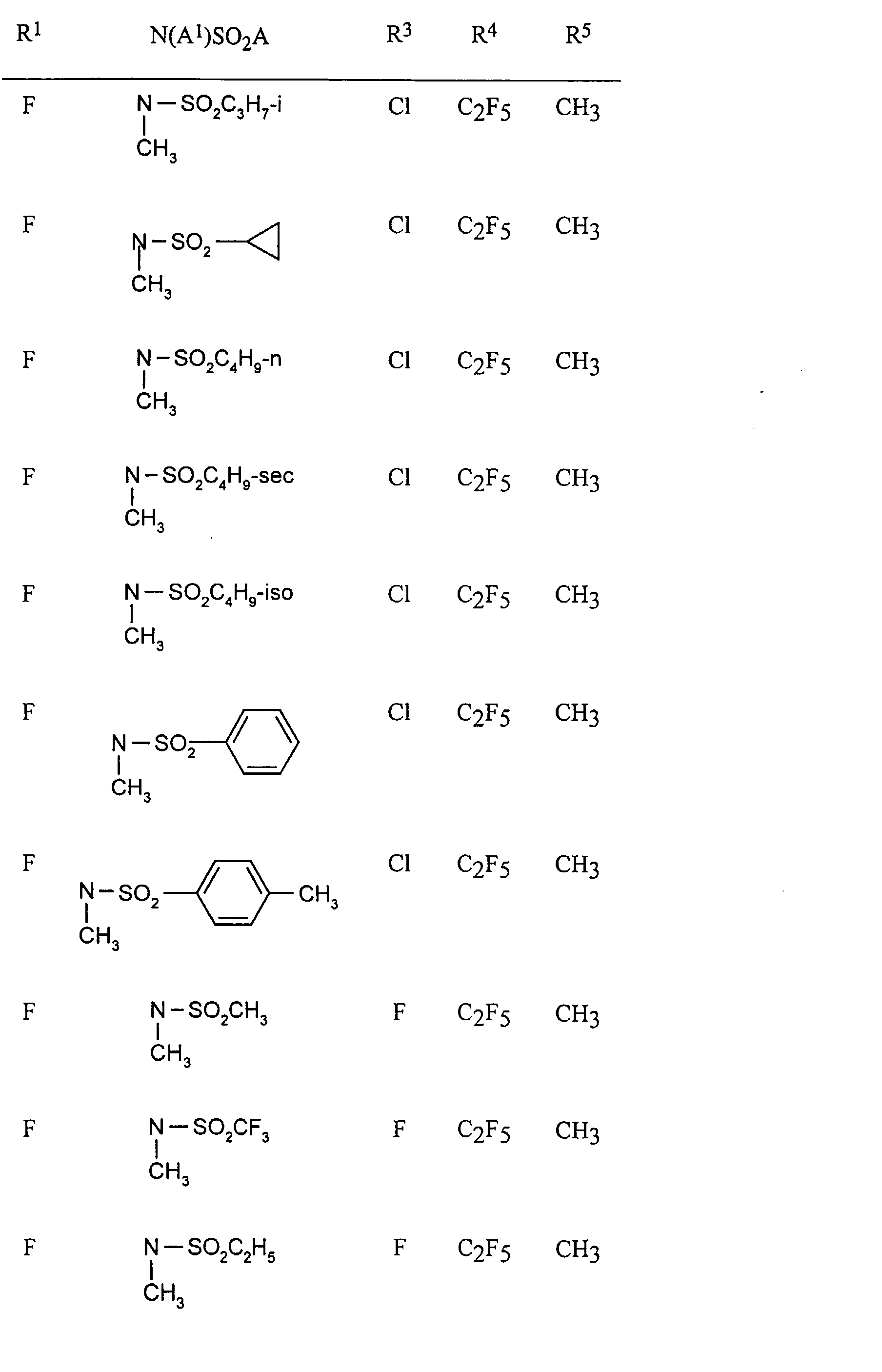

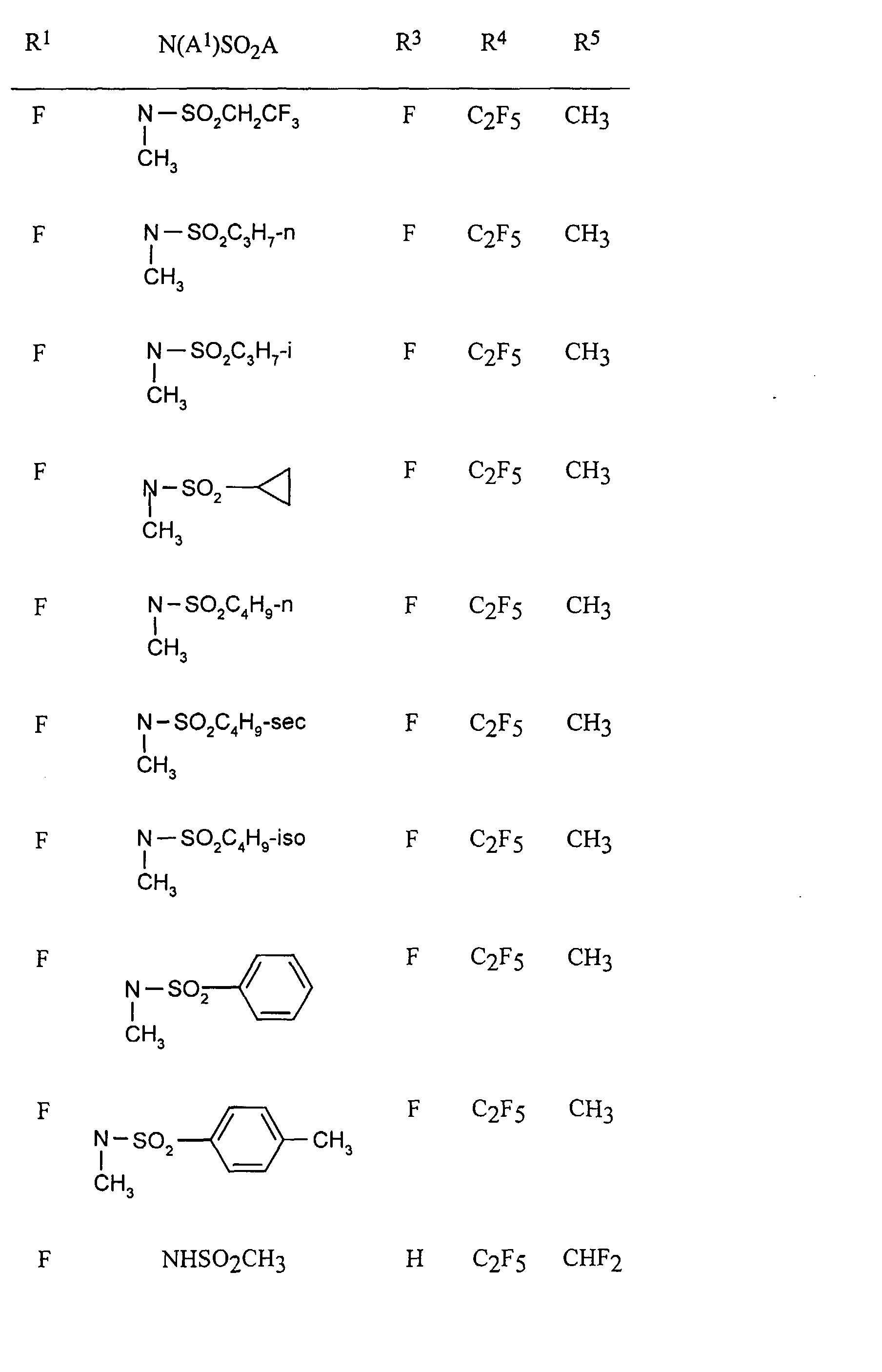

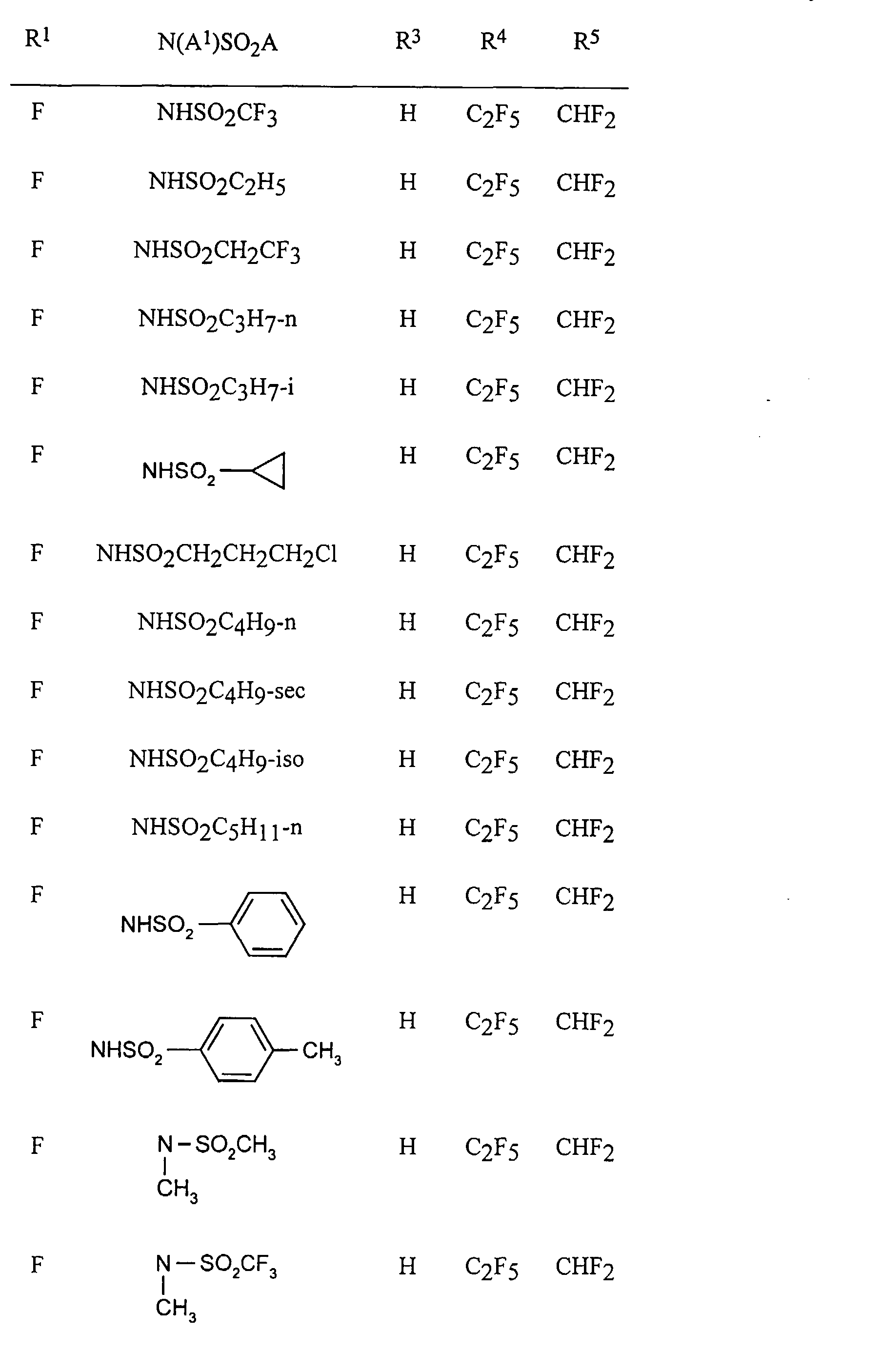

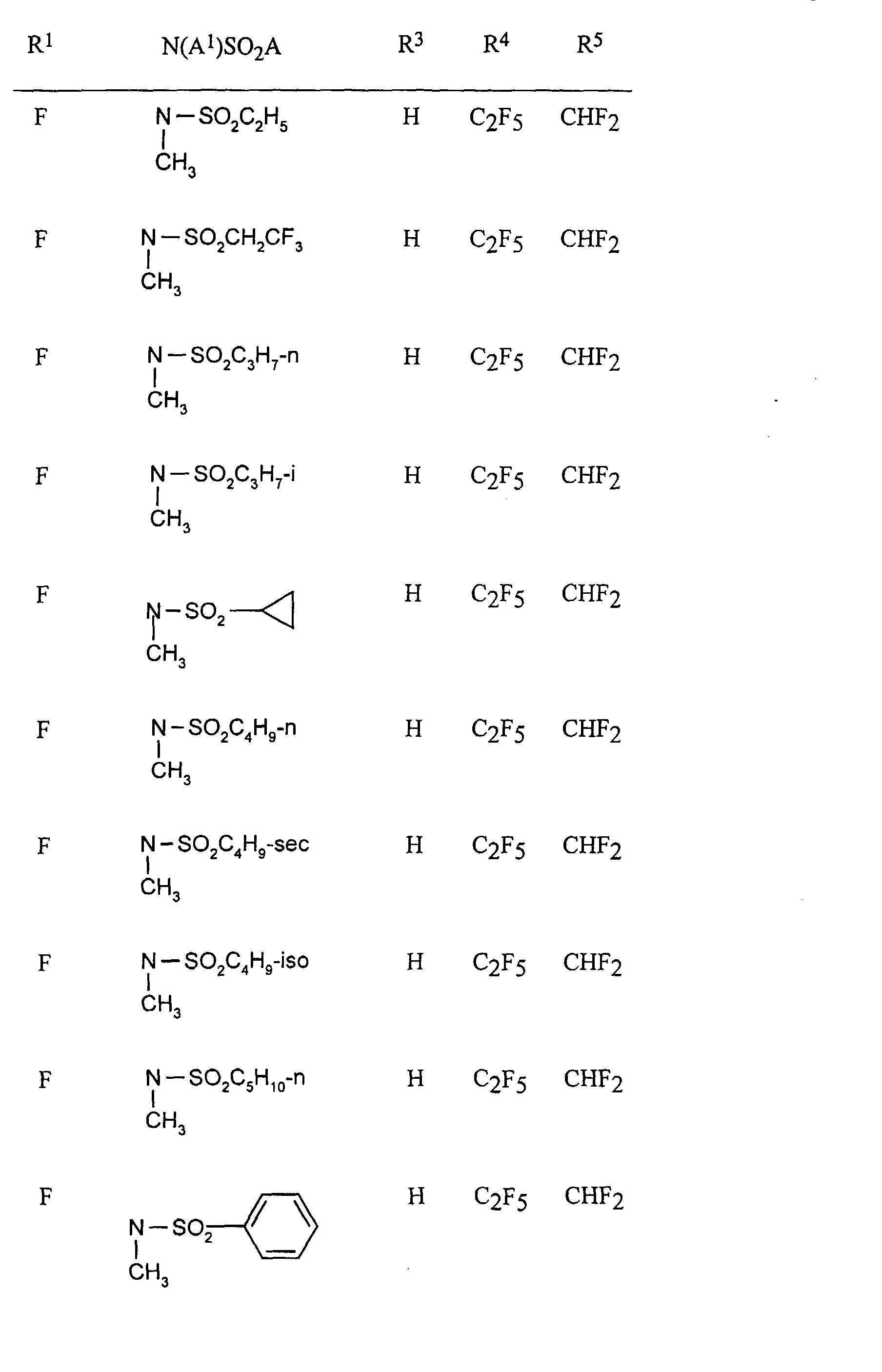

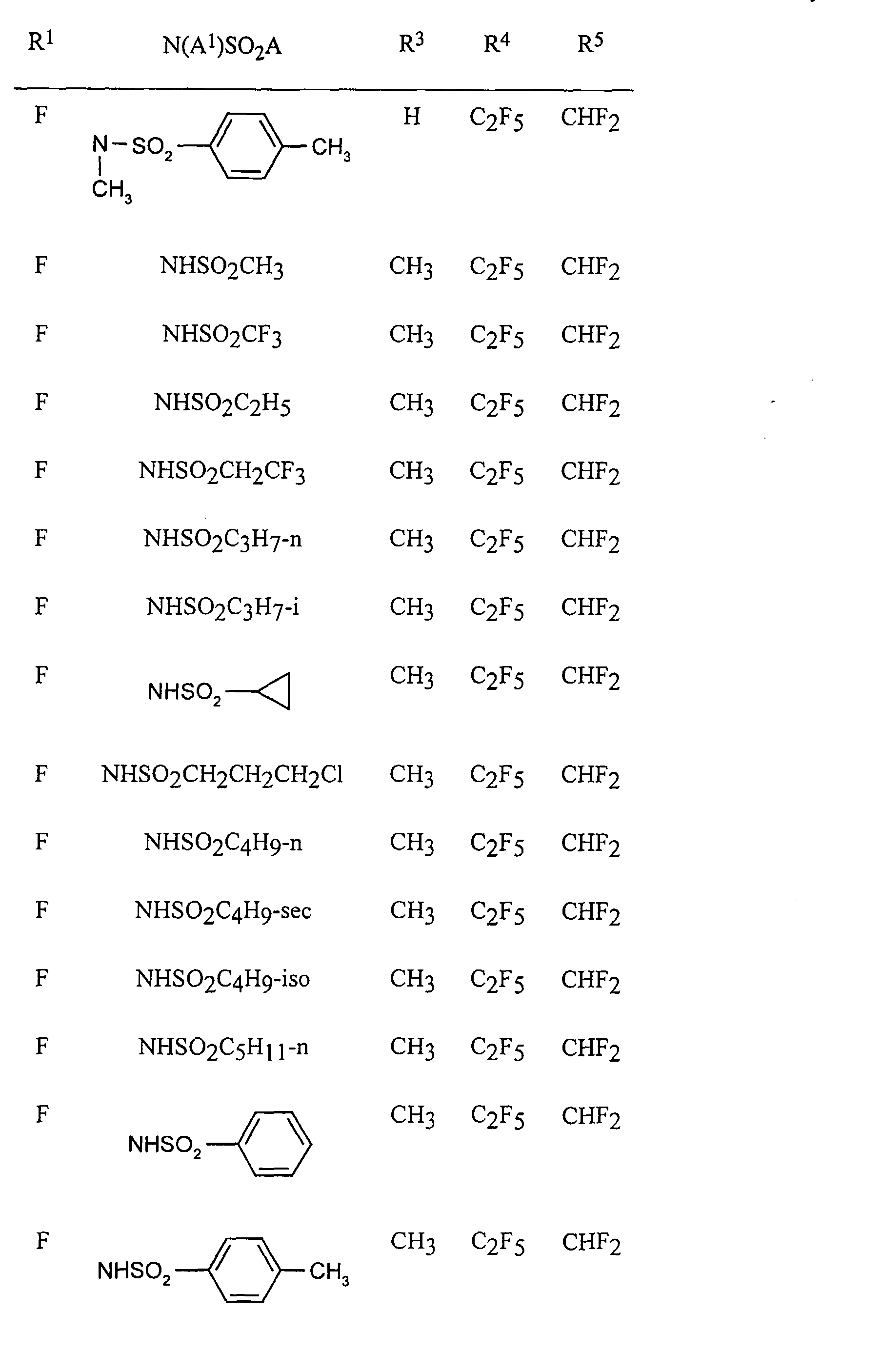

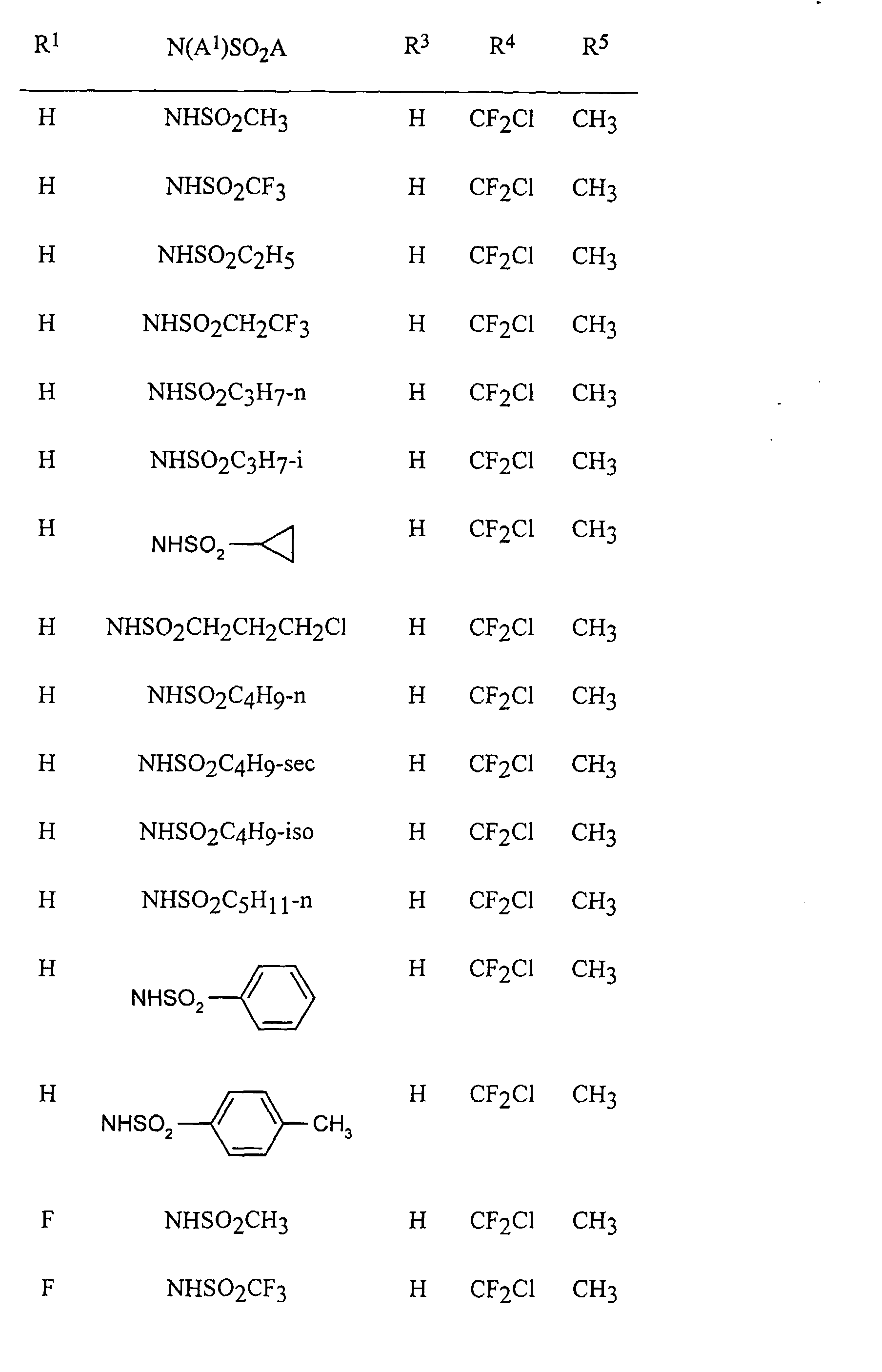

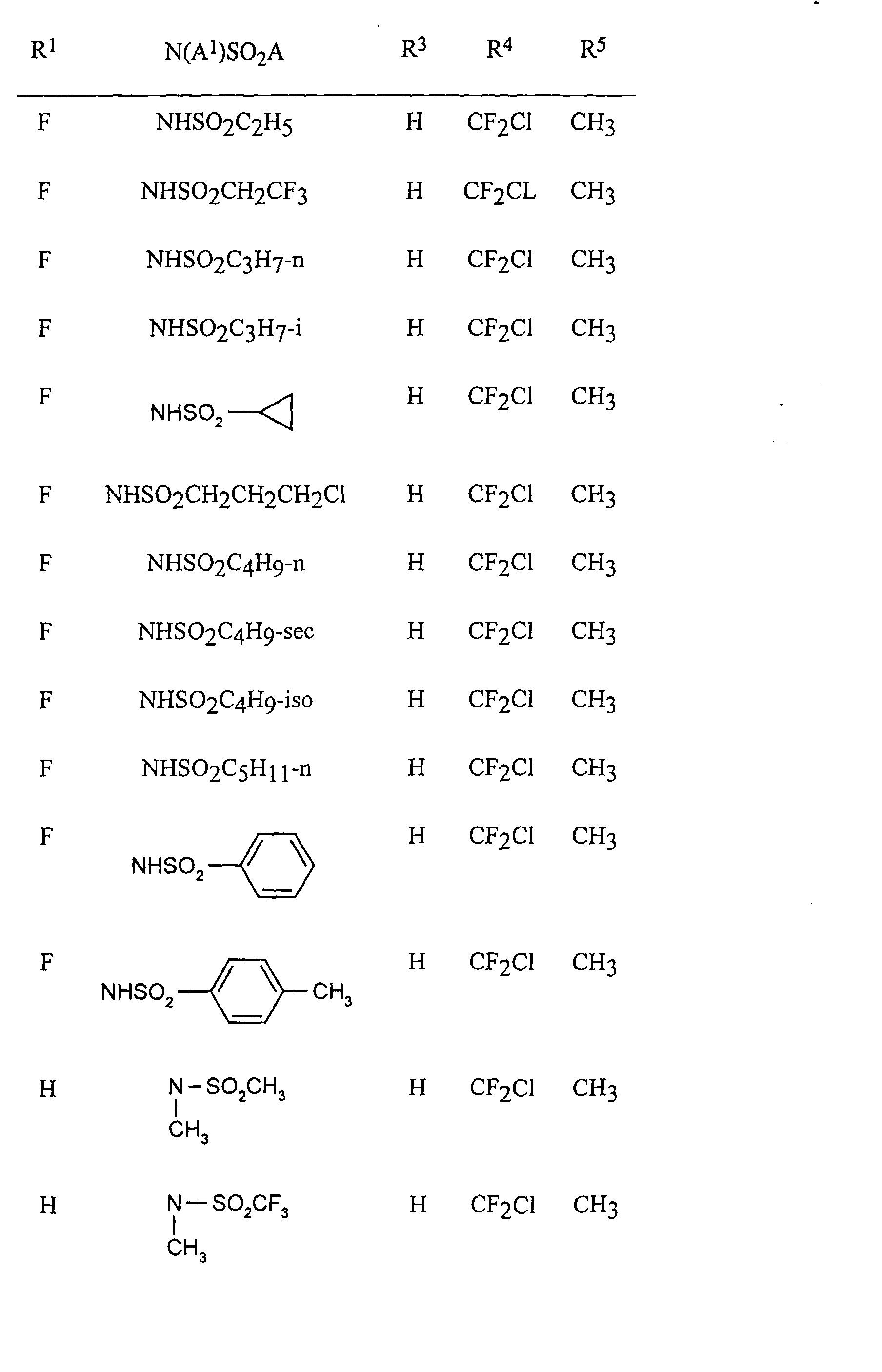

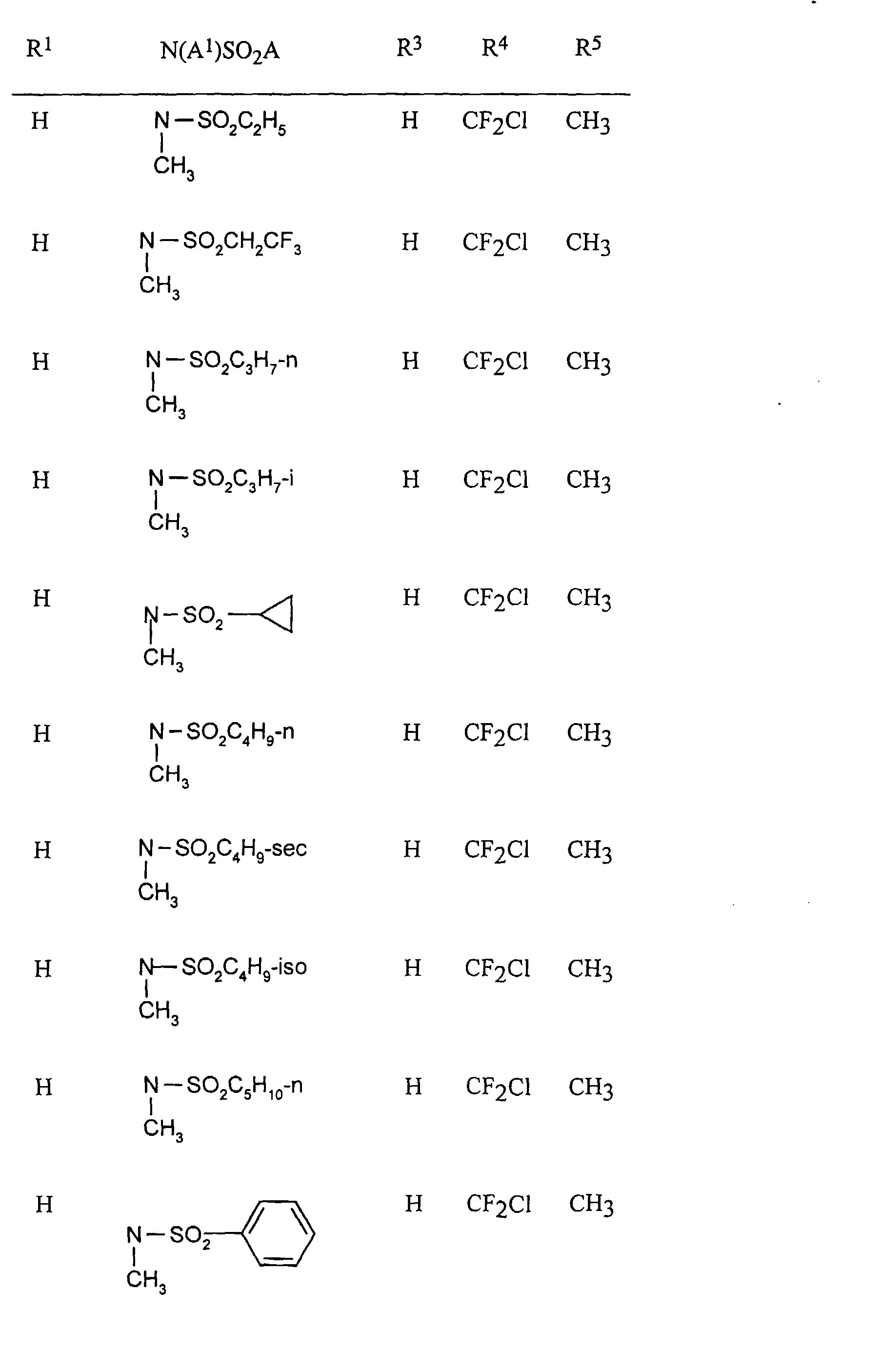

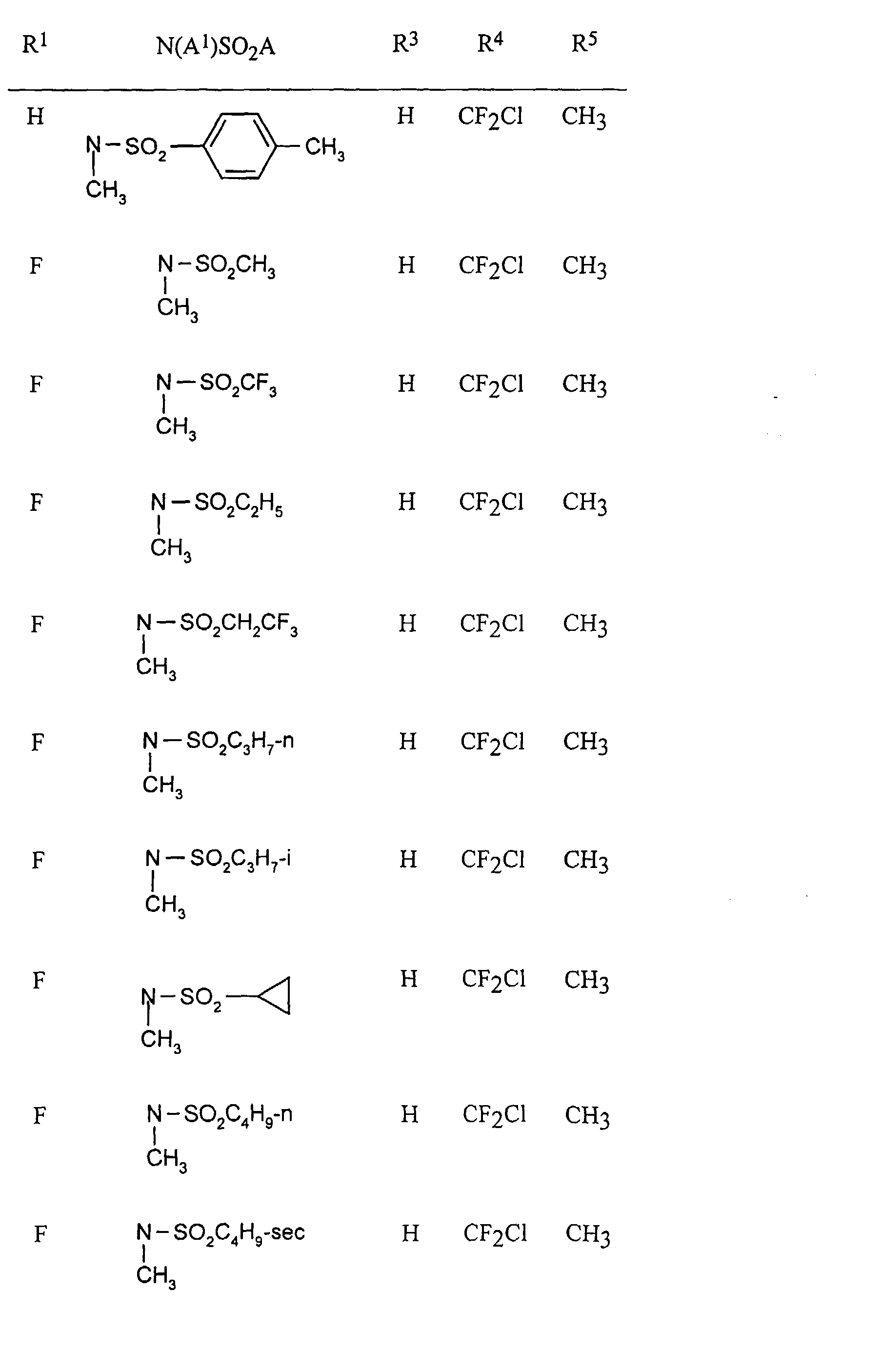

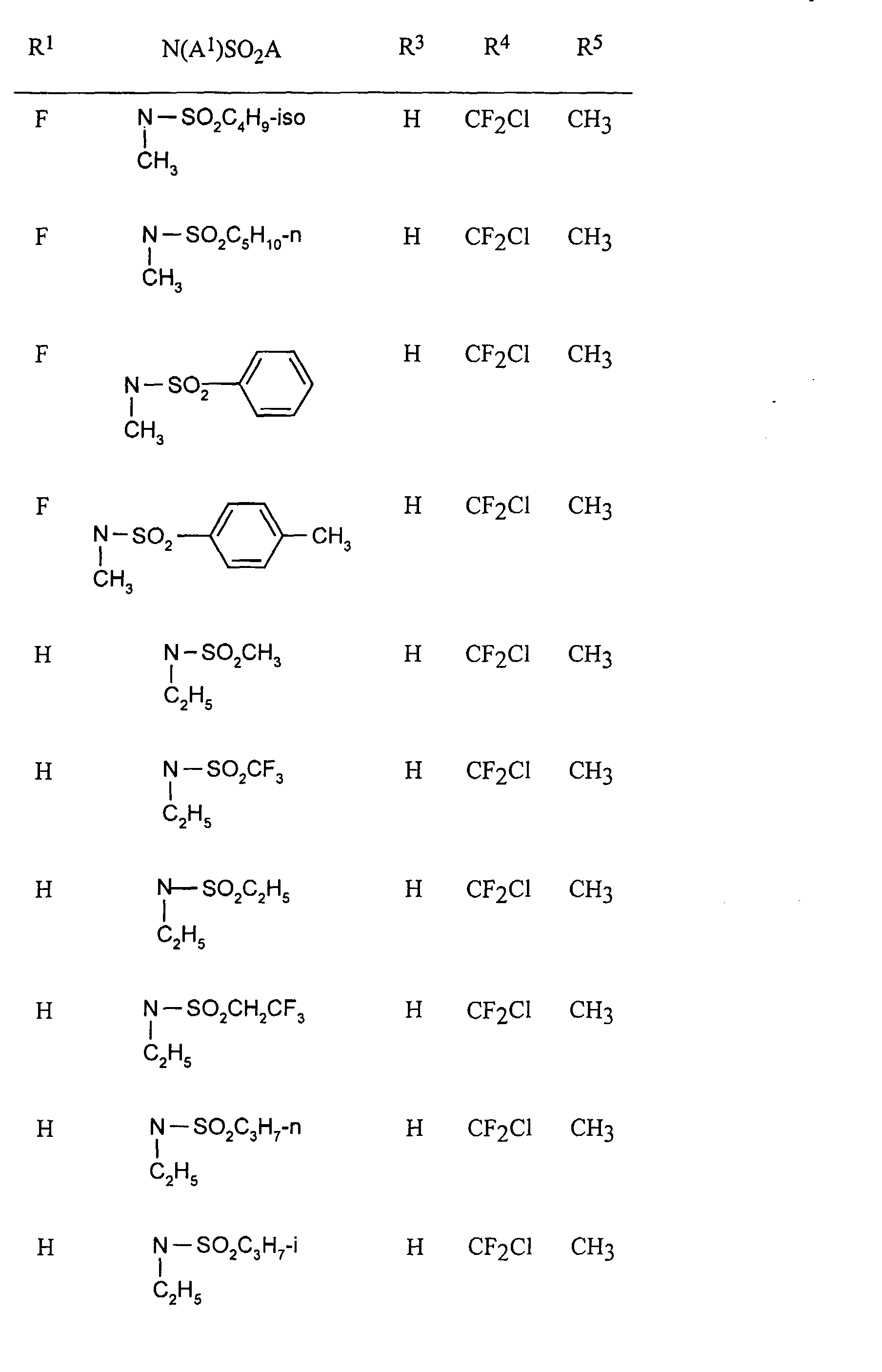

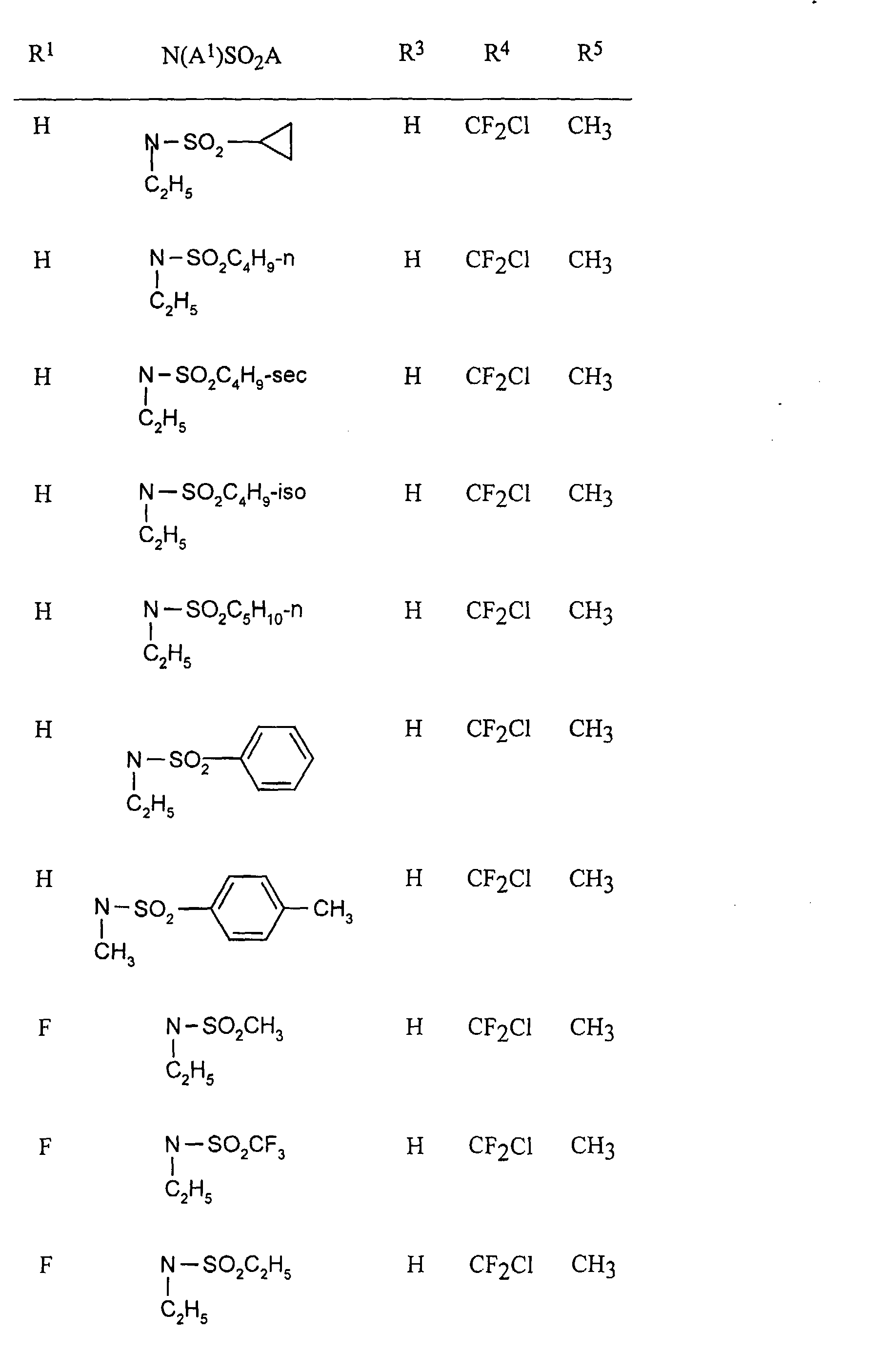

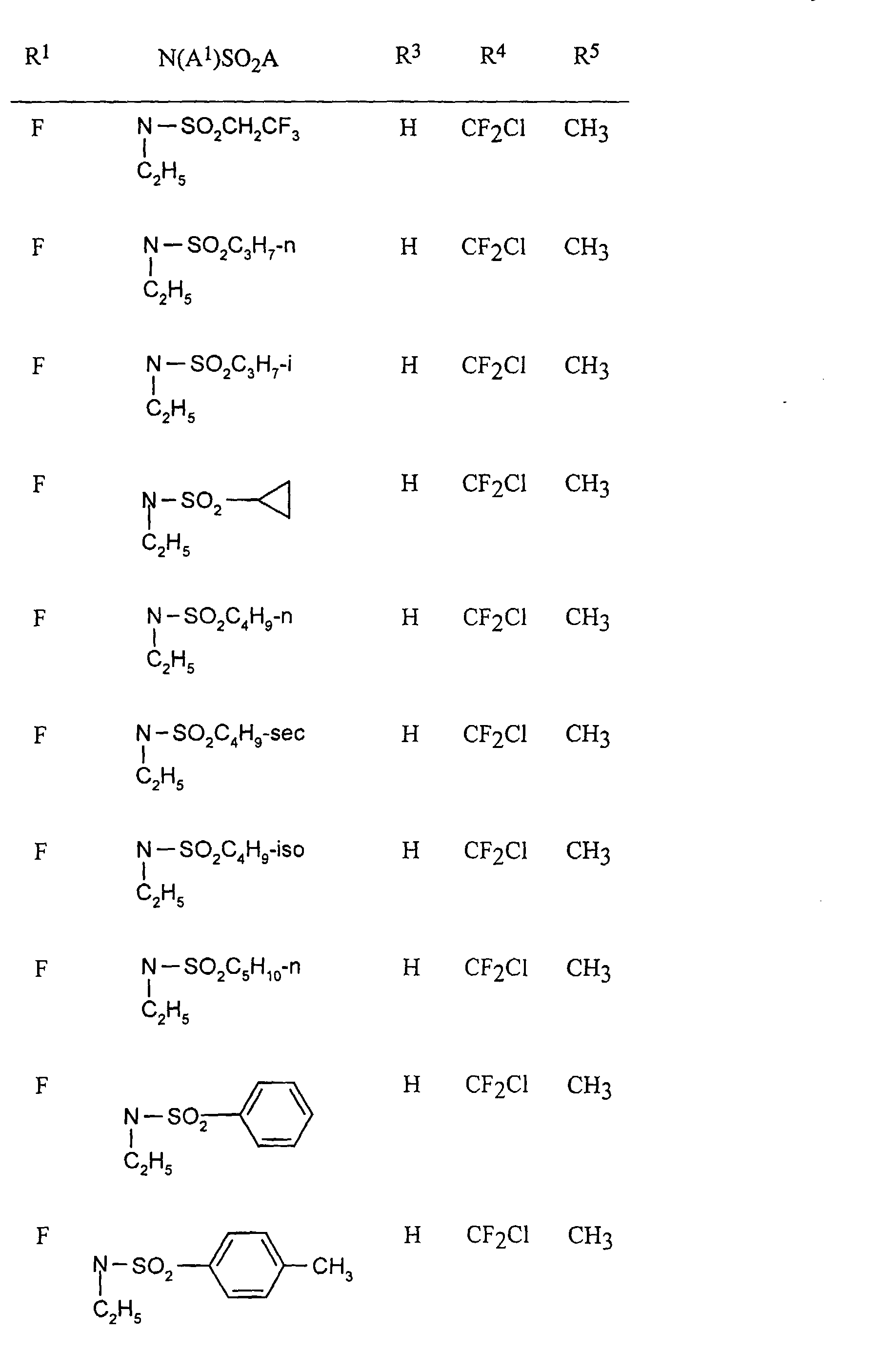

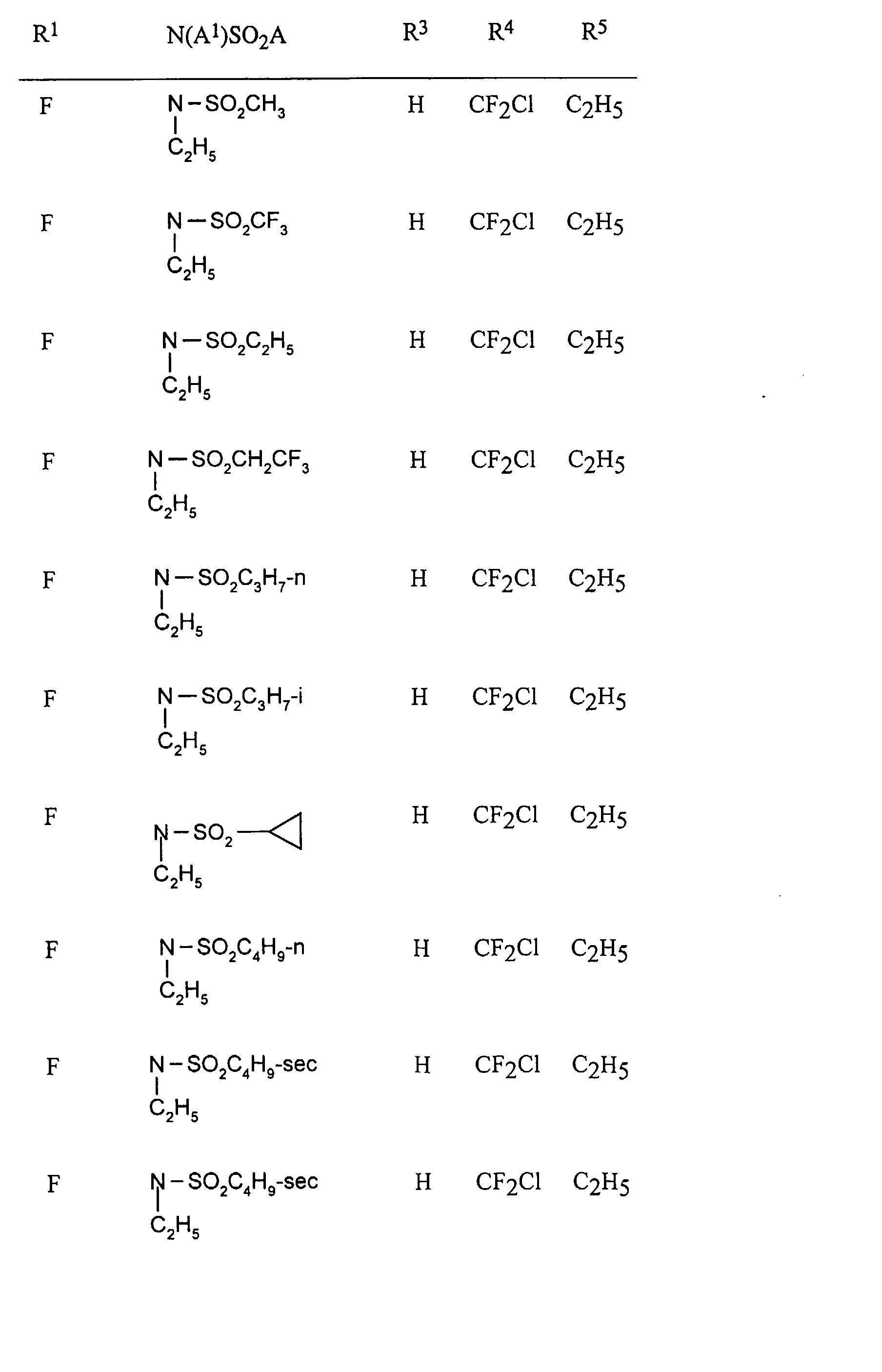

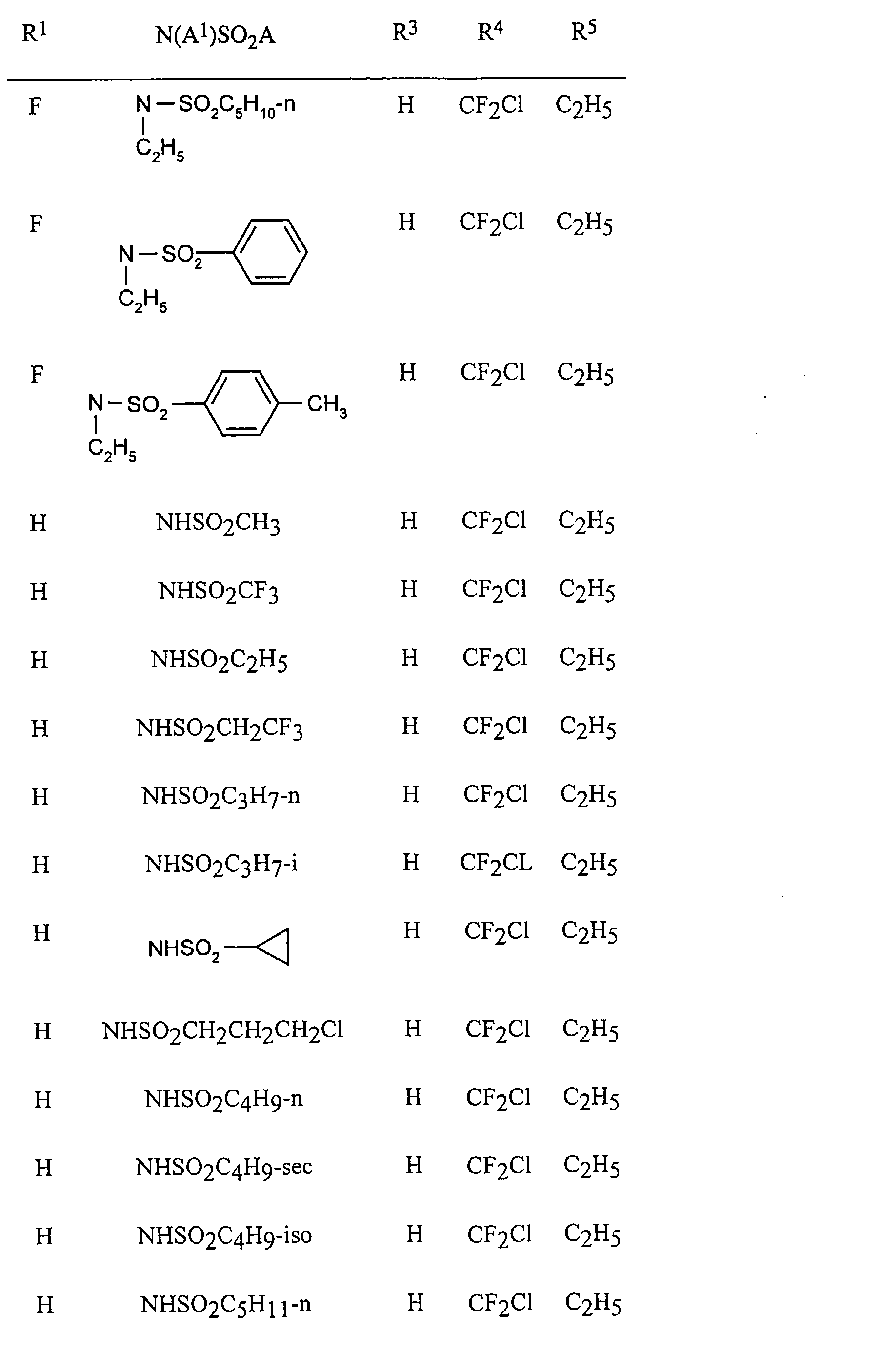

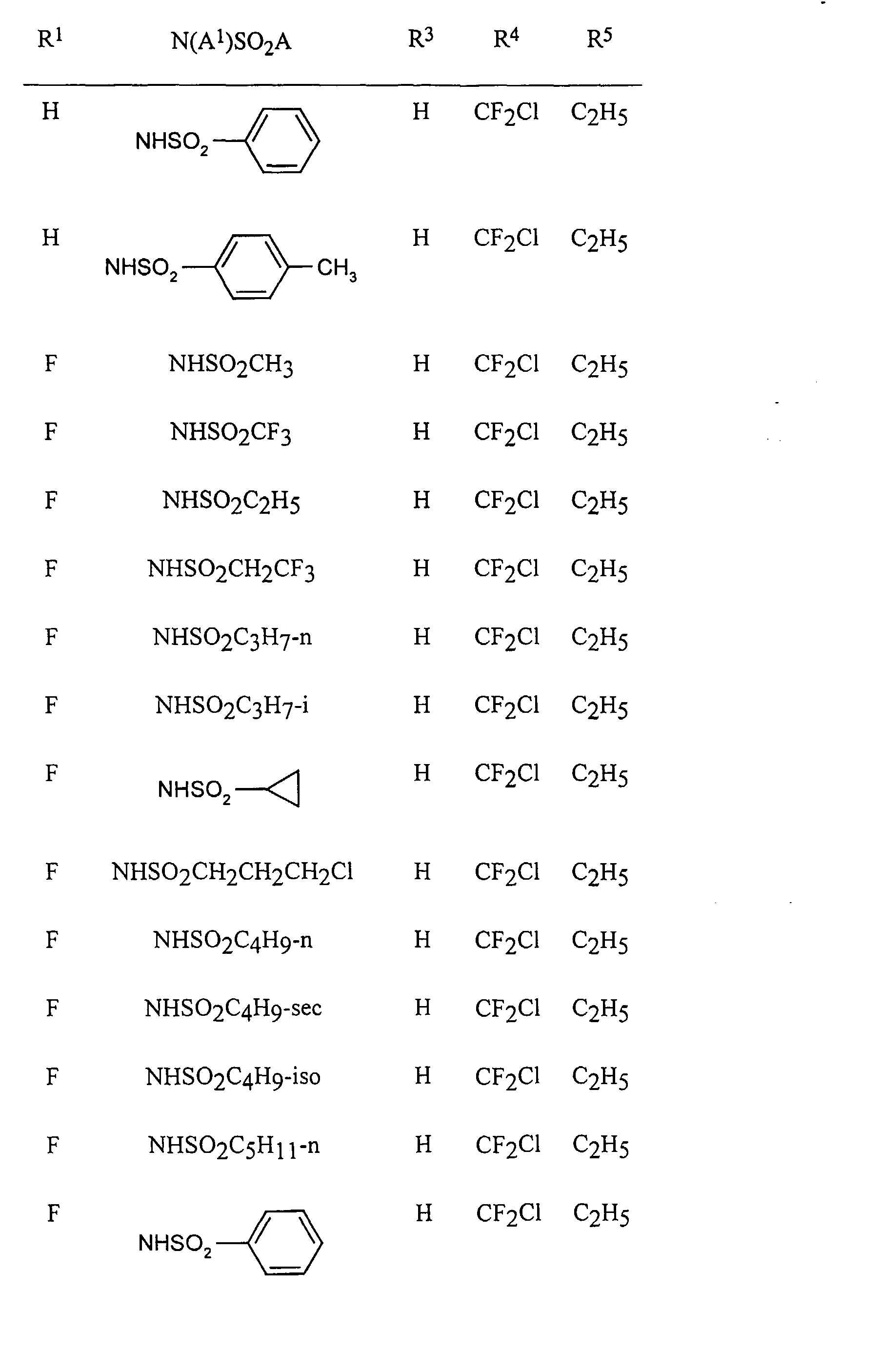

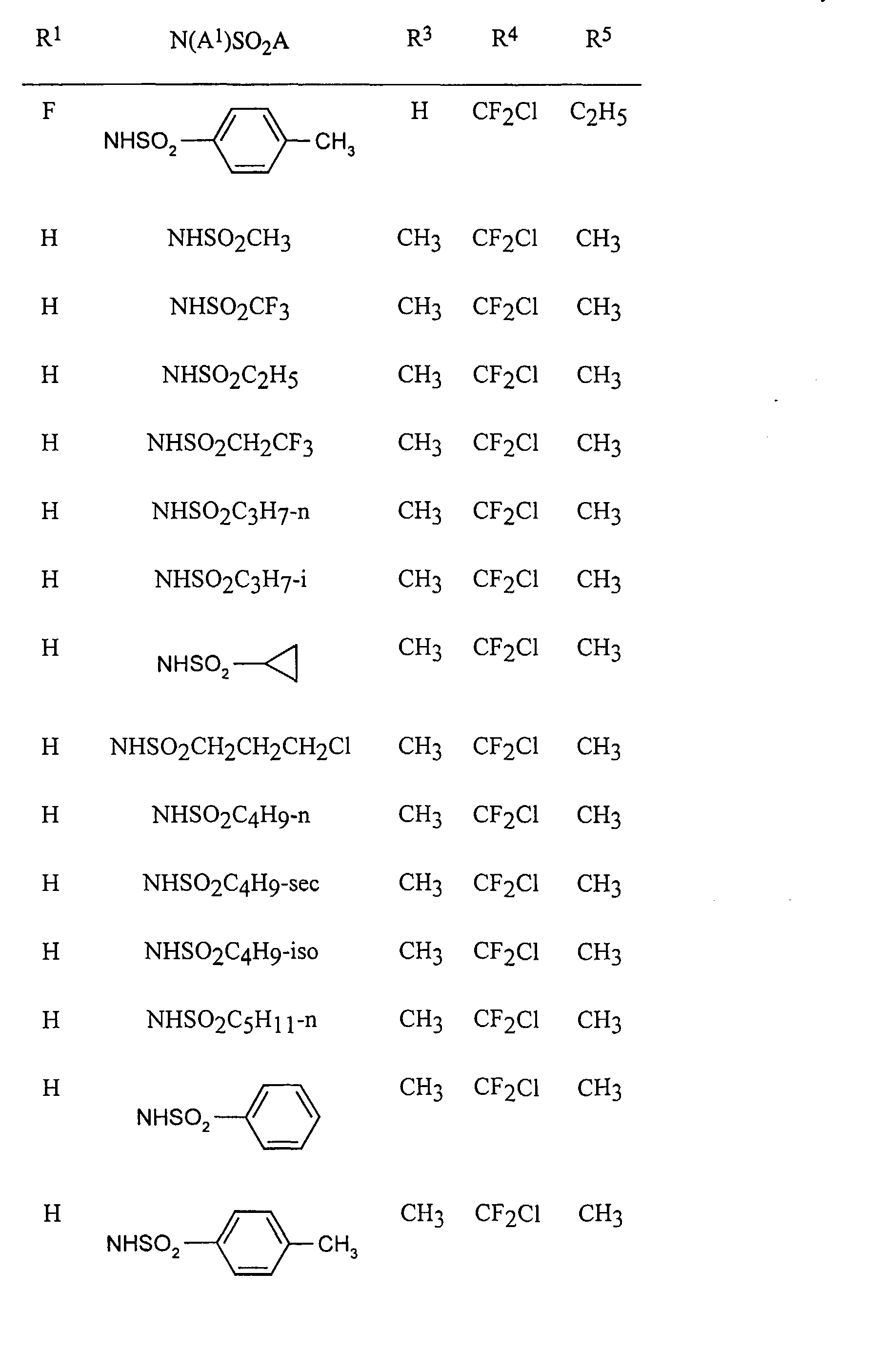

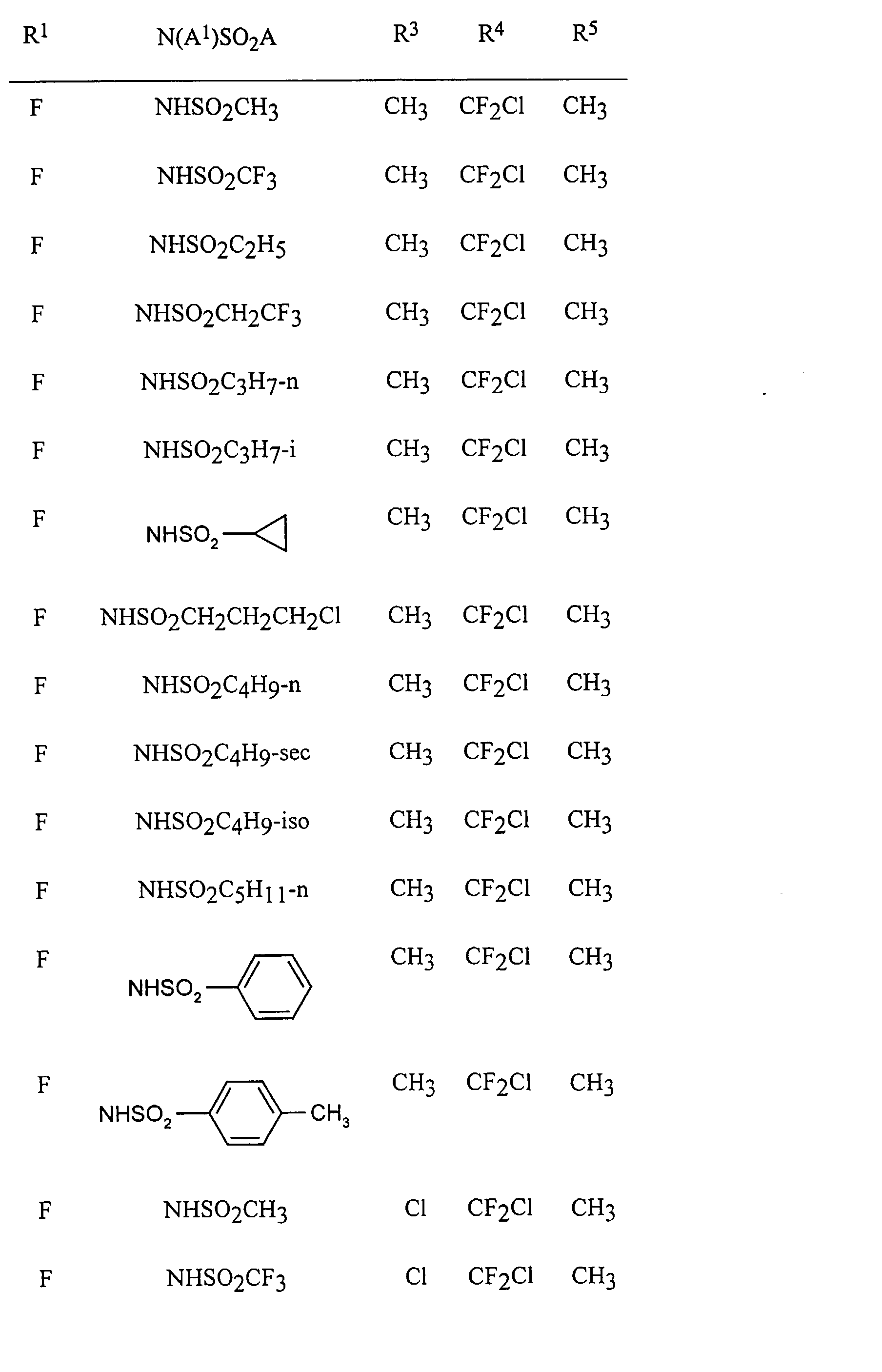

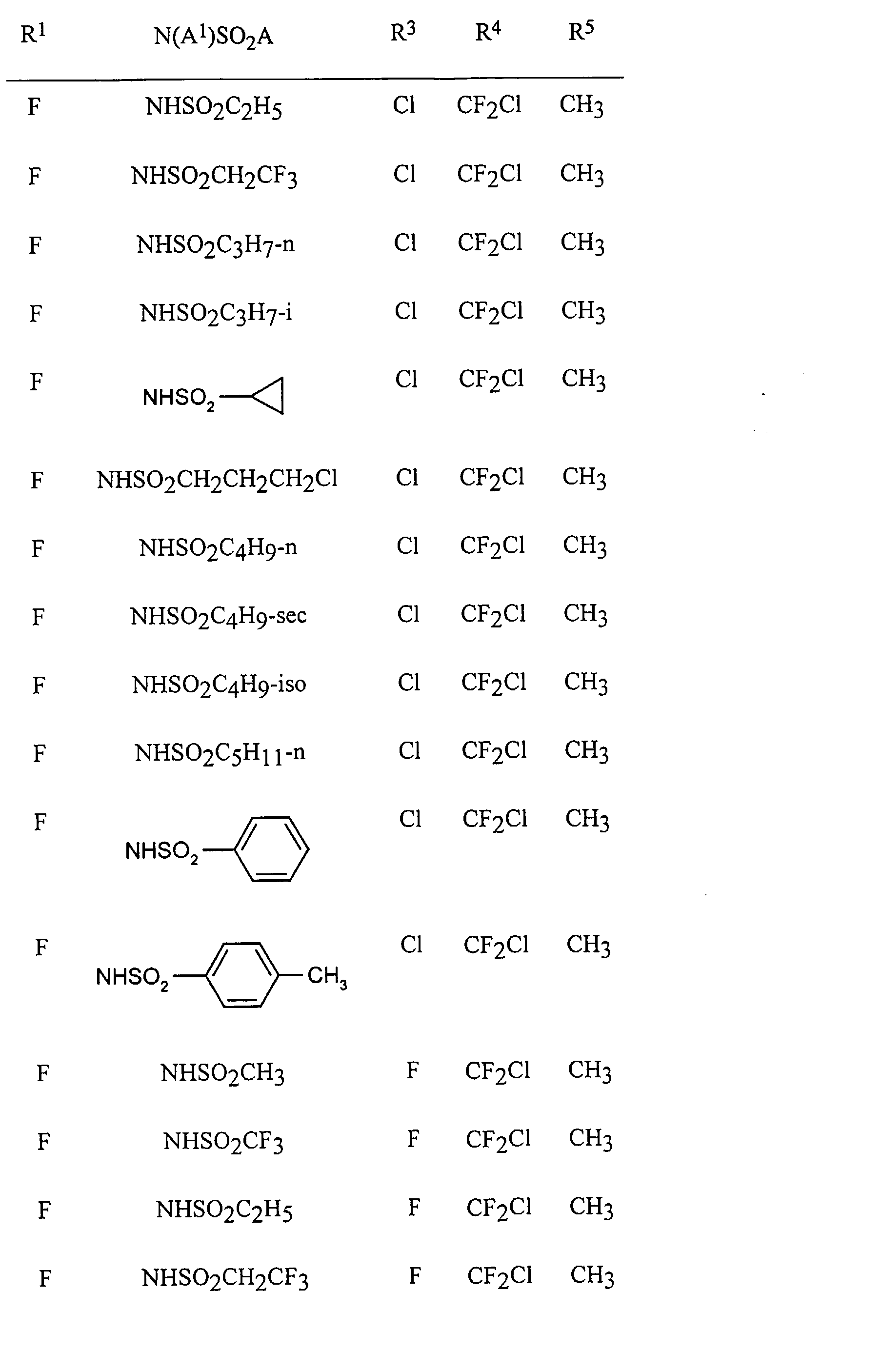

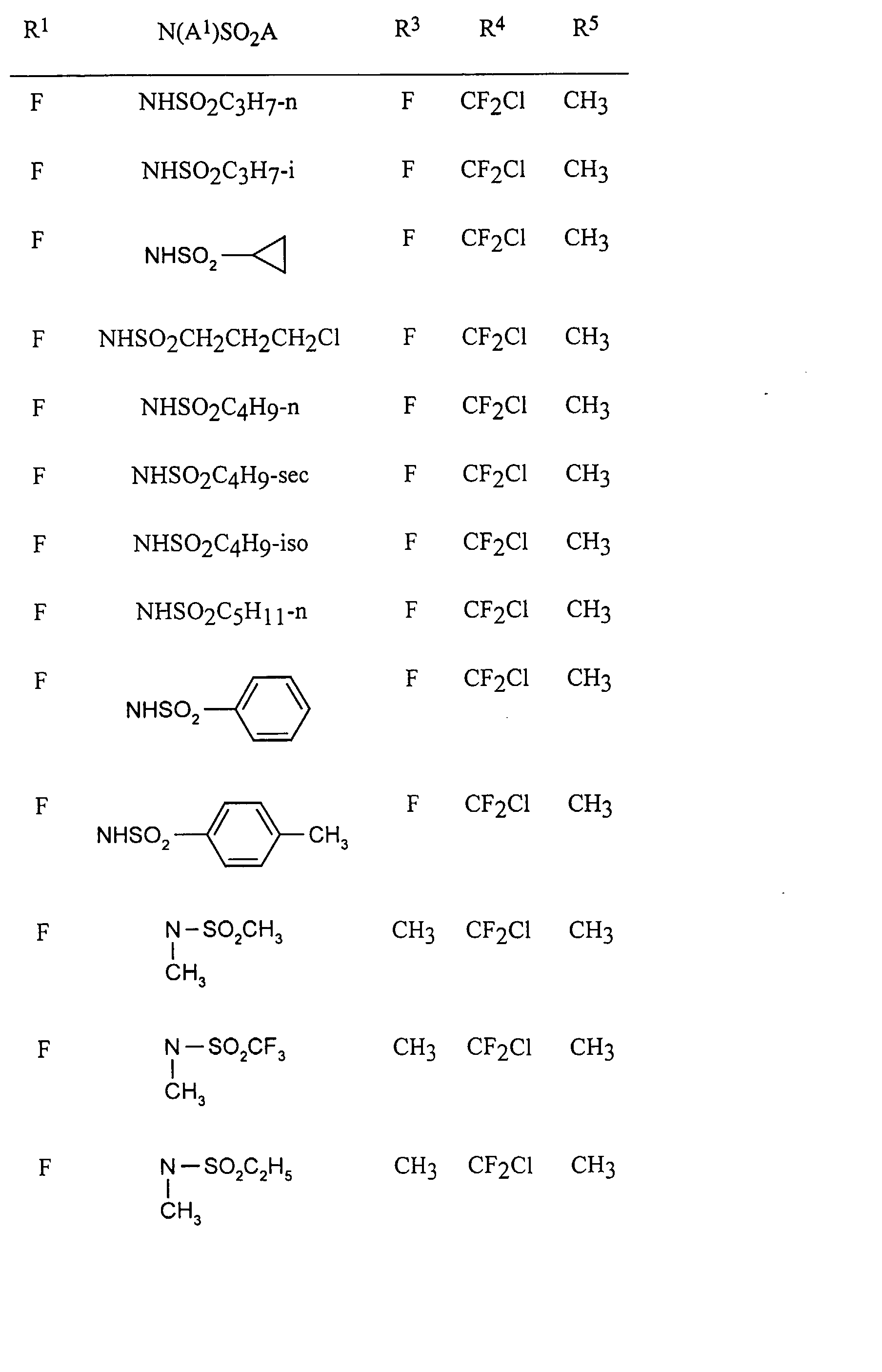

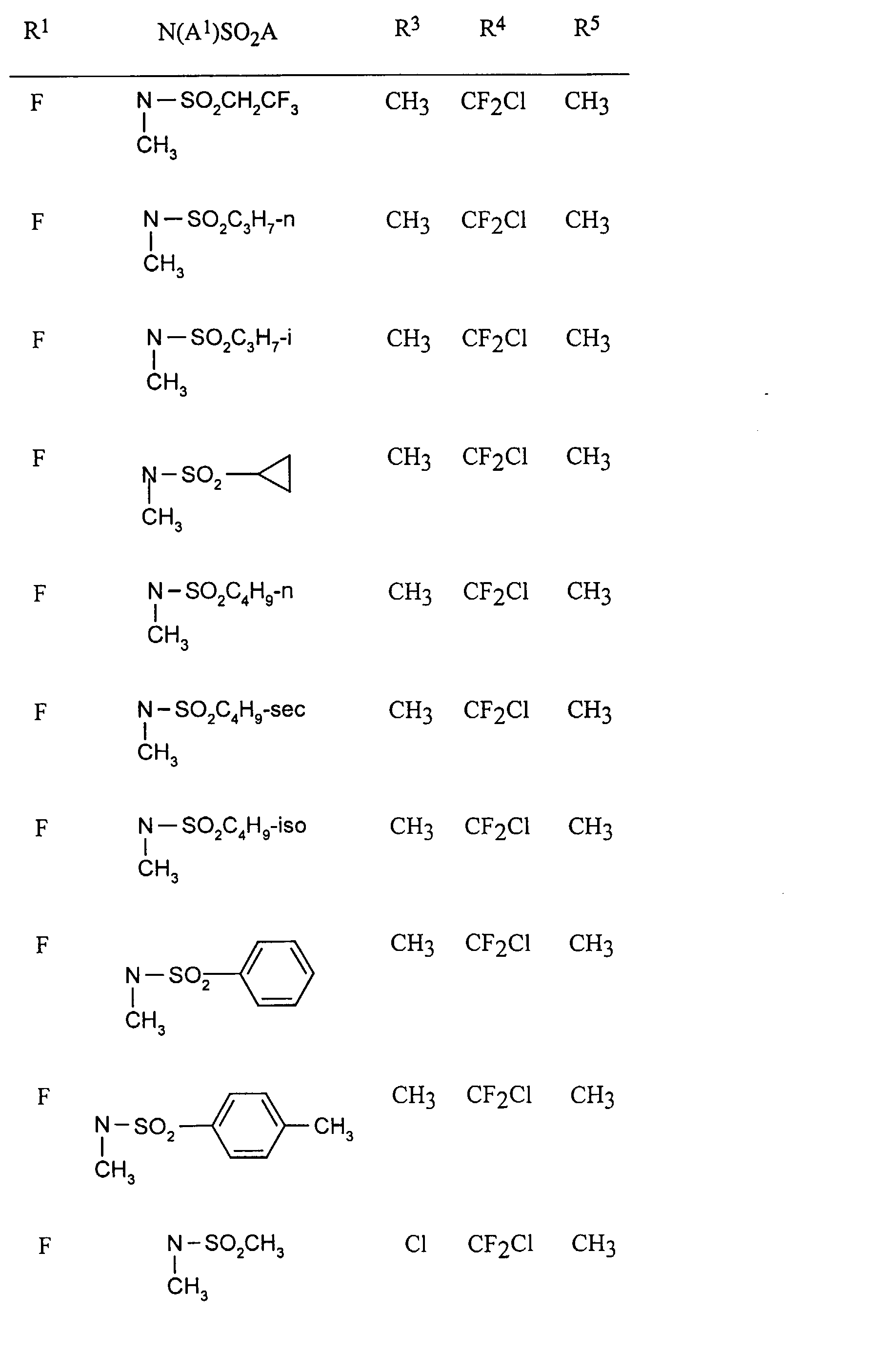

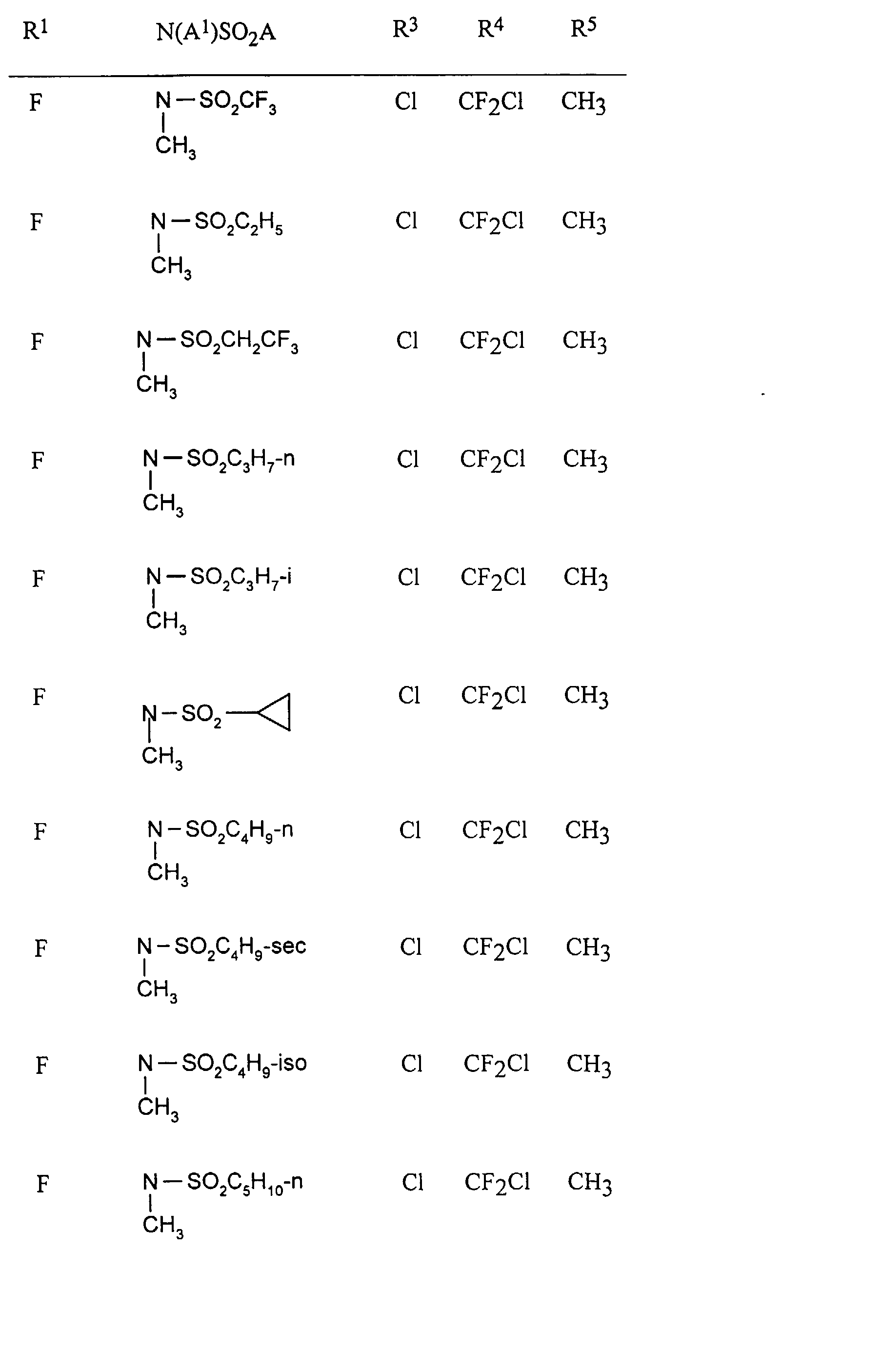

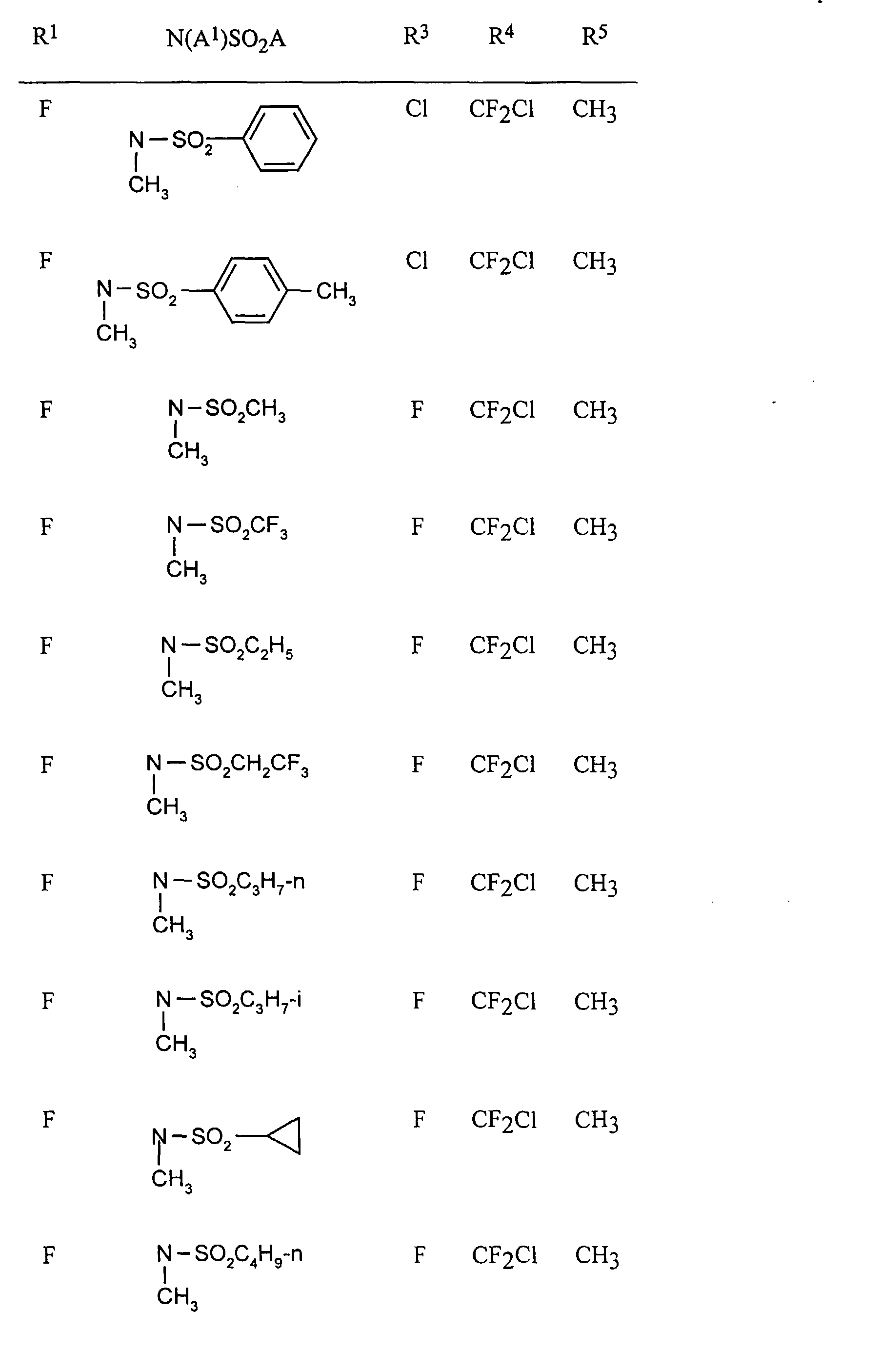

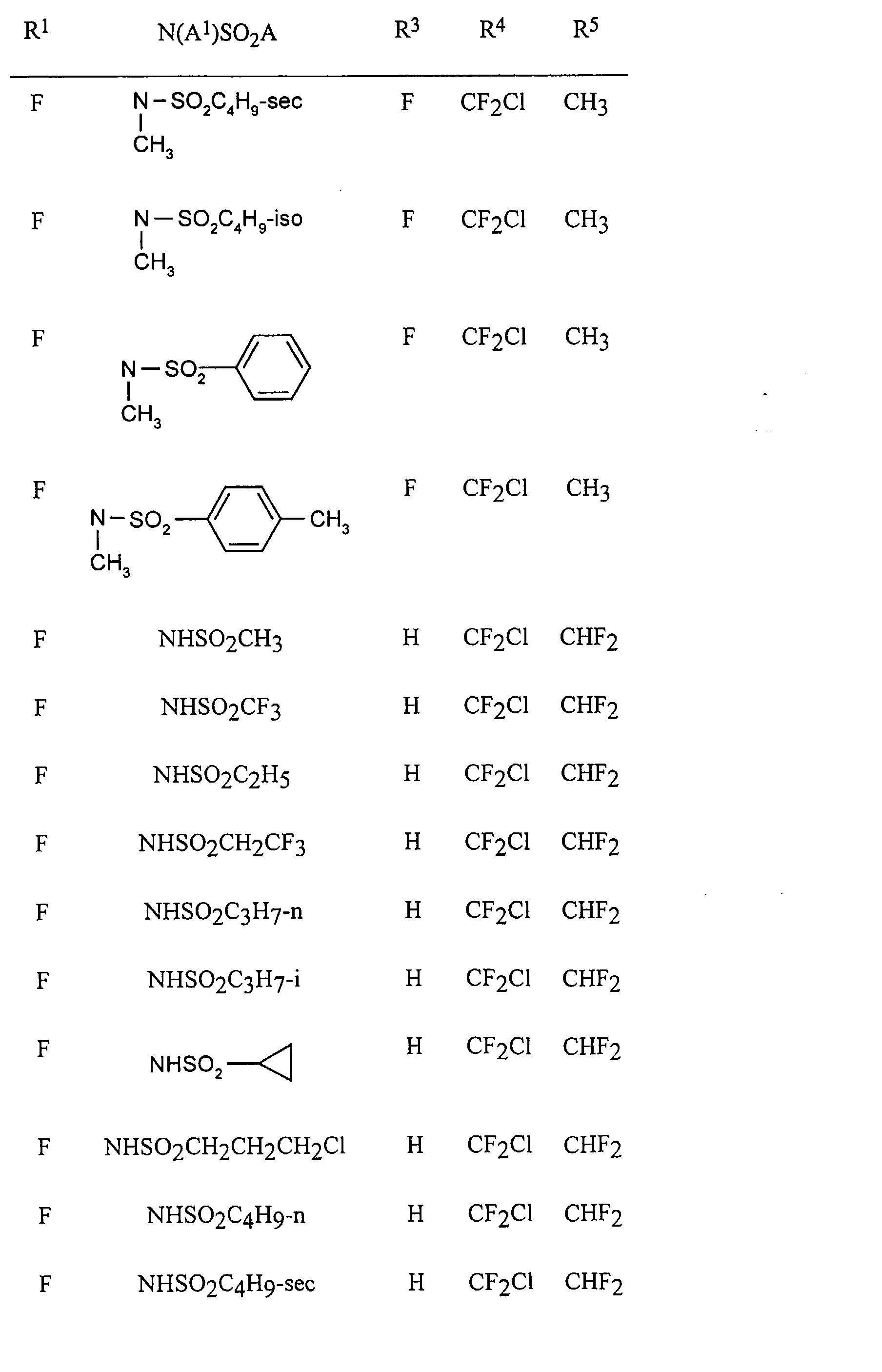

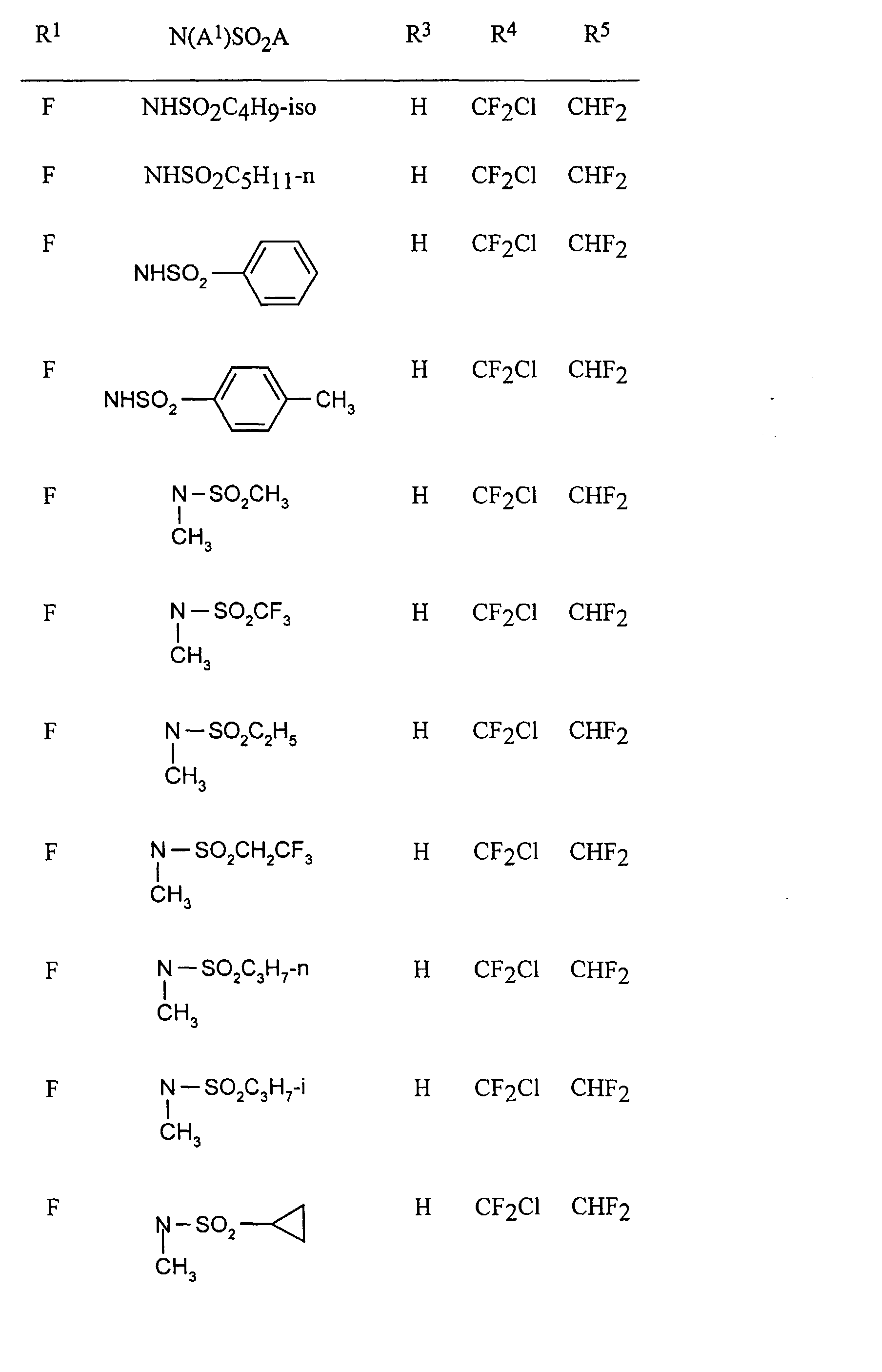

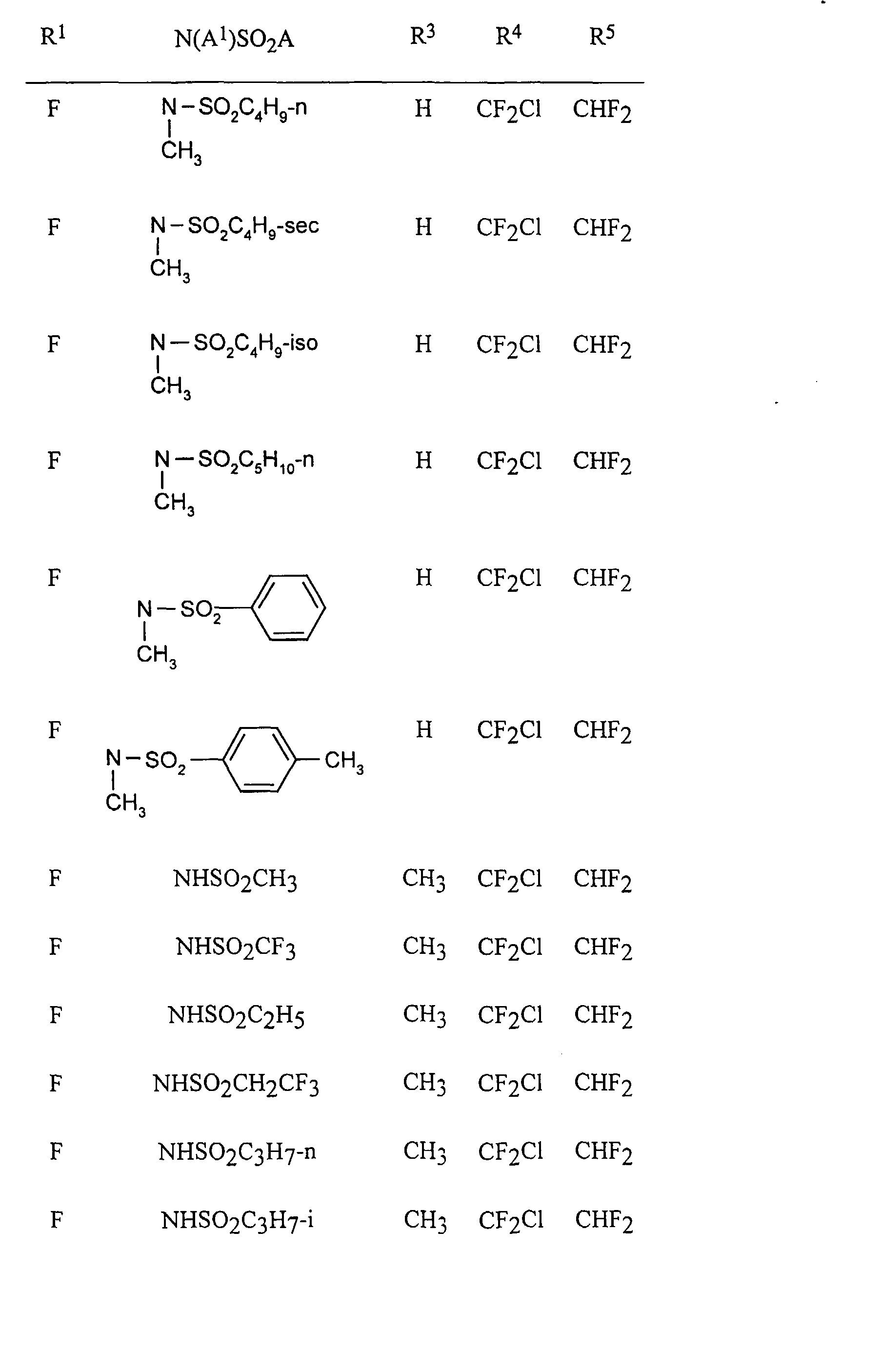

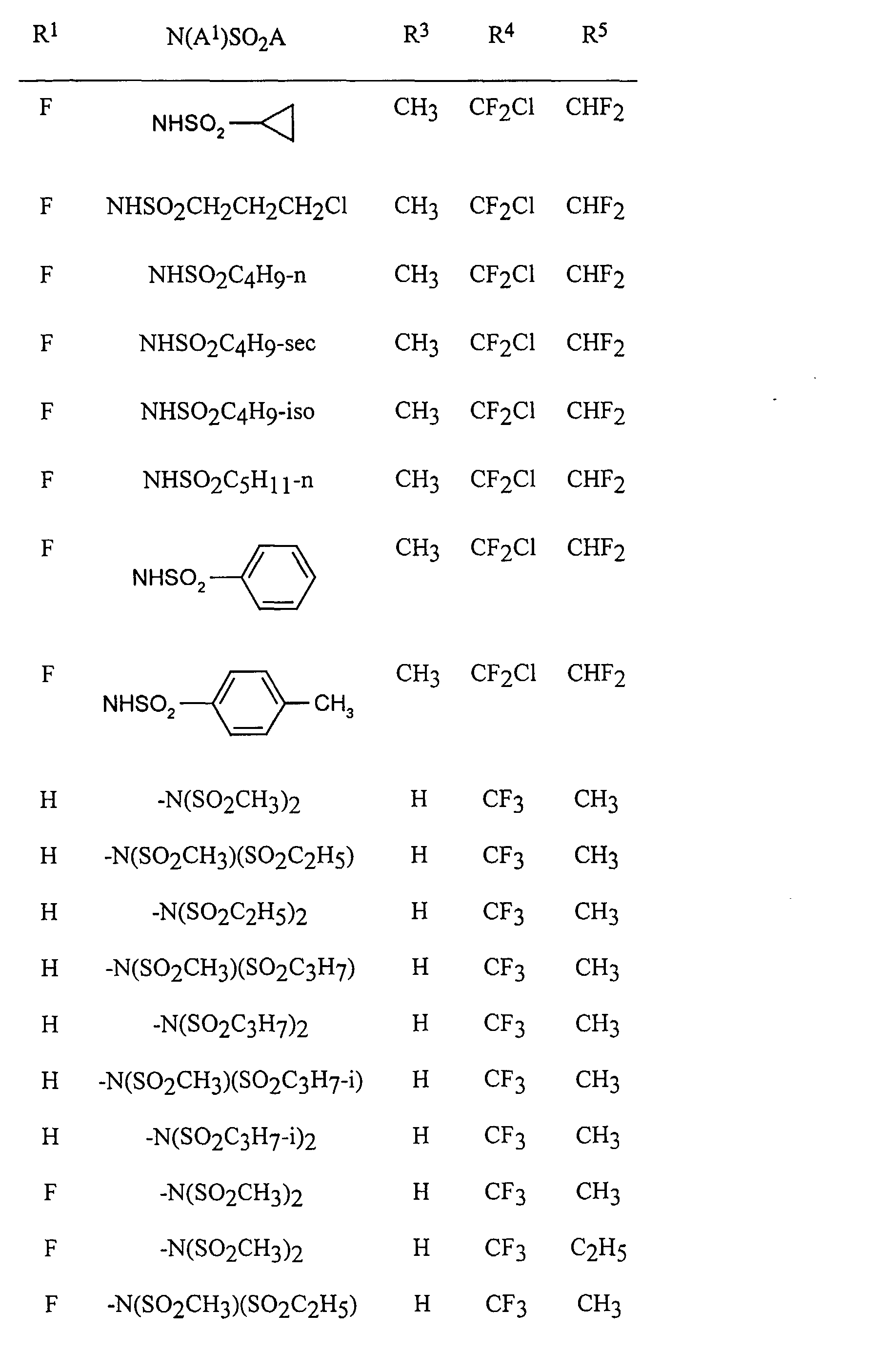

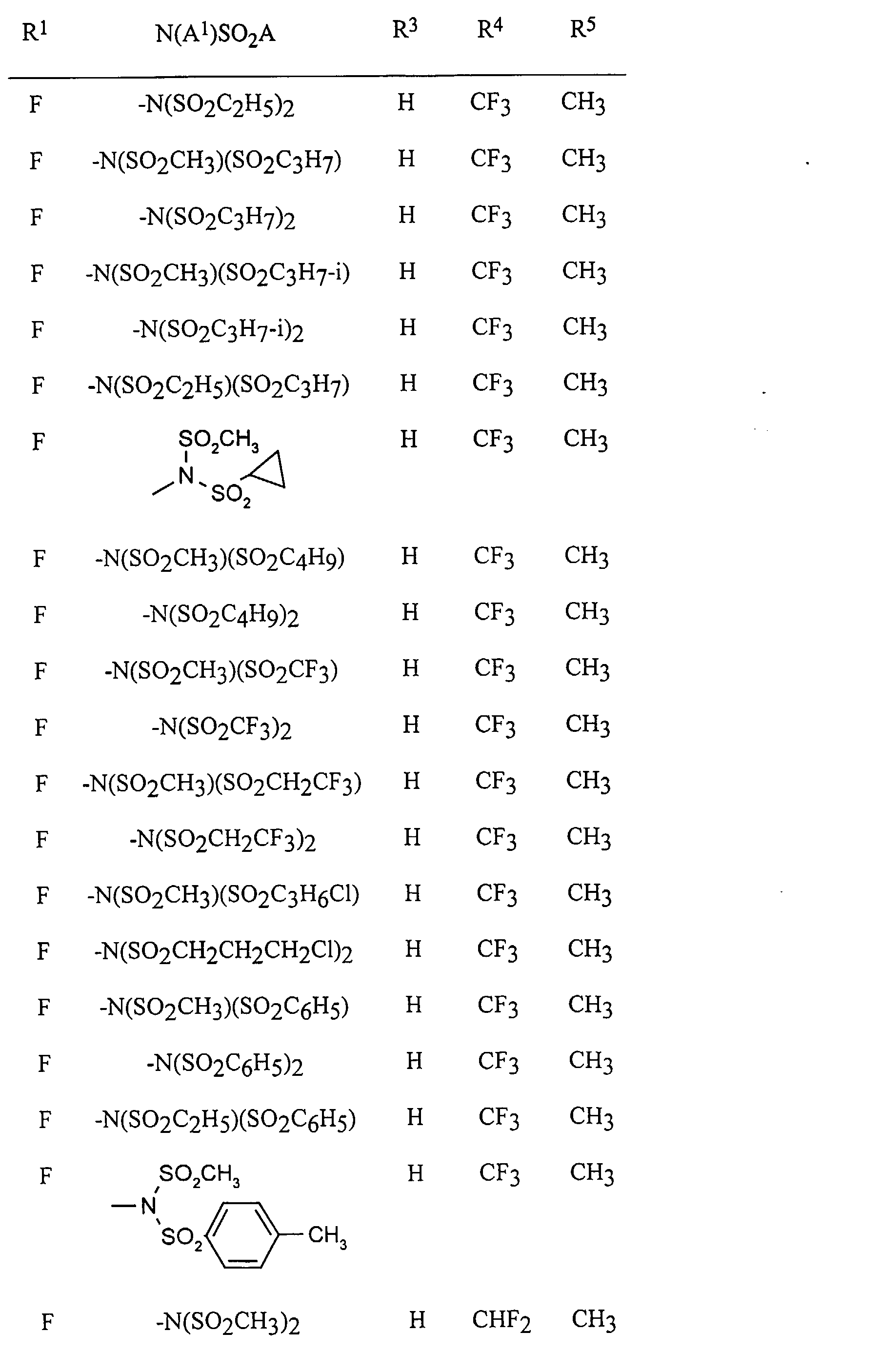

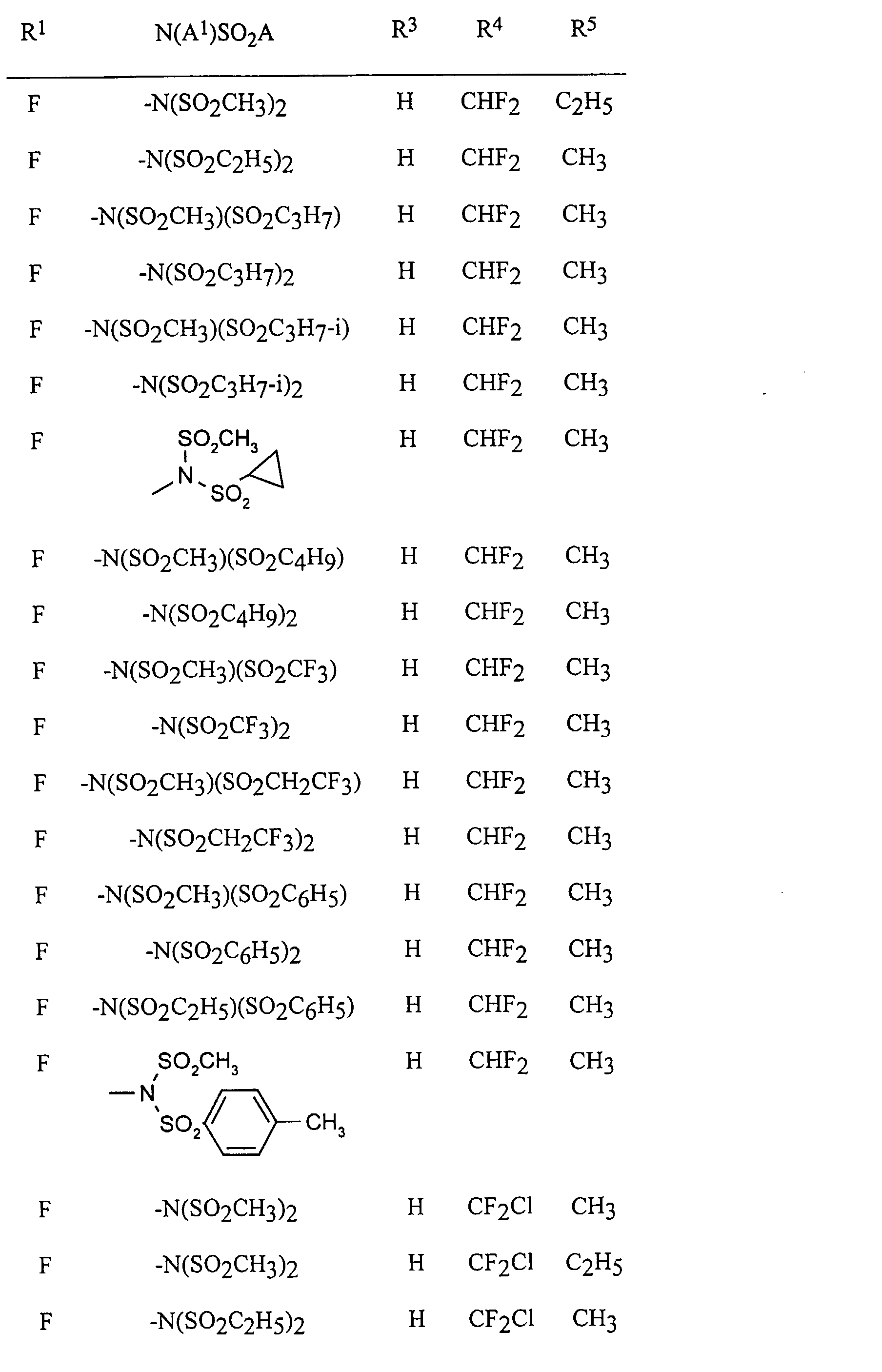

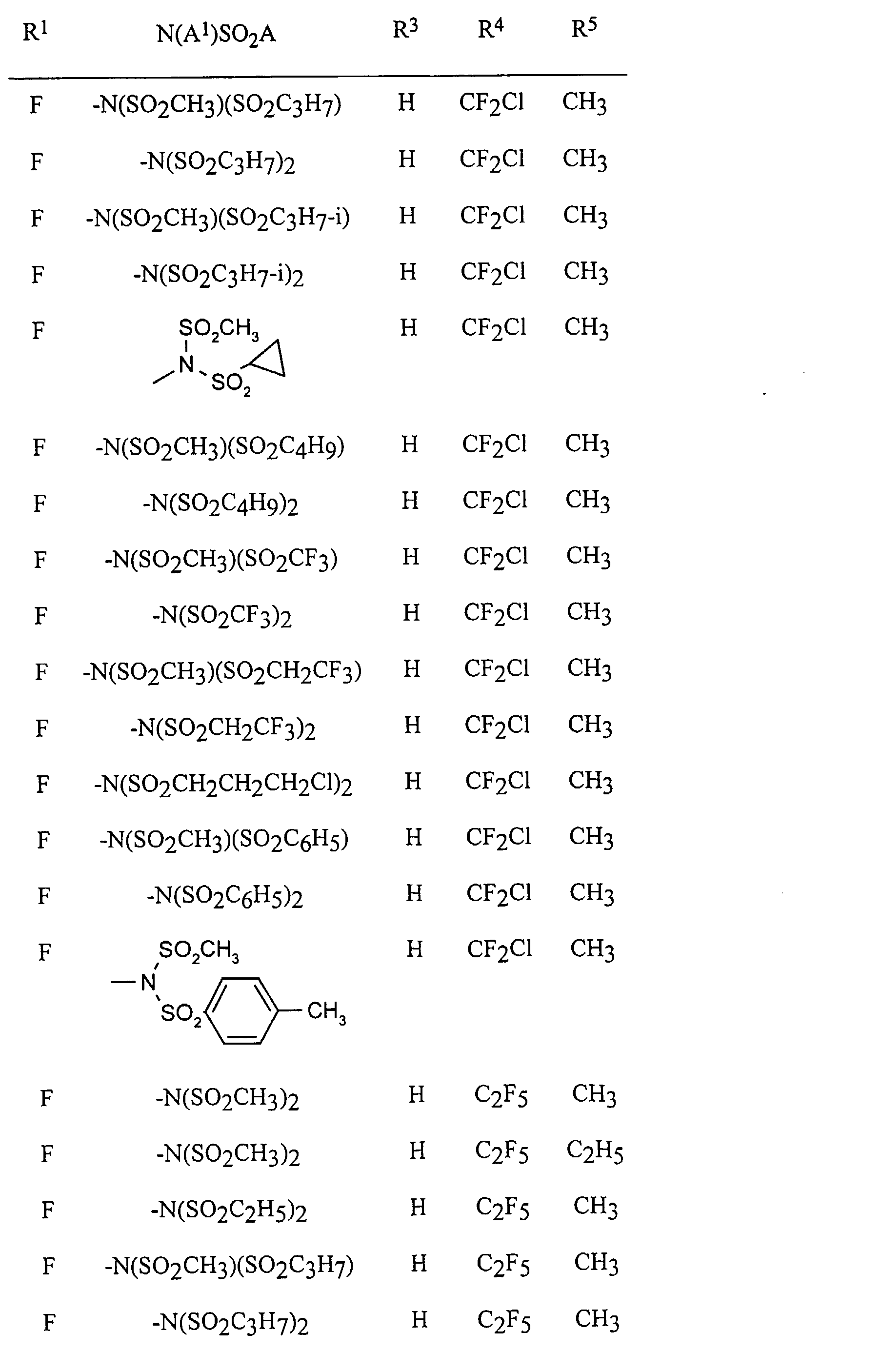

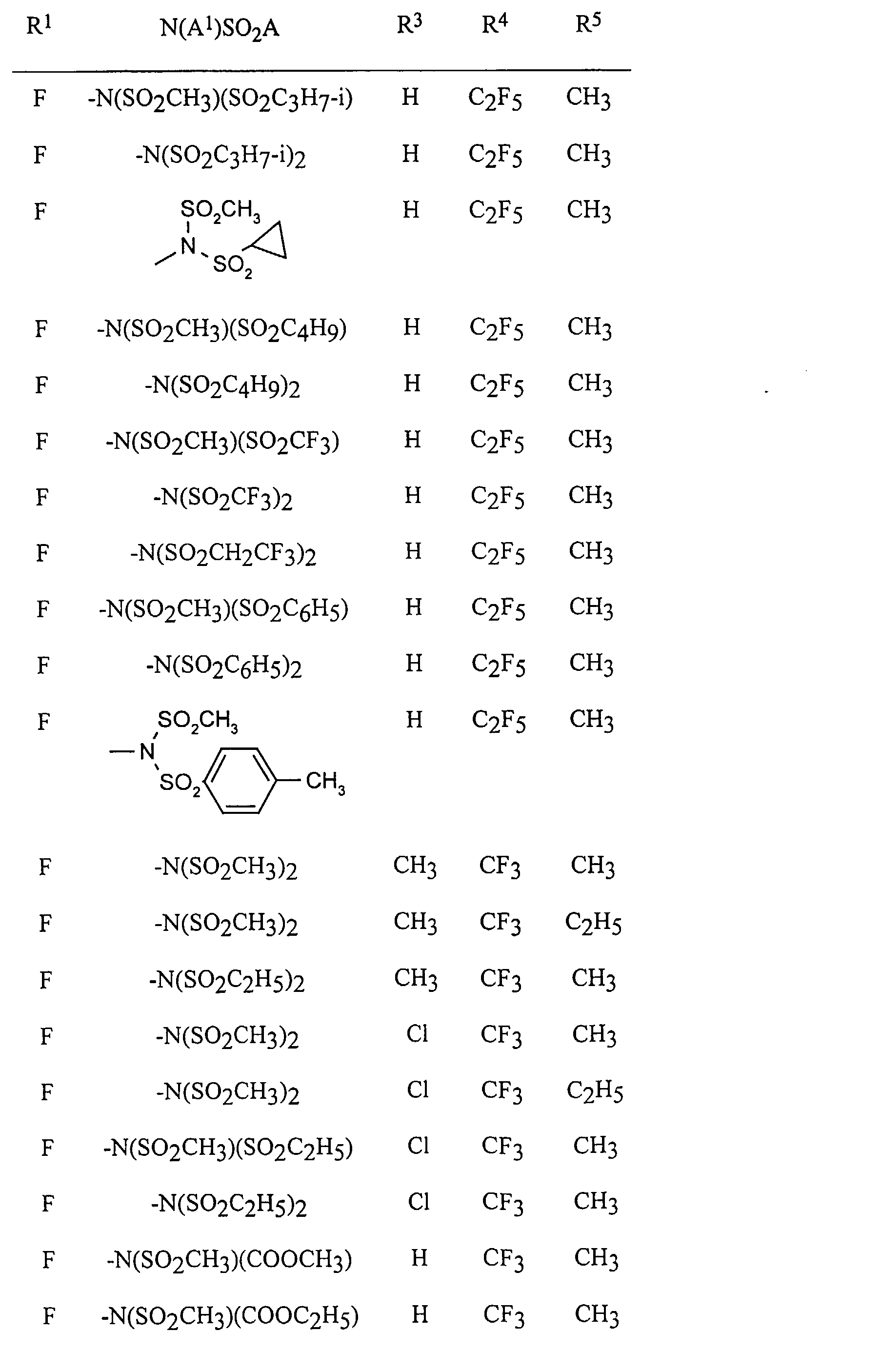

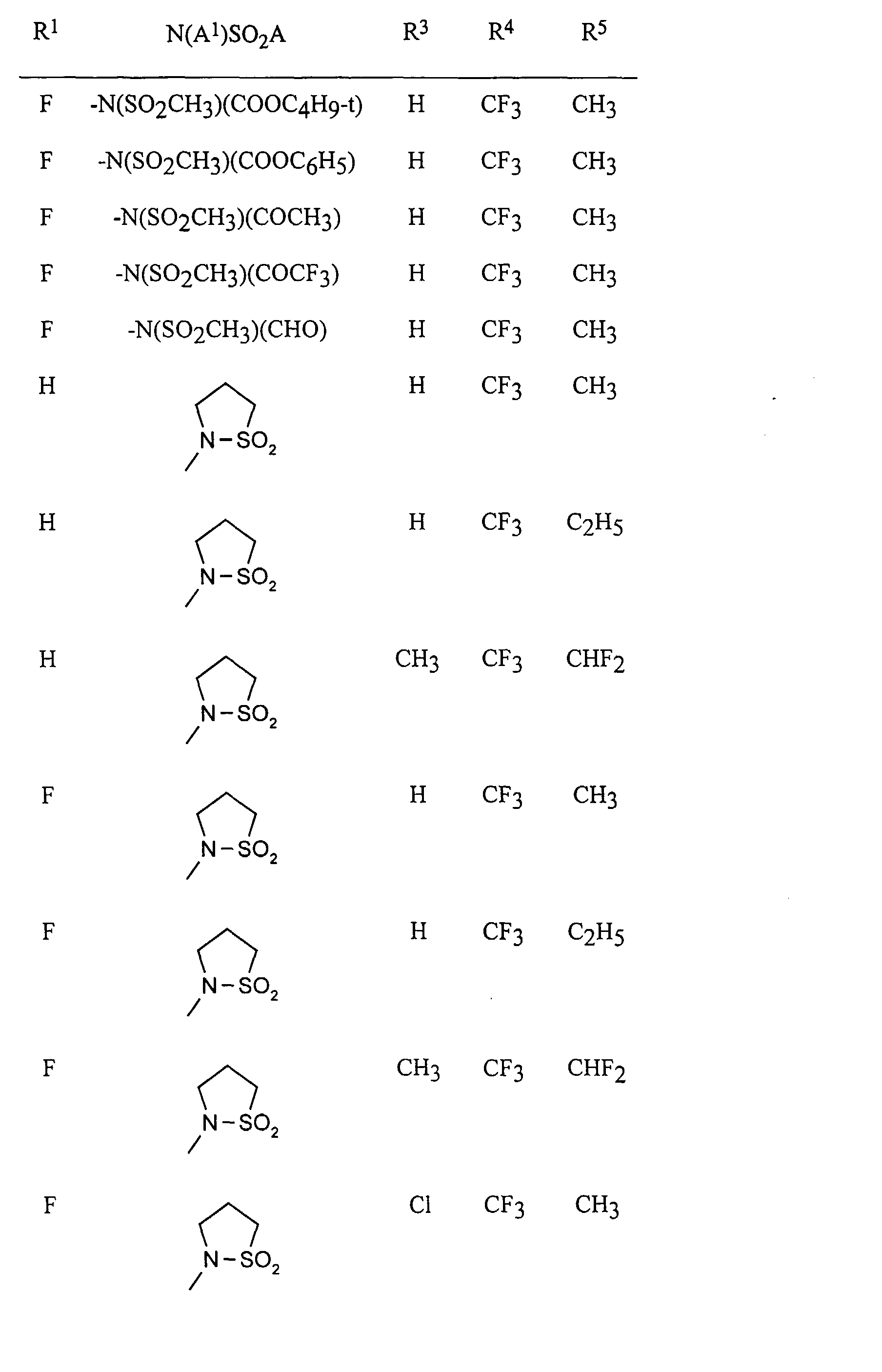

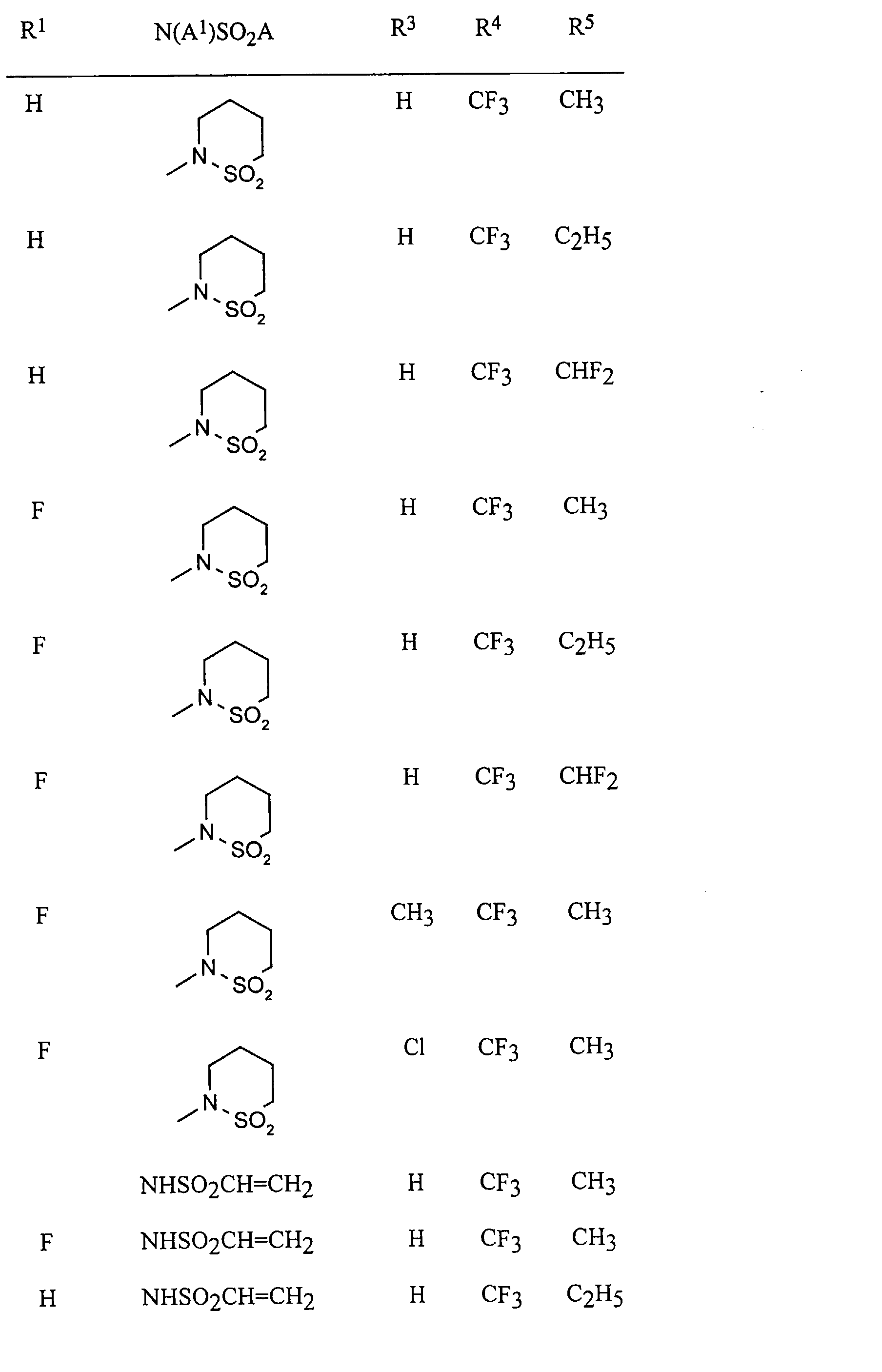

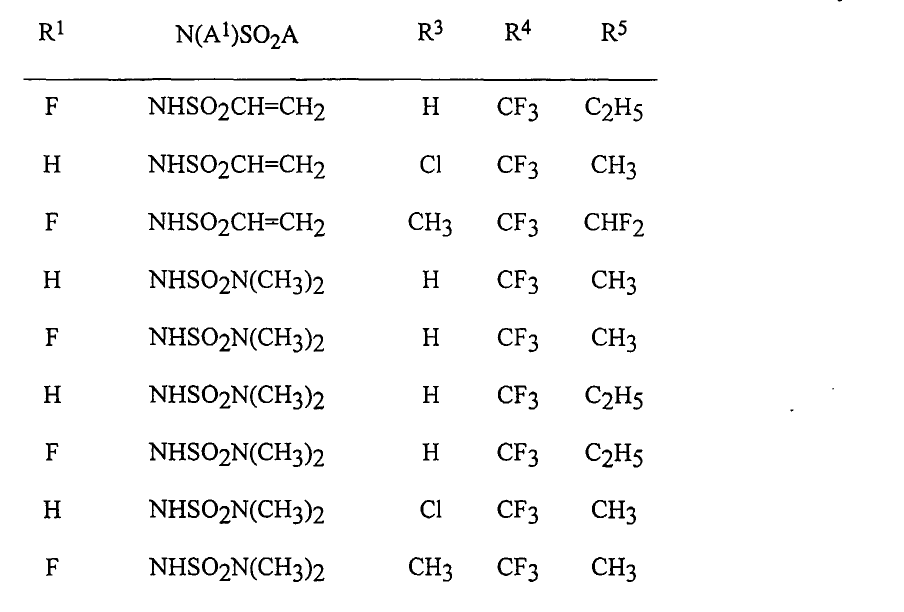

Die beim erfindungsgemäßen Verfahren (a) zur Herstellung von Verbindungen der Formel (I) als Ausgangsstoffe zu verwendenden Aminoalkensäure(thio)ester sind durch die Formel (II) allgemein definiert.

In Formel (II) haben R³ und R⁴ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R³ und R⁴ angegeben wurden;

R steht vorzugsweise für C₁-C₄-Alkyl, insbesondere für Methyl oder Ethyl.

Die Ausgangsstoffe der Formel (II) sind bekannt und/oder können nach bekannten Verfahren hergestellt werden (vgl. J. Heterocycl. Chem. 9 (1972), 513-522).

Die beim erfindungsgemäßen Verfahren (a) weiter als Ausgangsstoffe zu verwendenden Cyanoaryliso(thio)cyanate sind durch die Formel (III) allgemein definiert.

In Formel (III) hat R¹ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ angegeben wurde.

Die Ausgangsstoffe der Formel (III) sind bekannt und/oder können nach bekannten Verfahren hergestellt werden (vgl. J. Org. Chem. 30 (1965), 2465-2466; vgl. auch DE-P 4327743 und DE-P 4335438).

Man erhält die Cyanoaryliso(thio)cyanate der Formel (III), wenn man entsprechende Cyanoarylamine der allgemeinen Formel (VII) 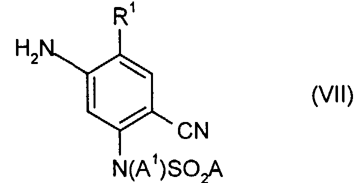 in welcher
- R¹: die oben angegebene Bedeutung hat,
mit Phosgen bzw. Thiophosgen, gegebenenfalls in Gegenwart eines Reaktionshilfsmittels, wie z.B. Calciumcarbonat, und gegebenenfalls in Gegenwart von Verdünnungsmitteln, wie z.B. Methylenchlorid, Toluol, Chlorbenzol und gegebenenfalls Wasser, bei Temperaturen zwischen 0°C und 150°C umsetzt (vgl. die Herstellungsbeispiele).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Betracht. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Das erfindungsgemäße Verfahren (a) wird vorzugsweise in Gegenwart eines Reaktionshilfsmittels durchgeführt. Als Reaktionshilfsmittel sind hierbei vor allem Säureakzeptoren geeignet. Vorzugsweise infrage kommen Alkalimetall- und Erdalkalimetall-hydride, wie Lithium-, Natrium-, Kalium- und Calcium-hydrid, Alkalimetallund Erdalkalimetall-hydroxide, wie Lithium-, Natrium-, Kalium- und Calciumhydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen -120°C und +100°C, vorzugsweise bei Temperaturen zwischen -70°C und +80°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Die beim erfindungsgemäßen Verfahren (b) zur Herstellung von Verbindungen der Formel (I) als Ausgangsstoffe zu verwendenden N-Cyanoaryl-Stickstoffheterocyclen sind durch die Formeln (I) - mit der Maßgabe, daß darin R⁵ für Wasserstoff steht-allgemein definiert.

In der Formel (I) haben R¹, R³ und R⁴ vorzugsweise bzw. insbesondere diejenigen Bedeutungen, die bereits oben im Zusammenhang mit der Beschreibung der erfindungsgemäßen Verbindungen der Formel (I) vorzugsweise für R¹, R³ und R⁴ angegeben wurden.

Die Ausgangsstoffe der Formel (IA) für Verfahren (b) sind erfindungsgemäße neue Verbindungen; sie können nach dem erfindungsgemäßen Verfahren (a) hergestellt werden.

Die beim erfindungsgemäßen Verfahren (b) weiter als Ausgangsstoffe zu verwendenden Alkylierungsmittel sind durch die Formeln (IV) und (V) allgemein definiert.

In den Formeln (IV) und (V) hat R⁵ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R⁵ angegeben wurde.

Die Ausgangsstoffe der Formeln (IV) und (V) sind bekannte organische Synthesechemikalien.

Das erfindungsgemäße Verfahren (b) wird vorzugsweise unter Verwendung eines Verdünnungsmittels durchgeführt. Es kommen hierbei vor allem diejenigen Verdünnungsmittel in Betracht, die bereits bei der Beschreibung des erfindungsgemäßen Verfahrens (a) genannt wurden.

Als Säureakzeptoren können bei dem erfindungsgemäßen Verfahren (b) alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tertbutylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0°C und 120°C, vorzugsweise bei Temperaturen zwischen 10°C und 100°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Die beim erfindungsgemäßen Verfahren (c) zur Herstellung von Verbindungen der Formel (I) als Ausgangsstoffe zu verwendenden N-Cyanoaryl-Stickstoffheterocyclen sind durch die Formel (IA) - mit der Maßgabe, daß darin R² für Halogen steht-allgemein definiert.

In Formel (IA) haben dann R¹, R³, R⁴ und R⁵ vorzugsweise diejenigen Bedeutungen, die bereits oben im Zusammenhang mit der Beschreibung der erfindungsgemäßen Verbindungen der Formel (IA) vorzugsweise für R¹, R³, R⁴ und R⁵ angegeben wurden.

Die Ausgangsstoffe der Formel (IA) für Verfahren (c) sind erfindungsgemäße neue Verbindungen; sie können nach den erfindungsgemäßen Verfahren (a) und (b) hergestellt werden.

Die beim erfindungsgemäßen Verfahren (c) weiter als Ausgangsstoffe zu verwendenden Amide sind durch die Formel (VI) allgemein definiert.

In Formel (VI) haben A und A¹ vorzugsweise diejenige Bedeutung, die bereits oben im Zusammenhang mit der Beschreibung der erfindungsgemäßen Verbindungen der Formel (I) vorzugsweise bzw. als insbesondere bevorzugt für A und A¹ angegeben wurde.

Die Ausgangsstoffe der Formel (VI) sind bekannte organische Synthesechemikalien.

Das erfindungsgemäße Verfahren (c) wird vorzugsweise unter Verwendung eines Verdünnungsmittels durchgeführt. Es kommen hierbei vor allem diejenigen Verdünnungsmittels in Betracht, die bereits bei der Beschreibung des erfindungsgemäßen Verfahrens (a) genannt wurden.

Das erfindungsgemäße Verfahren (c) wird gegebenenfalls in Gegenwart eines Säureakzeptors durchgeführt. Es kommen hierbei diejenigen Säureakzeptoren in Betracht, die bereits bei der Beschreibung des erfindungsgemäßen Verfahrens (b) genannt wurden.

Die Reaktionstemperaturen können bei dem erfindungsgemäßen Verfahren (c) in einem größeren Bereich variiert werden. Im allgemeinen arbeitet man bei Temperaturen zwischen 0°C und 200°C, vorzugsweise bei Temperaturen zwischen 20°C und 180°C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe eignen sich auch zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser. Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuronethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuronmethyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbuthylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinclorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

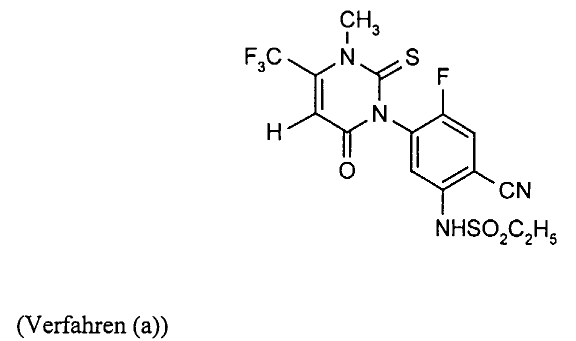

2,1 g (10,5 mMol) 3-Methylamino-4,4,4-trifluor-crotonsäure-ethylester werden in 50 ml Dimethylformamid und 5 ml Toluol vorgelegt und bei -70°C mit 0,4 g (10,9 mMol) Natriumhydrid (60%ig) versetzt. Das Gemisch wird bei der angegebenen Temperatur 15 Minuten gerührt und dann mit 2,0 g (7 mMol) 4-Cyano-2fluor-5-ethylsulfonylamino-phenyl-isothiocyanat versetzt. Die Reaktionsmischung wird dann 4 Stunden bei -60°C gerührt, anschließend mit 6 ml Essigsäure versetzt und mit Wasser auf etwa das doppelte Volumen verdünnt. Die organische Phase wird abgetrennt und eingeengt. Der Rückstand wird durch Digerieren mit Diisopropylether zur Kristallisation gebracht

Man erhält 0,7 g (25% der Theorie) 1-(4-Cyano-2-fluor-5-ethylsulfonylaminophenyl)-3,6-dihydro-2-thio-6-oxo-3-methyl-4-trifluormethyl-1(2H)-pyrimidin vom Schmelzpunkt 150°C.

Analog zu Beispiel 1 sowie entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

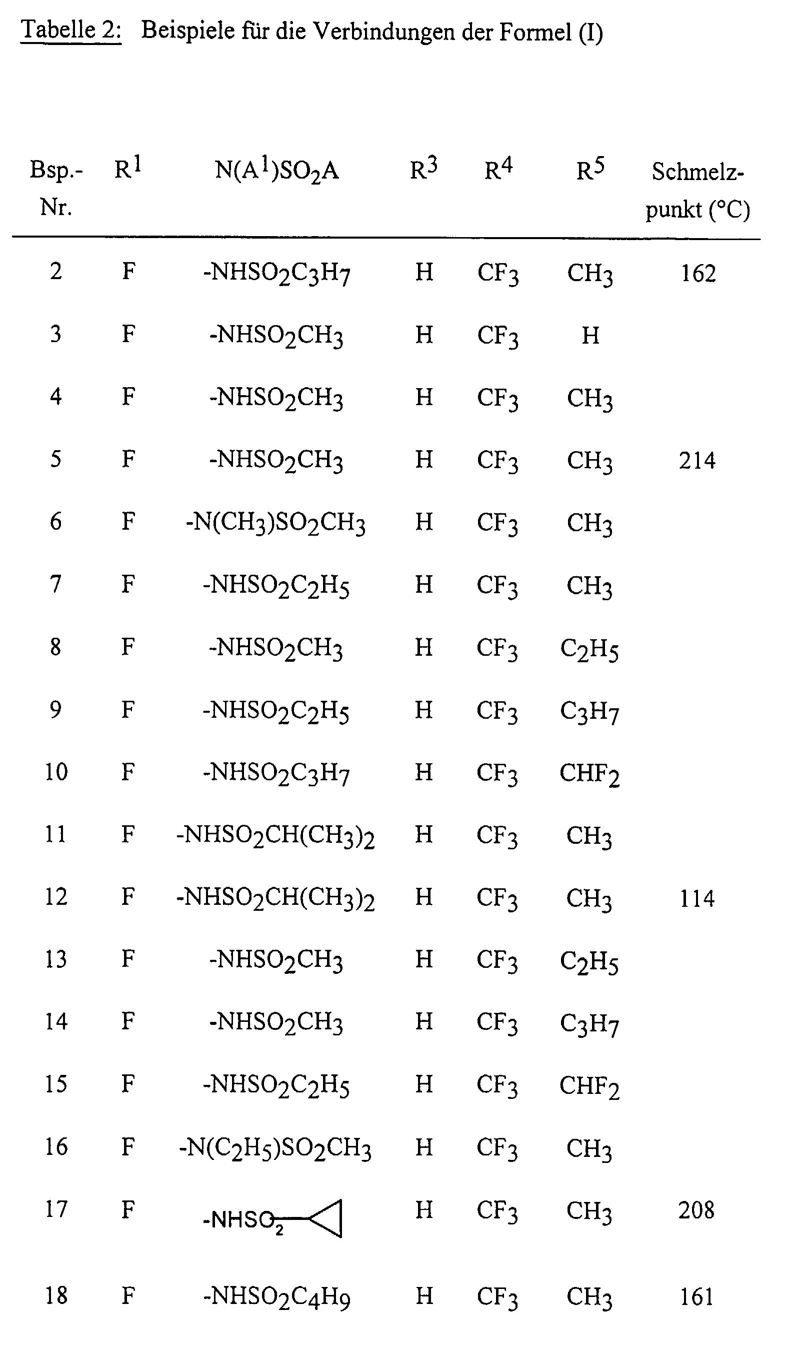

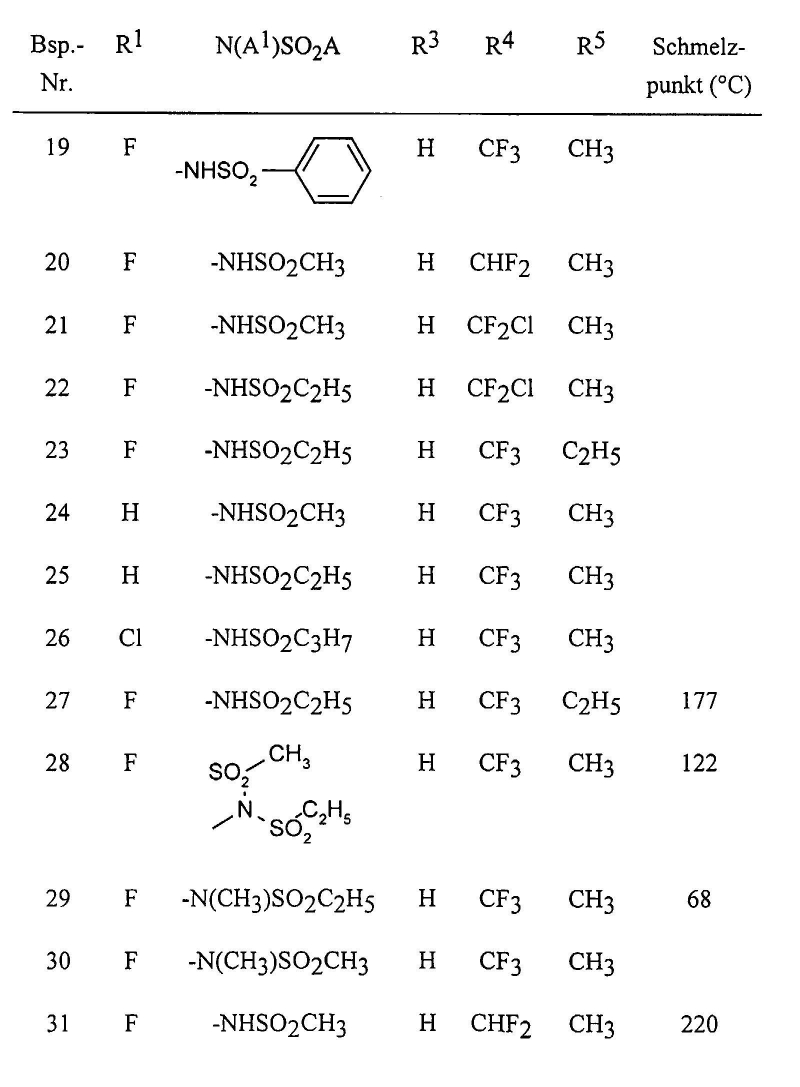

Die in Tabelle 2 als Beispiel 28 aufgeführte Verbindung kann beispielsweise wie folgt hergestellt werden:

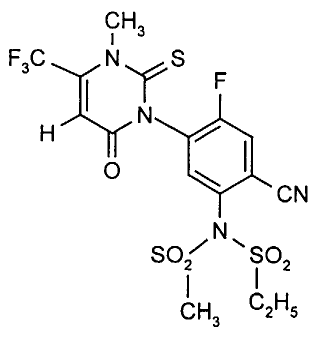

Ein Mischung aus 2,0 g (4,4 mMol) 1-(4-Cyano-2-fluor-5-ethylsulfonylaminophenyl)-3,6-dihydro-2-thio-6-oxo-3-methyl-4-trifluormethyl-1(2H)-pyrimidin, 0,64 g (5,6 mMol) Methansulfonsäurechlorid, 0,8 g (8 mMol) Triethylamin und 50 ml Acetonitril wird 15 Stunden bei 20°C gerührt und anschließend im Wasserstrahlvakuum eingeengt. Der Rückstand wird mit Wasser verrührt und das kristallin anfallende Produkt durch Abfiltrieren isoliert.

Man erhält 2,3 g (100% der Theorie) 1-(4-Cyano-2-fluor-5-(N-Ethylsulfonyl-N-methylsulfonyl-amino)-phenyl)-3,6-dihydro-2-thio-6-oxo-3-methyl-4-trifluormethyl-1(2H)-pyrimidin vom Schmelzpunkt 122°C.

Die in Tabelle 2 als Beispiel 29 aufgeführte Verbindung kann beispielsweise wie folgt hergestellt werden:

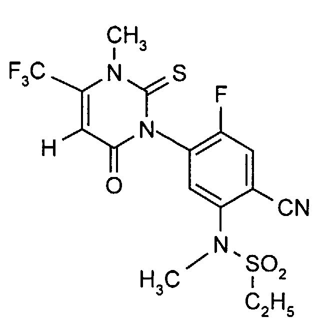

Eine Mischung aus 1,5 g (3,4 mMol) 1-(4-Cyano-2-fluor-5-ethylsulfonylaminophenyl)-3,6-dihydro-2-thio-6-oxo-3-methyl-4-trifluormethyl-1(2H)-pyrimidin, 0,5 g (3,8 mMol) Kaliumcarbonat und 30 ml Acetonitril wird 30 Minuten bei 20°C gerührt und dann mit 0,5 g (3,8 mMol) Dimethylsulfat versetzt. Das Reaktionsgemisch wird dann 5 Stunden unter Rückfluß erhitzt und weitere 60 Stunden bei 20°C gerührt. Dann wird im Wasserstrahlvakuum eingeengt, der Rückstand mit Wasser verrührt und das kristallin anfallende Produkt durch Abfiltrieren isoliert.

Man erhält 1,2 g (78% der Theorie) 1-(4-Cyano-2-fluor-5-(N-Ethylsulfonyl-N-methyl-amino)-phenyl)-3,6-dihydro-2-thio-6-oxo-3-methyl-4-trifluormethyl-1(2H)-pyrimidin vom Schmelzpunkt 68°C.

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = Keine Wirkung (wie unbehandelte Kontrolle)
100 % = totale Vernichtung
In diesem Test zeigen beispielsweise die Verbindungen gemäß den Herstellungsbeispielen 1, 4 und 5 bei guter Verträglichkeit gegenüber Kulturpflanzen, wie z.B. Weizen (0 %) starke Wirkung gegen Unkräuter wie Abutilon (80-100 %), Amaranthus (70-100 %), Ambrosia (95-100 %), Galinsoga (90-100 %), Portulaca (90-100 %) und Solanum (80-100 %) bei einer Aufwandmenge von 60 g pro Hektar.

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 1 und 5 bei durchweg sehr guter Verträglichkeit gegenüber Kulturpflanzen, wie z.B. Weizen (5 %) starke Wirkung gegen Unkräuter wie Abutilon (100 %), Chenopodium (100 %), Ipomoea (100 % ), Solanum (100 %) und Veronica (100 % ), bei einer Aufwandmenge von lediglich 30 g pro Hektar.

## Patentansprüche

1. N-Cyanoaryl-Stickstoffheterocyclen mit schwefelhaltigen Gruppierungen der allgemeinen Formel (I), 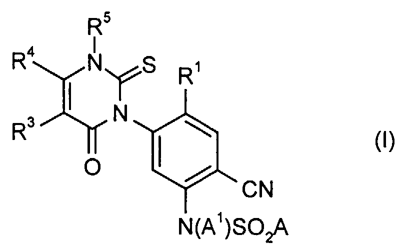 in welcher
R¹ für Wasserstoff, Fluor, Chlor oder Brom steht,
A für einen jeweils gegebenenfalls durch Fluor, Chlor, Brom, Cyano oder C₁-C₄-Alkoxy substituierten Rest der Reihe Alkyl, Alkenyl, Alkinyl oder Dialkylamino mit jeweils bis zu 10 Kohlenstoffatomen steht,
A weiterhin für jeweils gegebenenfalls durch Fluor, Chlor, Brom, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl oder Phenyloxy (welche jeweils gegebenenfalls durch Fluor, Chlor, Brom, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und gegebenenfalls 1 bis 4 Kohlenstoffatomen im Alkylteil steht, und
A¹ für Wasserstoff oder Formyl, oder für gegebenenfalls durch Fluor und/oder Chlor, Cyano oder C₁-C₄-Alkoxy-carbonyl substituiertes C₁-C₄-Alkyl, oder für jeweils gegebenenfalls durch Fluor, Chlor oder C₁-C₄-Alkoxy substituiertes C₁-C₄-Alkyl-carbonyl oder C₁-C₄-Alkoxy-carbonyl oder für gegebenenfalls durch Fluor und/oder Chlor substituiertes C₁-C₄-Alkylsulfonyl steht, oder
zusammen mit A für Alkandiyl mit 2 bis 8 Kohlenstoffatomen steht,
R³ für Wasserstoff, Fluor, Chlor, Brom, Cyano oder für gegebenenfalls durch Fluor und/oder Chlor substituiertes Alkyl mit 1 bis 4 Kohlenstoffatomen steht,
R⁴ für durch Fluor, Chlor, Brom substituiertes Alkyl mit 1 bis 6 Kohlenstoffatomen steht, und
R⁵ für Wasserstoff oder für jeweils gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl, Alkenyl, Alkinyl oder Alkylcarbonyl mit jeweils bis zu 6 Kohlenstoffatomen steht,
sowie Salze von Verbindungen der Formel (I).

2. N-Cyanoaryl-Stickstoffheterocyclen gemäß Anspruch 1, dadurch gekennzeichnet, daß
R¹ für Wasserstoff, Fluor oder Chlor steht,
A für einen jeweils gegebenenfalls durch Fluor oder Chlor substituierten Rest der Reihe Methyl, Ethyl, n- oder i-Propyl, n-, i-, s- oder t-Butyl, Ethenyl, Propenyl, Butenyl, oder für Dimethylamino steht,
A weiterhin für jeweils gegebenenfalls durch Fluor, Chlor, Brom, Methyl und/oder Ethyl substituiertes Cyclopropyl, Cyclopentyl, Cyclohexyl, Cyclopropylmethyl, Cyclopentylmethyl oder Cyclohexylmethyl steht,
A weiterhin für jeweils gegebenenfalls durch Fluor, Chlor, Brom, Cyano, Nitro, Carboxy, Methyl, Trifluormethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl, Dimethylaminosulfonyl, Methoxycarbonyl, Ethoxycarbonyl, Propoxycarbonyl oder Phenyl substituiertes Phenyl, Naphthyl, Phenylmethyl oder Phenylethyl steht, und
A¹ für Wasserstoff, Formyl, Methyl, Ethyl, Acetyl, Propionyl, Methoxycarbonyl, Ethoxycarbonyl, Methylsulfonyl oder Ethylsulfonyl steht, oder
zusammen mit A für Trimethylen oder Tetramethylen steht.
R³ für Wasserstoff, Fluor, Chlor, Brom, Methyl, Trifluormethyl, Ethyl, n-oder i-Propyl steht,
R⁴ für jeweils durch Fluor oder Chlor substituiertes Methyl, Ethyl, n-oder i-Propyl steht,
oder zusammen mit R³ für Trimethylen oder Tetramethylen steht, und
R⁵ für Wasserstoff, Methyl, Difluormethyl, Cyanomethyl, Ethyl, Fluorethyl, Chlorethyl, Difluorethyl, Dichlorethyl, Trifluorethyl, Chlordifluorethyl, Cyanoethyl, n- oder i-Propyl, Fluorpropyl, Chlorpropyl, Cyanopropyl, Allyl, Chlorallyl, Propargyl, Acetyl, Propionyl, Fluoracetyl, Chloracetyl, Difluoracetyl, Dichloracetyl, Trifluoracetyl oder Trichloracetyl steht.

3. N-Cyanoaryl-Stickstoffheterocyclen gemäß Anspruch 1, dadurch gekennzeichnet, daß sie in Form von Natrium-, Kalium-, Calcium-, Ammonium-, C₁-C₄-Alkyl-ammonium-, Di-(C₁-C₄-alkyl)-ammonium-, Tri-(C₁-C₄-alkyl)-ammonium-, Cyclopentyl- oder Cyclohexyl-ammonium- und Di-(C₁-C₄-alkyl)-ammonium-Salzen vorliegen.

4. Verfahren zur Herstellung von N-Cyanoaryl-Stickstoffheterocyclen gemäß Anspruch 1, dadurch gekennzeichnet, daß man
(a) Aminoalkensäureester der allgemeinen Formel (II) 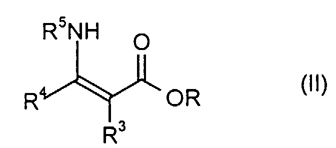 in welcher
R³, R⁴ und R⁵ die in Anspruch 1 angegebenen Bedeutungen haben und
R für Alkyl steht,
mit Cyanoarylisothiocyanaten der allgemeinen Formel (III) 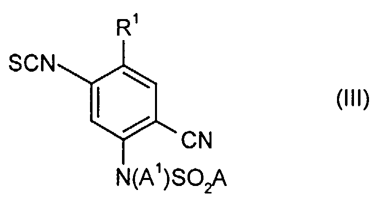 in welcher
R¹, A¹ and A die in Anspruch 1 angegebene Bedeutung hat,
gegebenenfalls in Gegenwart eines Reaktionshilfsmittels und gegebenenfalls in Gegenwart eines Verdünnungsmittels umsetzt,
oder daß man
(b) N-Cyanoaryl-Stickstoffheterocyclen der allgemeinen Formel (IA) 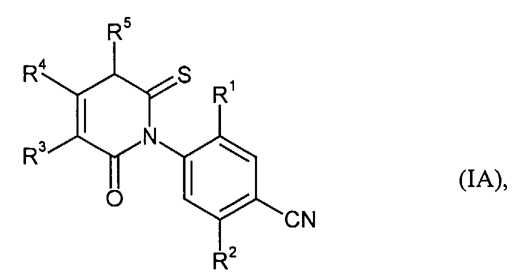 in welcher R² für Halogen steht sowie R¹, R³, R⁴ und R⁵ die in Anspruch 1 angegebenen Bedeutungen haben,
mit Amiden der allgemeinen Formel (VI)
HN(A¹)SO₂A (VI)
in welcher
A und A¹ die in Anspruch 1 angegebenen Bedeutungen haben,
gegebenenfalls in Gegenwart eines Säureakzeptors und gegebenenfalls in Gegenwart eines Verdünnungsmittels umsetzt,
oder daß man
(c) Aminoalkensäure(thio)ester der Formel (II) mit Cyanoarylthiomethanen der Formel (IIIa) 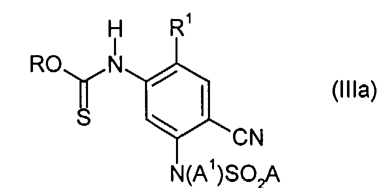 in welcher R¹, A¹ and A die in Anspruch 1 angegebene Bedeutung hat und R für Methyl oder Phenyl steht, umsetzt.

5. Verfahren zur Herstellung von N-Cyanoaryl-Stickstoffheterocyclen gemäß Anspruch 1.
worin R⁵ für jeweils gegebenenfalls substituiertes Alkyl, Alkenyl oder Alkinyl mit jeweils bis zu 6 Kohlenstoffatomen steht,
dadurch gekennzeichnet, daß man
N-Cyanoaryl-Stickstoffheterocyclen gemäß Anspruch 1, worin R⁵ für Wasserstoff steht mit Alkylierungsmitteln der allgemeinen Formeln (IV) oder (V)
X-R⁵ (IV)
R⁵-O-SO₂-O-R⁵ (V)
in welchen R⁵ die oben angegebene Bedeutung hat, gegebenenfalls in Gegenwart eines Säureakzeptors und gegebenenfalls in Gegenwart eines Verdünnungsmittels umsetzt.

6. Herbizide und insektizide Mittel, gekennzeichnet durch einen Gehalt an mindestens einem N-Cyanoaryl-Stickstoffheterocyclus gemäß Anspruch 1.

7. Verfahren zur Bekämpfung von unerwünschten Pflanzen, dadurch gekennzeichnet, daß man N-Cyanoaryl-Stickstoffheterocyclen gemäß Anspruch 1 auf unerwünschte Pflanzen und/oder ihren Lebensraum einwirken läßt.

8. Verwendung von substituierten N-Cyanoaryl-Stickstoffheterocyclen gemäß Anspruch 1 zur Bekämpfung von unerwünschten Pflanzen.

9. Verfahren zur Herstellung von herbiziden und insektiziden Mitteln, dadurch gekennzeichnet, daß man N-Cyanoaryl-Stickstoffheterocyclen gemäß Anspruch 1 mit Streckmitteln und/oder oberflächenaktiven Substanzen vermischt.

10. Verfahren zur Bekämpfung von unerwünschten Insekten, dadurch gekennzeichnet, daß man N-Cyanoaryl-Stickstoffheterocyclen gemäß Anspruch 1 auf unerwünschte Insekten und/oder ihren Lebensraum einwirken läßt.

11. Verwendung von substituierten N-Cyanoaryl-Stickstoffheterocyclen gemäß Anspruch 1 zur Bekämpfung von unerwünschten Insekten.

## Claims

1. N-Cyanoaryl nitrogen heterocycles containing sulphur groups of the general formula (I), 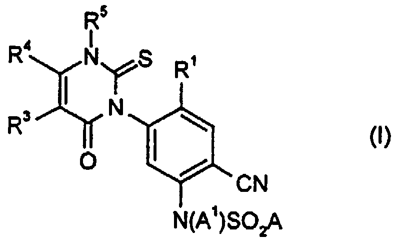 where
R¹ represents hydrogen, fluorine, chlorine or bromine,
A represents a radical from the group consisting of alkyl, alkenyl, alkinyl or dialkylamino, each of which has up to 10 carbon atoms and each of which is optionally substituted by fluorine, chlorine, bromine, cyano or C₁-C₄-alkoxy,
A additionally represents cycloalkyl or cycloalkylalkyl having 3 to 8 carbon atoms in the cycloalkyl moiety and optionally has 1 to 4 carbon atoms in the alkyl moiety and each of which is optionally substituted by fluorine, chlorine, bromine, cyano or C₁-C₄-alkyl,
A additionally represents aryl or arylalkyl having 6 or 10 carbon atoms in the aryl moiety and optionally has 1 to 4 carbon atoms in the alkyl moiety and each of which is optionally substituted by fluorine, chlorine, bromine, cyano, nitro, carboxy, carbamoyl, by C₁-C₄-alkyl, C₁-C₄-alkoxy, C₁-C₄-alkylthio, C₁-C₄-alkylsulphinyl or C₁-C₄-alkylsulphonyl (each of which is optionally substituted by fluorine and/or chlorine), by dimethylaminosulphonyl or diethylaminosulphonyl, by C₁-C₄-alkoxycarbonyl (each of which is optionally substituted by fluorine, chlorine, bromine, methoxy or ethoxy), by phenyl or phenyloxy (each of which is optionally substituted by fluorine, chlorine, bromine, cyano, methyl, methoxy, trifluoromethyl and/or trifluoromethoxy), and
A¹ represents hydrogen or formyl, or C₁-C₄-alkyl which is optionally substituted by fluorine and/or chlorine, cyano or C₁-C₄-alkoxycarbonyl, or C₁-C₄-alkylcarbonyl or C₁-C₄-alkoxycarbonyl, each of which is optionally substituted by fluorine, chlorine or C₁-C₄-alkoxy, or C₁-C₄-alkylsulphonyl which is optionally substituted by fluorine and/or chlorine, or
together with A represents alkanediyl having 2 to 8 carbon atoms,
R³ represents hydrogen, fluorine, chlorine, bromine, cyano or alkyl having 1 to 4 carbon atoms which is optionally substituted by fluorine and/or chlorine,
R⁴ represents fluorine-, chlorine-, bromine-substituted alkyl having 1 to 6 carbon atoms, and
R⁵ represents hydrogen or alkyl, alkenyl, alkinyl or alkylcarbonyl, each of which has up to 6 carbon atoms and each of which is optionally substituted by fluorine, chlorine, bromine, cyano, C₁-C₄-alkoxy, C₁-C₄-alkylcarbonyl or C₁-C₄-alkoxycarbonyl,
and salts of compounds of the formula (I).

2. N-Cyanoaryl nitrogen heterocycles according to Claim 1, characterized in that
R¹ represents hydrogen, fluorine or chlorine,
A represents a radical selected from the group consisting of methyl, ethyl, n-propyl or isopropyl, n-butyl, isobutyl, sec-butyl or tert-butyl, ethenyl, propenyl, butenyl, each of which is optionally substituted by fluorine or chlorine, or represents dimethylamino,
A additionally represents cyclopropyl, cyclopentyl, cyclohexyl, cyclopropylmethyl, cyclopentylmethyl or cyclohexylmethyl, each of which is optionally substituted by fluorine, chlorine, bromine, methyl and/or ethyl,
A additionally represents phenyl, naphthyl, phenylmethyl or phenylethyl, each of which is optionally substituted by fluorine, chlorine, bromine, cyano, nitro, carboxy, methyl, trifluoromethyl, methoxy, ethoxy, difluoromethoxy, trifluoromethoxy, methylthio, ethylthio, methylsulphinyl, ethylsulphinyl, methylsulphonyl, ethylsulphonyl, dimethylaminosulphonyl, methoxycarbonyl, ethoxycarbonyl, propoxycarbonyl or phenyl, and
A¹ represents hydrogen, formyl, methyl, ethyl, acetyl, propionyl, methoxycarbonyl, ethoxycarbonyl, methylsulphonyl or ethylsulphonyl,
or
together with A represents trimethylene or tetramethylene,
R³ represents hydrogen, fluorine, chlorine, bromine, methyl, trifluoromethyl, ethyl, n-propyl or isopropyl,
R⁴ represents methyl, ethyl, n-propyl or isopropyl, in each case substituted by fluorine or chlorine,
or together with R³ represents trimethylene or tetramethylene, and
R⁵ represents hydrogen, methyl, difluoromethyl, cyanomethyl, ethyl, fluoroethyl, chloroethyl, difluoroethyl, dichloroethyl, trifluoroethyl, chlorodifluoroethyl, cyanoethyl, n-propyl or isopropyl, fluoropropyl, chloropropyl, cyanopropyl, allyl, chloroallyl, propargyl, acetyl, propionyl, fluoroacetyl, chloroacetyl, difluoroacetyl, dichloroacetyl, trifluoroacetyl or trichloroacetyl.

3. N-Cyanoaryl nitrogen heterocycles according to Claim 1, characterized in that they are present in the form of sodium salts, potassium salts, calcium salts, ammonium salts, C₁-C₄-alkylammonium salts, di- (C₁-C₄-alkyl) ammonium salts, tri (C₁-C₄-alkyl)ammonium salts, cyclopentylammonium or cyclohexylammonium salts and di-(C₁-C₄-alkyl)ammonium salts.

4. Process for the preparation of n-cyanoaryl nitrogen heterocycles according to Claim 1,
characterized in that
(a) aminoalkenoic esters of the general formula (II) 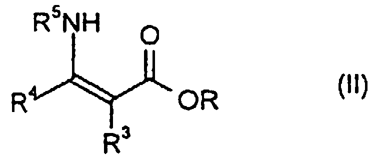 where
R³, R⁴ and R⁵ have the meanings specified in Claim 1 and
R represents alkyl,
are reacted with cyanoaryl isothiocyanates of the general formula (III) 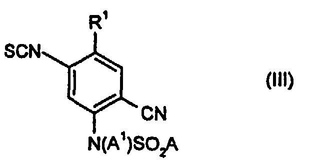 where
R¹, A¹ and A have the meanings specified in Claim 1,
in the presence or absence of a reaction auxiliary and the presence or absence of a diluent,
or in that
(b) N-cyanoaryl nitrogen heterocycles of the general formula (IA) 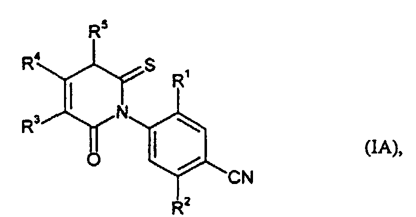 where R² represents halogen and R¹, R³, R⁴ and R⁵ have the meanings specified in Claim 1
are reacted with amides of the general formula (VI)
HN (A¹) SO₂A (VI)
where
A and A¹ have the meanings specified in Claim 1,
in the presence or absence of an acid acceptor and in the presence or absence of a diluent,
or in that
(c) aminoalkenoic (thio)esters of the formula (II) are reacted with cyanoarylthiomethanes of the formula (IIIa) 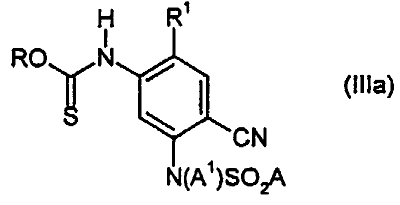 where R¹, A¹ and A have the meanings specified in Claim 1 and R represents methyl or phenyl.

5. Process for the preparation of N-cyanoaryl nitrogen heterocycles according to Claim 1,
where R⁵ represents alkyl, alkenyl or alkinyl, each of which has up to 6 carbon atoms and each of which is optionally substituted, characterized in that
N-cyanoaryl nitrogen heterocycles according to Claim 1, where R⁵ represents hydrogen are reacted with alkylation agents of the general formulae (IV) or (V)
X-R⁵ (IV)
R⁵-O-SO₂-O-R⁵ (V)
where R₅ has the meaning specified above, in the presence or absence of an acid acceptor and in the presence or absence of a diluent.

6. Herbicidal and insecticidal compositions characterized by a content of at least one N-cyanoaryl nitrogen heterocycle according to Claim 1.

7. Process for controlling undesirable plants,
characterized in that N-cyanoaryl nitrogen heterocycles according to Claim 1 are allowed to act on undesirable plants and/or their habitat.

8. The use of substituted N-cyanoaryl nitrogen heterocycles according to Claim 1 for controlling undesirable plants.

9. Process for the preparation of herbicidal and insecticidal compositions, characterized in that N-cyanoaryl nitrogen heterocycles according to Claim 1 are mixed with extenders and/or surfaceactive substances.

10. Process for controlling undesirable insects,
characterized in that N-cyanoaryl nitrogen heterocycles according to Claim 1 are allowed to act on undesirable insects and/or their habitat.

11. The use of substituted N-cyanoaryl nitrogen heterocycles according to Claim 1 for controlling undesirable insects.

## Revendications

1. N-cyanaryl-hétérocycles azotés porteurs de groupements contenant du soufre, de formule générale (I) 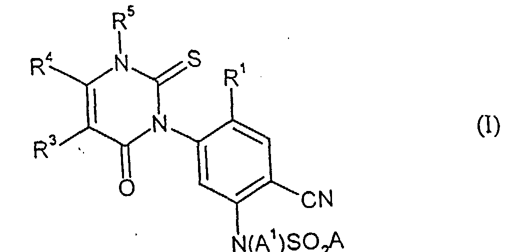 dans laquelle
R¹ représente l'hydrogène, le fluor, le chlore ou le brome,
A est un reste de la série alkyle, alcényle, alcynyle ou dialkylamino ayant chacun jusqu'à 10 atomes de carbone portant éventuellement dans chaque cas un substituant fluoro, chloro, bromo, cyano ou alkoxy en C₁ à C₄,
A représente en outre un reste cycloalkyle ou cyloalkylalkyle ayant 3 à 8 atomes de carbone dans la partie cycloalkyle et le cas échéant 1 à 4 atomes de carbone dans la partie alkyle et portant chacun le cas échéant un substituant fluoro, chloro, bromo, cyano, ou alkyle en C₁ à C₄,
A représente en outre un reste aryle ou arylalkyle ayant 6 ou 10 atomes de carbone dans la partie aryle et le cas échéant 1 à 4 atomes de carbone dans la partie alkyle, chacun étant substitué le cas échéant par un radical fluoro, chloro, bromo, cyano, nitro, carboxy, carbamoyle, par un radical alkyle en C₁ à C₄, alkoxy en C₁ à C₄, alkylthio en C₁ à C₄, alkylsulfinyle en C₁ à C₄ ou alkylsulfonyle en C₁ à C₄ (chacun étant substitué le cas échéant par du fluor et/ou du chlore), par un radical diméthylaminosulfonyle, ou diéthylaminosulfonyle, par un radical (alkoxy en C₁ à C₄)-carbonyle (qui est substitué le cas échéant par du fluor, du chlore, du brome, un radical méthoxy ou éthoxy), par un radical phényle ou phényloxy (chacun étant substitué le cas échéant par du fluor, du chlore, du brome, un radical cyano, méthyle, méthoxy, trifluorométhyle et/ou trifluorométhoxy), et
A¹ représente l'hydrogène ou le groupe formyle, ou un groupe alkyle en C₁ à C₄, éventuellement substitué par du fluor et/ou du chlore, un radical cyano ou (alkoxy en C₁ à C₄)-carbonyle, ou bien un groupe (alkyle en C₁ à C₄)-carbonyle ou (alkoxy en C₁ à C₄)-carbonyle portant chacun le cas échéant un substituant fluoro, chloro ou alkoxy en C₁ à C₄, ou un groupe alkylsulfonyle en C₁ à C₄ éventuellement substitué par du fluor et/ou du chlore, ou bien forme conjointement avec A un groupe alcanediyle ayant 2 à 8 atomes de carbone,
R³ représente l'hydrogène, le fluor, le chlore, le brome, un groupe cyano ou un groupe alkyle ayant 1 à 4 atomes de carbone éventuellement substitué par du fluor et/ou du chlore,
R⁴ est un groupe alkyle ayant 1 à 6 atomes de carbone substitué par du fluor, du chlore ou du brome et
R⁵ représente l'hydrogène ou un groupe alkyle, alcényle, alcynyle ou alkylcarbonyle ayant chacun jusqu'à 6 atomes de carbone et portant chacun le cas échéant un substituant fluoro, chloro, bromo, cyano, alkoxy en C₁ à C₄, (alkyle en C₁ à C₄)-carbonyle ou (alkoxy en C₁ à C₄)-carbonyle,
ainsi que des sels de composés de formule (I).

2. N-cyanaryl-hétérocycles azotés suivant la revendication 1, caractérisés en ce que
R¹ représente l'hydrogène, le fluor ou le chlore,
A représente un reste de la série méthyle, éthyle, n-propyle, isopropyle, n-butyle, iso-butyle, sec.-butyle, tertio-butyle, éthényle, propényle, butényle portant chacun le cas échéant un substituant fluoro ou chloro, ou le reste diméthylamino,
A représente en outre un reste cyclopropyle, cyclopentyle, cyclohexyle, cyclopropylméthyle, cyclopentylméthyle ou cyclohexylméthyle portant chacun le cas échéant un substituant fluoro, chloro, bromo, méthyle et/ou éthyle,
A représente en outre un reste phényle, naphtyle, phénylméthyle ou phényléthyle portant chacun le cas échéant un substituant fluoro, chloro, bromo, cyano, nitro, carboxy, méthyle, trifluorométhyle, méthoxy, éthoxy, difluorométhoxy, trifluorométhoxy, méthylthio, éthylthio, méthylsulfinyle, éthylsulfinyle, méthylsulfonyle, éthylsulfonyle, diméthylaminosulfonyle, méthoxycarbonyle, éthoxycarbonyle, propoxycarbonyle ou phényle, et
A¹ représente l'hydrogène, le groupe formyle, méthyle, éthyle, acétyle, propionyle, méthoxycarbonyle, éthoxycarbonyle, méthylsulfonyle ou éthylsulfonyle, ou bien
forme conjointement avec A un groupe triméthylène ou tétraméthylène,
R³ représente l'hydrogène, le fluor, le chlore, le brome, le groupe méthyle, trifluorométhyle, éthyle, n-propyle ou iso-propyle,
R⁴ est un reste méthyle, éthyle, n-propyle ou isopropyle portant chacun un substituant fluoro ou chloro,
ou forme conjointement avec R³ un groupe triméthylène ou tétraméthylène, et
R⁴ R⁵ représente l'hydrogène, le groupe méthyle, difluorométhyle, cyanométhyle, éthyle, fluoréthyle, chloréthyle, difluoréthyle, dichloréthyle, trifluoréthyle, chlorodifluoréthyle, cyanéthyle, n-propyle, iso-propyle, fluoropropyle, chloropropyle, cyanopropyle, allyle, chlorallyle, propargyle, acétyle, propionyle, fluoracétyle, chloracétyle, difluoracétyle, dichloracétyle, trifluoracétyle ou trichloracétyle;

3. N-cyanaryl-hétérocycles azotés suivant la revendication 1, caractérisés en ce qu'ils se présentent sous forme de sels de sodium, de potassium, de calcium, d'ammonium, de (alkyle en C₁ à C₄)-ammonium, de di-(alkyle en C₁ à C₄) -ammonium, de tri-(alkyle en C₁ à C₄) -ammonium, de cylopentyl-ammonium, de cyclohexyl-ammonium et de di-(alkyle en C₁ à C₄)-ammonium.

4. Procédé de production de N-cyanaryl-hétérocycles azotés suivant la revendication 1, caractérisés en ce que,
(a) on fait réagir des esters d'acide aminoalcénoïques de formule générale (II) 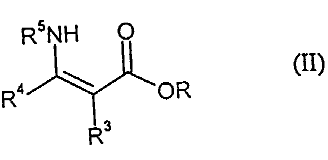 dans laquelle
R³, R⁴ et R⁵ ont les définitions indiquées dans la revendication 1
et
R est un groupe alkyle,
avec des isothiocyanates de cyanaryle de formule générale (III) 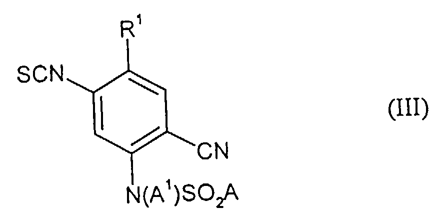 dans laquelle
R¹, A¹ et A ont la définition indiquée dans la revendication 1,
le cas échéant en présence d'un auxiliaire de réaction et en présence éventuelle d'un diluant,
ou bien
(b) on fait réagir des N-cyanaryl-hétérocycles azotés de formule générale (IA) 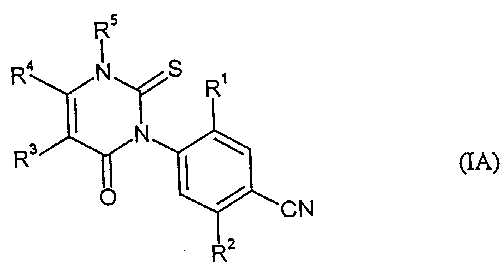 dans laquelle R² représente un halogène et R¹, R³, R⁴ et R⁵ ont les définitions indiquées dans la revendication 1,
avec des amides de formule générale (VI)
HN (A¹) SO₂A (VI)
dans laquelle A et A¹ ont les définitions indiquées dans la revendication 1,
le cas échéant en présence d'un accepteur d'acide et en présence éventuelle d'un diluant,
ou bien
(c) on fait réagir des (thio)esters d'acide amino-alcénoïques de formule (II) avec des cyanarylthiométhanes de formule (IIIa) 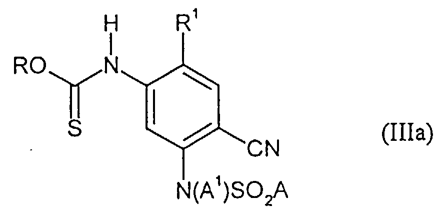 dans laquelle R¹, A¹ et A ont la définition indiquée dans la revendication 1 et R représente le groupe méthyle ou phényle.

5. Procédé de production de N-cyanaryl-hétérocycles azotés suivant la revendication 1,
dans lesquels R⁵ représente un groupe alkyle, alcényle ou alcynyle ayant chacun jusqu'à 6 atomes de carbone et chacun étant éventuellement substitué
caractérisé en ce qu'on fait réagir des N-cyanaryl-hétérocycles azotés suivant la revendication 1 dans lesquels R⁵ est l'hydrogène, avec des agents d'alkylation de formules générales (IV) ou (V)
X-R⁵ (IV)
R⁵-O-SO₂-O-R⁵ (V)
dans lesquelles R⁵ a la définition indiquée ci-dessus, le cas échéant en présence d'un accepteur d'acide et en présence éventuelle d'un diluant.

6. Compositions herbicides et insecticides, caractérisées par une teneur en au moins un N-cyanaryl-hétérocycle azoté suivant la revendication 1.

7. Procédé pour combattre des plantes indésirables, caractérisé en ce qu'on fait agir des N-cyanaryl-hétérocycles azotés suivant la revendication 1 sur les plantes indésirables et/ou sur leurs milieux.

8. Utilisation de N-cyanaryl-hétérocycles azotés substitués suivant la revendication 1 pour combattre des plantes indésirables.

9. Procédé de préparation de compositions herbicides et insecticides, caractérisé en ce qu'on mélange des N-cyanaryl-hétérocycles azotés suivant la revendication 1 avec des diluants et/ou des agents tensio-actifs.

10. Procédé pour combattre des insectes indésirables, caractérisé en ce que l'on fait agir des N-cyanaryl-hétérocycles azotés suivant la revendication 1 sur des insectes indésirables et/ou leur environnement,

11. Utilisation de N-cyanaryl-hétérocycles azotés substitués suivant la revendication 1 pour combattre des insectes indésirables.
